# EUROPEAN PATENT APPLICATION

(11) **EP 4 548 757 A1**
(43) Date of publication of application: **07.05.2025**
(21) Application number: 23831563.4
(22) Date of filing: 29.06.2023
(51) Int. Cl.: A01K 67/027, A61K 9/127, A61K 9/51, A61K 31/7088, A61K 35/76, A61K 38/17, A61K 47/34, A61K 47/44, A61K 48/00, A61P 9/00, A61P 9/10, A61P 25/00, A61P 43/00, C07D 211/58, C07K 14/47, C12N 15/12, C12N 15/864, C12N 15/867, C12Q 1/02, G01N 33/15, G01N 33/48

(54) **METHOD FOR PRODUCING NON-HUMAN PRIMATE MODEL OF CEREBRAL INFARCTION AND PHARMACEUTICAL COMPOSITION FOR TREATING CEREBRAL INFARCTION**

(30) Priority: 30.06.2022 JP 2022106308; 28.12.2022 JP 2022211994; 15.03.2023 JP 2023041345
(71) Applicant: Astellas Pharma, Inc., Chuo-ku Tokyo 103-8411 (JP)
(72) Inventor: NAKAHARA Soichiro, Tokyo 103-8411 (JP); MAEDA Masashi, Tokyo 103-8411 (JP)
(74) Representative: Hoffmann Eitle
(86) International application number: PCT/JP2023/024127
(87) International publication number: WO 2024/005124

(57) **Abstract**

The present invention relates to a method for producing a nonhuman primate animal model of cerebral infarction, comprising administering endothelin to basal ganglia and thalamic region of a nonhuman primate, and thereby inducing basal ganglia damage, thalamus damage, and internal capsule damage; and a pharmaceutical composition for the treatment of cerebral infarction at a subacute to chronic stage, penetrating branch infarction, or cerebral infarction having brain damage in a penetrating branch territory, comprising a NeuroD1 protein or a polynucleotide encoding the NeuroD1 protein.

## Description

### [Technical Field]

The present invention relates to a nonhuman primate animal model of cerebral infarction and a method for producing the same, a method for screening for a therapeutic agent for cerebral infarction using the animal model, a method for treating penetrating branch infarction, or cerebral infarction having brain damage in a penetrating branch territory, and a pharmaceutical composition for the treatment of penetrating branch infarction, or cerebral infarction having brain damage in a penetrating branch territory, etc.

### [Background Art]

The major cerebral arteries are a general name for a plurality of thick vascular vessels that supply oxygen or nutrition to the brain, and include the internal carotid artery, the middle cerebral artery, the anterior cerebral artery which supply blood to the cerebrum, and the vertebral artery and the basilar artery which supply blood to the cerebellum or the brain stem. The penetrating branch, a thin artery given off from the middle cerebral artery which constitutes the major cerebral arteries, delivers oxygen or nutrition to the basal ganglia, the thalamus, and the internal capsule situated in a deep part of the brain. The basal ganglia and the thalamus are gray matter where the cell bodies of neurons gather, whereas the internal capsule is white matter where nerve fibers mainly accumulate and run, being poor in the cell bodies of neurons. The surface of cerebral hemisphere is covered with a layer called cerebral cortex, and the cerebral cortex is gray matter where the cell bodies of neurons gather, as in the basal ganglia and the thalamus.

Cerebral infarction is a condition having neuronal death in a part to which blood is no longer delivered due to the blockage of a vascular vessel in the brain. Guidelines for the treatment of stroke recommend performing procedures with a therapeutic agent for cerebral infarction in order to minimize damage on the brain at the time of an aura symptom or within 4.5 hours from the occurrence of a seizure in cerebral infarction. Despite the presence of such a method for treating cerebral infarction at an acute stage, there exists no method for treating cerebral infarction at a subacute to chronic stage.

A model with ischemia induced by the craniotomy of a rat followed by the administration of endothelin having a strong vasoconstrictive effect to the surface of the middle cerebral artery is known as a rodent cerebral infarction model (Non Patent Literature 1). In many previous studies on therapeutic agents for cerebral infarction at an acute stage, a plurality of rodent cerebral infarction models including the rodent cerebral infarction model described in Non Patent Literature 1 have been used. Clinical trials on therapeutic agents for cerebral infarction at an acute stage have been carried out in view of efficacy in these rodent cerebral infarction models. However, few therapeutic agents have been demonstrated to have efficacy in the clinical trials (Non Patent Literatures 2 and 3). The discrepancy between efficacy in nonclinical trials using rodent cerebral infarction models and efficacy in clinical trials is caused by the difference in microcirculation in vascular vessels and structures of the brain, and the size of white matter occupying the cerebrum, etc. between rodents and humans. Hence, the guideline of the International Stroke Conference recommends making evaluation using primates having more similar vascular vessels and tissue structures of the brain to the human ones for the purpose of reducing the discrepancy between nonclinical and clinical results in the development of therapeutic agents for cerebral infarction.

A middle cerebral artery occlusion (MCAO) model which is a model with artificial blockage of the middle cerebral artery as a frequent site of vascular blockage in humans is generally used as a nonhuman primate model of cerebral infarction. In the MCAO model, it is known that a wide range of damage are induced in the cerebral cortex, the internal capsule, and the basal ganglia, which are similar to damage sites in human cerebral infarction (Non Patent Literature 4). The MCAO model has been reported to reach plateau high 3 to 5 days after induction of infarction and to reach plateau low 28 to 30 days after induction of infarction in evaluation using a Non-Human Primate Stroke Scale (NHPSS) which is a modified evaluation scale of National Institutes of Health Stroke Scale (NIHSS) as a neurological severity evaluation scale of human stroke adapted to nonhuman primate models (Non Patent Literature 4). The results indicate that the nonhuman primate MCAO model is a model that gradually recovers spontaneously, and therefore it is considered that the model is not suitable for the drug evaluation of cerebral infarction at a chronic stage.

A method for producing a nonhuman primate stroke model by causing vascular damage localized in an internal capsule region of the brain using a vascular damage inducer such as endothelin is known (Patent Literature 1). This stroke model recovers from impairment caused by vascular damage in the internal capsule region with time by natural healing ability, and many individuals have been shown to recover from impairment in gait, forelimb and hindlimb movement, and the like to the same level as that before injury approximately 2 weeks after injury (Patent Literature 1). In Patent Literature 1, Examples thereof indicate that motor dysfunction with high severity is not maintained for a long period in the stroke model, though motor dysfunction scoring such as NHPSS has not been performed as to the stroke model.

A method of causing thrombus in the posterior limb of the internal capsule of the brain using a photosensitive dye Rose Bengal and green light irradiation has been reported for the purpose of producing a white matter infarction model using a nonhuman primate (Non Patent Literature 5). In this cerebral infarction model, it has also been reported that persistent motor dysfunction cannot be induced when a damage site is slightly deviated from the target site in the posterior limb of the internal capsule, though it has been shown that complete damage on hand motor nerve can bring about persistent hand motor impairment and gait impairment (Non Patent Literature 5). This indicates that this production method of the model has the difficulty in controlling a site where thrombus occurs, and has the difficulty in inducing persistent motor dysfunction with high reproducibility.

There is no previous report on a cerebral infarction model that can be produced with high reproducibility and maintains motor dysfunction with high severity for a long period, and the efficacy evaluation of a therapeutic agent for cerebral infarction at a subacute to chronic stage 72 hours or later after the onset of cerebral infarction using the model. There is no report on a cerebral infarction model in which damage is induced specifically for a territory supplied by the penetrating branch by administering a vascular damage inducer to the basal ganglia or the thalamus as a territory supplied by the penetrating branch.

In therapeutic areas of cerebral infarction at a subacute to chronic stage, drugs capable of regenerating nerve have been demanded for many years. Although nerve regeneration approaches by cell transplantation or the activation of endogenous stem cells have been pursued so far, none of them have succeeded in clinical trials. A method using *in vivo* direct reprogramming (DR) is an approach that demarcates those two types of nerve regeneration approaches. The DR means the induction of differentiation of somatic cells into the cells of interest without involving pluripotent stem cells such as iPS cells.

A transcriptional factor NeuroD1 is known as a factor that achieves DR capable of converting astrocytes into neurons in the brain. It has been reported that in mice, forced expression of NeuroD1 in astrocytes in the brain caused DR from astrocytes into neurons (Patent Literature 2 and Non Patent Literature 6). It has also been reported that in mice, NeuroD1 converts microglia into neurons and maturates neural stem cells or neural progenitor cells into neurons (Non Patent Literatures 7 to 9).

It has been reported that: rodent cerebral cortex infarction models were replenished with neurons through DR by forced expression of NeuroD1 in astrocytes in the brain at a subacute stage 7 to 9 days after brain injury, and thereby recovered from impairment of fine motor skills such as food taking action; and in mouse models of transient blockage of the middle cerebral artery, forced expression of NeuroD1 1 week after brain injury caused conversion of microglia/macrophages into neurons and recovery from impairment of fine motor skills (Non Patent Literatures 10 to 13). It has been further reported that in the primate cerebral cortex as well, forced expression of NeuroD1 10 to 30 days after brain injury converted astrocytes into neurons through DR (Non Patent Literature 14). Although there are a plurality of reports on such cases of neural conversion mediated by NeuroD1, a reported case of functional recovery mediated by NeuroD1 merely remains at the recovery of fine motor skills by the expression of NeuroD1 at an acute to subacute stage in rodent cerebral infarction models. There is no report on (1) a case of the recovery of gross motor skills such as gait or forelimb and hindlimb movement mediated by NeuroD1, (2) a case of functional recovery when NeuroD1 is administered at a chronic stage, and (3) a case of functional recovery in nonhuman primates mediated by NeuroD1.

### [Citation List]

### [Patent Literature]

[Patent Literature 1] JP Patent Publication No. 2015-231338 A
[Patent Literature 2] International Publication No. WO2014/015261

### [Non Patent Literature]

[Non Patent Literature 1] Sharkey J et al., J. Cereb. Blood Flow Metab., vol. 13, pp. 865-871, 1993
[Non Patent Literature 2] Clark WM et al., Neurology, vol. 57, pp. 1595-1602, 2001
[Non Patent Literature 3] Diener HC et al., Stroke, vol. 31, pp. 2543-2551, 2000
[Non Patent Literature 4] Ramirez-Garcia G.et al., Neurosci., vol. 397, pp. 41-55, 2019
[Non Patent Literature 5] Hyung-Sun K.et al., Exp. Neurobiol., vol. 30, pp. 356-364, 2021
[Non Patent Literature 6] Guo Z et al., Cell Stem Cell, vol. 14, pp. 188-202, 2014
[Non Patent Literature 7] Taito M et al., Neuron, vol. 101, pp. 472-485, 2019
[Non Patent Literature 8] Kevin R et al., Brain, vol. 138, pp. 440-455, 2015
[Non Patent Literature 9] Zhengliang G et al., Nat. Neurosci., Vol. 12, pp. 1090-1092, 2009
[Non Patent Literature 10] Chen YC et al., Mol. Ther., vol. 28, pp. 217-234, 2020
[Non Patent Literature 11] Livingston J et al., bioRxiv, 2020, doi:https://doi.org/10.1101/2020.02.02.929091
[Non Patent Literature 12] Jiang MQ et al., Front. Aging Neurosci., vol. 13, 612856, 2021
[Non Patent Literature 13] Irie T et al., bioRxiv, 2021, doi:https://doi.org/10.1101/2021.09.26.461831
[Non Patent Literature 14] Ge LJ et al. Front. Cell Dev. Biol., vol. 5, 590008, 2020

### [Summary of Invention]

### [Technical Problem]

An object of the present invention is to provide a nonhuman primate animal model of cerebral infarction that can be used in the screening for or evaluation of a therapeutic agent for cerebral infarction, particularly, a therapeutic agent for cerebral infarction at a subacute to chronic stage, penetrating branch infarction, or cerebral infarction having brain damage in a penetrating branch territory; and a method for producing the animal model. Another object of the present invention is to provide a therapeutic agent or a treatment method for penetrating branch infarction, or cerebral infarction having brain damage in a penetrating branch territory.

### [Solution to Problem]

The present inventors have conducted diligent studies to attain the objects described above and consequently found that: basal ganglia damage and thalamus damage as well as internal capsule damage can be induced by administering endothelin to basal ganglia and thalamic region of a nonhuman primate; the resulting nonhuman primate having basal ganglia damage, thalamus damage, and internal capsule damage is capable of serving as a nonhuman primate animal model of cerebral infarction; and the nonhuman primate animal model of cerebral infarction is particularly useful as a nonhuman primate animal model of cerebral infarction at a subacute to chronic stage, penetrating branch infarction, or cerebral infarction having brain damage in a penetrating branch territory (Example 1). The present inventors have also found using the nonhuman primate animal model that NeuroD1 is effective as a therapeutic agent for penetrating branch infarction, or cerebral infarction having brain damage in a penetrating branch territory (Example 2 to Example 5, Example 11, and Example 12). The present invention has been completed on the basis of these findings.

Specifically, the present invention provides the following aspects.

[1] A method for producing a nonhuman primate animal model of cerebral infarction, comprising administering endothelin to basal ganglia and thalamic region of a nonhuman primate, and thereby inducing basal ganglia damage, thalamus damage, and internal capsule damage.
[2] The method according to [1], wherein endothelin is administered to one or two or more nerve nuclei selected from the group consisting of putamen, caudate nucleus, globus pallidus, ventral lateral nucleus of thalamus, and ventral posterior nucleus of thalamus in the basal ganglia and thalamic region.
[3] The method according to [1] or [2], wherein endothelin is administered to at least three nerve nuclei selected from the group consisting of putamen, caudate nucleus, globus pallidus, ventral lateral nucleus of thalamus, and ventral posterior nucleus of thalamus in the basal ganglia and thalamic region.
[4] The method according to any of [1] to [3], wherein endothelin is administered to at least four nerve nuclei selected from the group consisting of putamen, caudate nucleus, globus pallidus, ventral lateral nucleus of thalamus, and ventral posterior nucleus of thalamus in the basal ganglia and thalamic region.
[5] The method according to any of [1] to [4], wherein endothelin is administered to all of five nerve nuclei of the group consisting of putamen, caudate nucleus, globus pallidus, ventral lateral nucleus of thalamus, and ventral posterior nucleus of thalamus in the basal ganglia and thalamic region.
[6] The method according to any of [1] to [5], wherein the cerebral infarction is cerebral infarction at a subacute to chronic stage.
[7] The method according to any of [1] to [6], wherein the cerebral infarction is cerebral infarction at a chronic stage.
[8] The method according to any of [1] to [7], wherein the cerebral infarction is penetrating branch infarction.
[9] The method according to any of [1] to [7], wherein the cerebral infarction is cerebral infarction having brain damage in a penetrating branch territory.
[10] The method according to [8] or [9], wherein the penetrating branch infarction, or the cerebral infarction having brain damage in a penetrating branch territory is cerebral infarction at a subacute to chronic stage.
[11] The method according to [8] or [9], wherein the penetrating branch infarction or the cerebral infarction having brain damage in a penetrating branch territory is cerebral infarction at a chronic stage.
[12] The method according to any of [2] to [11], comprising determining an administration site so as to target said nerve nuclei, and administering endothelin to the administration site.
[13] A method for producing a nonhuman primate animal model of cerebral infarction, comprising administering endothelin to basal ganglia and thalamic region of a nonhuman primate, and thereby inducing basal ganglia damage, thalamus damage, and internal capsule damage, wherein the method comprises the steps of:
   (a) determining administration sites so as to target at least four nerve nuclei selected from the group consisting of putamen, caudate nucleus, globus pallidus, ventral lateral nucleus of thalamus, and ventral posterior nucleus of thalamus in the basal ganglia and thalamic region; and
   (b) administering endothelin to the administration sites.
[14] A method for producing a nonhuman primate animal model of cerebral infarction, comprising administering endothelin to basal ganglia and thalamic region of a nonhuman primate, and thereby inducing basal ganglia damage, thalamus damage, and internal capsule damage, wherein the method comprises the steps of:
   (a) determining administration sites so as to target all of five nerve nuclei of the group consisting of putamen, caudate nucleus, globus pallidus, ventral lateral nucleus of thalamus, and ventral posterior nucleus of thalamus in the basal ganglia and thalamic region; and
   (b) administering endothelin to the administration sites.
[15] The method according to [13] or [14], wherein the administration sites are at least three sites.
[16] The method according to any of [1] to [15], wherein the nonhuman primate is a cynomolgus macaque.
[17] The method according to any of [1] to [16], wherein a dose of endothelin per administration site is at least 10 µg, preferably at least 20 µg.
[18] The method according to any of [1] to [17], wherein the endothelin is endothelin-1.
[19] A nonhuman primate animal model of cerebral infarction, which has basal ganglia damage, thalamus damage, and internal capsule damage, wherein the nonhuman primate animal model is produced by a method according to any of [1] to [18].
[20] A method for producing a nonhuman primate animal model of cerebral infarction at a subacute to chronic stage, comprising administering endothelin to a cynomolgus macaque, and thereby inducing basal ganglia damage, thalamus damage, and internal capsule damage, wherein endothelin administration sites include the following:
   administration sites respectively positioned at (i) B: 1 to 3 mm, L: 7 to 9 mm, and D: 18 to 20 mm, (ii) B: 4 to 6 mm, L: 7 to 9 mm, and D: 18 to 20 mm, and (iii) B: 8 to 12 mm, L: 4 to 13 mm, and D: 12 to 20 mm from Bregma as a base point.
[21] The method according to [20], wherein the endothelin administration sites include any one of the following (1) to (3):
   (1) administration sites respectively positioned at (i) B: 1 to 3 mm, L: 7 to 9 mm, and D: 18 to 20 mm, (ii) B: 4 to 6 mm, L: 7 to 9 mm, and D: 18 to 20 mm, (iii) B: 8 to 10 mm, L: 4 to 6 mm, and D: 12 to 14 mm, and (iv) B: 8 to 10 mm, L: 11 to 13 mm, and D: 14 to 16 mm from Bregma as a base point;
   (2) administration sites respectively positioned at (i) B: 1 to 3 mm, L: 7 to 9 mm, and D: 18 to 20 mm, (ii) B: 4 to 6 mm, L: 7 to 9 mm, and D: 18 to 20 mm, and (iii) B: 8 to 10 mm, L: 7 to 9 mm, and D: 18 to 20 mm from Bregma as a base point; and
   (3) administration sites respectively positioned at (i) B: 1 to 3 mm, L: 7 to 9 mm, and D: 18 to 20 mm, (ii) B: 4 to 6 mm, L: 7 to 9 mm, and D: 18 to 20 mm, and (iii) B: 10 to 12 mm, L: 7 to 9 mm, and D: 18 to 20 mm from Bregma as a base point.
[22] The method according to [20] or [21], wherein the endothelin administration sites include any one of the following (1) to (3):
   (1) administration sites respectively positioned at (i) B: 2 mm, L: 8 mm, and D: 19 mm, (ii) B: 5 mm, L: 8 mm, and D: 19 mm, (iii) B: 9 mm, L: 5 mm, and D: 13 mm, and (iv) B: 9 mm, L: 12 mm, and D: 15 mm from Bregma as a base point;
   (2) administration sites respectively positioned at (i) B: 2 mm, L: 8 mm, and D: 19 mm, (ii) B: 5 mm, L: 8 mm, and D: 19 mm, and (iii) B: 9 mm, L: 8 mm, and D: 19 mm from Bregma as a base point; and
   (3) administration sites respectively positioned at (i) B: 2 mm, L: 8 mm, and D: 19 mm, (ii) B: 5 mm, L: 8 mm, and D: 19 mm, and (iii) B: 11 mm, L: 8 mm, and D: 19 mm from Bregma as a base point.
[23] The method according to any of [20] to [22], wherein the endothelin administration sites include the following administration sites:
   administration sites respectively positioned at (i) B: 2 mm, L: 8 mm, and D: 19 mm, (ii) B: 5 mm, L: 8 mm, and D: 19 mm, (iii) B: 9 mm, L: 5 mm, and D: 13 mm, and (iv) B: 9 mm, L: 12 mm, and D: 15 mm from Bregma as a base point.
[24] The method according to any of [20] to [23], wherein the endothelin administration sites are the following administration sites:
   administration sites respectively positioned at (i) B: 2 mm, L: 8 mm, and D: 19 mm, (ii) B: 5 mm, L: 8 mm, and D: 19 mm, (iii) B: 9 mm, L: 5 mm, and D: 13 mm, and (iv) B: 9 mm, L: 12 mm, and D: 15 mm from Bregma as a base point.
[25] The method according to any of [20] to [24], wherein the cerebral infarction is cerebral infarction at a chronic stage.
[26] The method according to any of [20] to [25], wherein the cerebral infarction is penetrating branch infarction, or cerebral infarction having brain damage in a penetrating branch territory.
[27] The method according to any of [20] to [26], wherein a dose of endothelin per administration site is at least 10 µg, preferably at least 20 µg.
[28] The method according to any of [20] to [27], wherein the endothelin is endothelin-1.
[29] A cynomolgus macaque model of cerebral infarction at a subacute to chronic stage, produced by a method according to any of [20] to [28].
[30] A method for screening for a therapeutic agent for cerebral infarction, comprising the steps of: administering a test substance to a nonhuman primate animal model according to [19] or [29]; and determining an effect of the test substance on cerebral infarction in the nonhuman primate animal model.
[31] A pharmaceutical composition for the treatment of penetrating branch infarction, or cerebral infarction at a subacute to chronic stage, comprising a NeuroD1 protein or a polynucleotide encoding the NeuroD1 protein.
[32] A pharmaceutical composition for the treatment of cerebral infarction having brain damage in a penetrating branch territory, comprising a NeuroD1 protein or a polynucleotide encoding the NeuroD1 protein.
[33] The pharmaceutical composition according to [31] or [32], wherein the penetrating branch infarction or the cerebral infarction having brain damage in a penetrating branch territory is cerebral infarction at a subacute to chronic stage.
[34] The pharmaceutical composition according to any of [31] to [33], wherein the penetrating branch infarction or the cerebral infarction having brain damage in a penetrating branch territory is cerebral infarction at a chronic stage.
[35] The pharmaceutical composition according to [31], wherein the cerebral infarction at a subacute to chronic stage is cerebral infarction at a chronic stage.
[36] The pharmaceutical composition according to any of [31] to [35], wherein the pharmaceutical composition comprises the polynucleotide encoding the NeuroD1 protein, and said polynucleotide is a polynucleotide comprising a nucleotide sequence encoding a protein consisting of the amino acid sequence as set forth by SEQ ID NO: 8.
[37] The pharmaceutical composition according to any of [31] to [36], wherein the polynucleotide encoding the NeuroD1 protein comprises a polynucleotide having 70% or higher sequence identity to the nucleotide sequence as set forth by SEQ ID NO: 7 or 10.
[38] The pharmaceutical composition according to any of [31] to [37], wherein the polynucleotide encoding the NeuroD1 protein is a polynucleotide comprising the nucleotide sequence as set forth by SEQ ID NO: 7 or 10.
[39] The pharmaceutical composition according to any of [31] to [38], wherein the polynucleotide encoding the NeuroD1 protein is mRNA encoding the NeuroD1 protein.
[40] The pharmaceutical composition according to [39], wherein the mRNA encoding the NeuroD1 protein comprises a nucleotide sequence encoding the NeuroD1 protein of positions 44 to 1114 or positions 44 to 1120 of SEQ ID NO: 11 or 13.
[41] The pharmaceutical composition according to [39] or [40], wherein the mRNA encoding the NeuroD1 protein comprises 5' UTR and/or 3' UTR.
[42] The pharmaceutical composition according to any of [39] to [41], wherein the mRNA encoding the NeuroD1 protein is mRNA comprising a polynucleotide consisting of the nucleotide sequence of positions 1 to 1231 of SEQ ID NO: 11 or 13.
[43] The pharmaceutical composition according to any of [39] to [42], wherein the mRNA encoding the NeuroD1 protein comprises a polyA sequence.
[44] The pharmaceutical composition according to [43], wherein the polyA sequence is 50 to 200 nucleotides in length.
[45] The pharmaceutical composition according to any of [39] to [44], wherein the mRNA encoding the NeuroD1 protein has a 5' cap structure.
[46] The pharmaceutical composition according to any of [39] to [45], wherein the mRNA encoding the NeuroD1 protein is mRNA containing a modified nucleoside.
[47] The pharmaceutical composition according to [46], wherein the modified nucleoside is N1-methylpseudouridine.
[48] The pharmaceutical composition according to [47], wherein the mRNA encoding the NeuroD1 protein is a polynucleotide in which some or all uridines are replaced by N1-methylpseudouridine.
[49] The pharmaceutical composition according to any of [39] to [48], wherein the mRNA encoding the NeuroD1 protein is incorporated in a drug delivery system (DDS).
[50] The pharmaceutical composition according to any of [39] to [49], wherein the mRNA encoding the NeuroD1 protein is encapsulated into lipid nanoparticles.
[51] The pharmaceutical composition according to any of [31] to [38], wherein the pharmaceutical composition comprises the polynucleotide encoding the NeuroD1 protein, and said polynucleotide is DNA.
[52] The pharmaceutical composition according to any of [31] to [38] and [51], wherein the pharmaceutical composition comprises an expression vector comprising the polynucleotide encoding the NeuroD1 protein.
[53] The pharmaceutical composition according to [52], wherein the expression vector is an adeno-associated virus vector, a retrovirus vector, a Sendai virus vector, or a lentivirus vector.
[54] The pharmaceutical composition according to [53], wherein the expression vector is an adeno-associated virus vector.
[55] A method for treating penetrating branch infarction, or cerebral infarction at a subacute to chronic stage, comprising the step of administering a pharmaceutical composition according to any of [31] to [54], a NeuroD1 protein, or a polynucleotide encoding the NeuroD1 protein to a subject.
[56] A method for treating cerebral infarction having brain damage in a penetrating branch territory, comprising the step of administering a pharmaceutical composition according to any of [31] to [54], a NeuroD1 protein, or a polynucleotide encoding the NeuroD1 protein to a subject.
[57] The method according to [55] or [56], wherein the penetrating branch infarction, or the cerebral infarction having brain damage in a penetrating branch territory is cerebral infarction at a subacute to chronic stage.
[58] The method according to any of [55] to [57], wherein the penetrating branch infarction, or the cerebral infarction having brain damage in a penetrating branch territory is cerebral infarction at a chronic stage.
[59] The method according to [55], wherein the cerebral infarction at a subacute to chronic stage is cerebral infarction at a chronic stage.
[60] The method according to any of [55] to [59], wherein the method comprises the step of administering the polynucleotide encoding the NeuroD1 protein to the subject, and wherein said polynucleotide is a polynucleotide comprising a nucleotide sequence encoding a protein consisting of the amino acid sequence as set forth by SEQ ID NO: 8.
[61] The method according to any of [55] to [60], wherein the polynucleotide encoding the NeuroD1 protein comprises a polynucleotide having 70% or higher sequence identity to the nucleotide sequence as set forth by SEQ ID NO: 7 or 10.
[62] The method according to any of [55] to [61], wherein the polynucleotide encoding the NeuroD1 protein is a polynucleotide comprising the nucleotide sequence as set forth by SEQ ID NO: 7 or 10.
[63] The method according to any of [55] to [62], wherein the method comprises the step of administering the polynucleotide encoding the NeuroD1 protein to the subject, and wherein said polynucleotide is mRNA encoding the NeuroD1 protein.
[64] The method according to [63], wherein the mRNA encoding the NeuroD1 protein comprises a nucleotide sequence encoding the NeuroD1 protein of positions 44 to 1114 or positions 44 to 1120 of SEQ ID NO: 11 or 13.
[65] The method according to [63] or [64], wherein the mRNA encoding the NeuroD1 protein comprises 5' UTR and/or 3' UTR.
[66] The method according to any of [63] to [65], wherein the mRNA encoding the NeuroD1 protein is mRNA comprising a polynucleotide consisting of the nucleotide sequence of positions 1 to 1231 of SEQ ID NO: 11 or 13.
[67] The method according to any of [63] to [66], wherein the mRNA encoding the NeuroD1 protein comprises a polyA sequence.
[68] The method according to [67], wherein the polyA sequence is 50 to 200 nucleotides in length.
[69] The method according to any of [63] to [68], wherein the mRNA encoding the NeuroD1 protein has a 5' cap structure.
[70] The method according to any of [63] to [69], wherein the mRNA encoding the NeuroD1 protein is mRNA containing a modified nucleoside.
[71] The method according to [70], wherein the modified nucleoside is N1-methylpseudouridine.
[72] The method according to [71], wherein the mRNA encoding the NeuroD1 protein is a polynucleotide in which some or all uridines are replaced by N1-methylpseudouridine.
[73] The method according to any of [63] to [72], wherein the mRNA encoding the NeuroD1 protein is incorporated in a drug delivery system (DDS).
[74] The method according to any of [63] to [73], wherein the mRNA encoding the NeuroD1 protein is encapsulated into lipid nanoparticles.
[75] The method according to any of [55] to [62], wherein the method comprises the step of administering the polynucleotide encoding the NeuroD1 protein to the subject, and wherein said polynucleotide is DNA.
[76] The method according to any of [55] to [62] and [75], wherein the method comprises the step of administering an expression vector comprising the polynucleotide encoding the NeuroD1 protein to the subject.
[77] The method according to [76], wherein the expression vector is an adeno-associated virus vector, a retrovirus vector, a Sendai virus vector, or a lentivirus vector.
[78] The method according to [77], wherein the expression vector is an adeno-associated virus vector.
[79] A NeuroD1 protein or a polynucleotide encoding the NeuroD1 protein, for use in the treatment of penetrating branch infarction, or cerebral infarction at a subacute to chronic stage.
[80] A NeuroD1 protein or a polynucleotide encoding the NeuroD1 protein, for use in the treatment of cerebral infarction having brain damage in a penetrating branch territory.
[81] The protein or the polynucleotide for use according to [79] or [80], wherein the penetrating branch infarction, or the cerebral infarction having brain damage in a penetrating branch territory is cerebral infarction at a subacute to chronic stage.
[82] The protein or the polynucleotide for use according to any of [79] to [81], wherein the penetrating branch infarction, or the cerebral infarction having brain damage in a penetrating branch territory is cerebral infarction at a chronic stage.
[83] The protein or the polynucleotide for use according to [79], wherein the cerebral infarction at a subacute to chronic stage is cerebral infarction at a chronic stage.
[84] The polynucleotide for use according to any of [79] to [83], wherein the polynucleotide encoding the NeuroD1 protein is a polynucleotide comprising a nucleotide sequence encoding a protein consisting of the amino acid sequence as set forth by SEQ ID NO: 8.
[85] The polynucleotide for use according to any of [79] to [84], wherein the polynucleotide encoding the NeuroD1 protein comprises a polynucleotide having 70% or higher sequence identity to the nucleotide sequence as set forth by SEQ ID NO: 7 or 10.
[86] The polynucleotide for use according to any of [79] to [85], wherein the polynucleotide encoding the NeuroD1 protein is a polynucleotide comprising the nucleotide sequence as set forth by SEQ ID NO: 7 or 10.
[87] The polynucleotide for use according to any of [79] to [86], wherein said polynucleotide is mRNA encoding the NeuroD1 protein.
[88] The polynucleotide for use according to [87], wherein the mRNA encoding the NeuroD1 protein comprises a nucleotide sequence encoding the NeuroD1 protein of positions 44 to 1114 or positions 44 to 1120 of SEQ ID NO: 11 or 13.
[89] The polynucleotide for use according to [87] or [88], wherein the mRNA encoding the NeuroD1 protein comprises 5' UTR and/or 3' UTR.
[90] The polynucleotide for use according to any of [87] to [89], wherein the mRNA encoding the NeuroD1 protein is mRNA comprising a polynucleotide consisting of the nucleotide sequence of positions 1 to 1231 of SEQ ID NO: 11 or 13.
[91] The polynucleotide for use according to any of [87] to [90], wherein the mRNA encoding the NeuroD1 protein comprises a polyA sequence.
[92] The polynucleotide for use according to [91], wherein the polyA sequence is 50 to 200 nucleotides in length.
[93] The polynucleotide for use according to any of [87] to [92], wherein the mRNA encoding the NeuroD1 protein has a 5' cap structure.
[94] The polynucleotide for use according to any of [87] to [93], wherein the mRNA encoding the NeuroD1 protein is a polynucleotide containing a modified nucleoside.
[95] The polynucleotide for use according to [94], wherein the modified nucleoside is N1-methylpseudouridine.
[96] The polynucleotide for use according to [95], wherein the mRNA encoding the NeuroD1 protein is a polynucleotide in which some or all uridines are replaced by N1-methylpseudouridine.
[97] The polynucleotide for use according to any of [87] to [96], wherein the mRNA encoding the NeuroD1 protein is incorporated in a drug delivery system (DDS).
[98] The polynucleotide for use according to any of [87] to [97], wherein the mRNA encoding the NeuroD1 protein is encapsulated into lipid nanoparticles.
[99] The polynucleotide for use according to any of [79] to [86], wherein said polynucleotide is DNA.
[100] The polynucleotide for use according to any of [79] to [86] and [99], wherein said polynucleotide is an expression vector comprising the polynucleotide encoding the NeuroD1 protein.
[101] The polynucleotide for use according to [100], wherein the expression vector is an adeno-associated virus vector, a retrovirus vector, a Sendai virus vector, or a lentivirus vector.
[102] The polynucleotide for use according to [101], wherein the expression vector is an adeno-associated virus vector.
[103] Use of a NeuroD1 protein or a polynucleotide encoding the NeuroD1 protein in the manufacture of a pharmaceutical composition for the treatment of penetrating branch infarction, or cerebral infarction at a subacute to chronic stage.
[104] Use of a NeuroD1 protein or a polynucleotide encoding the NeuroD1 protein in the manufacture of a pharmaceutical composition for the treatment of cerebral infarction having brain damage in a penetrating branch territory.
[105] Use according to [103] or [104], wherein the penetrating branch infarction, or the cerebral infarction having brain damage in a penetrating branch territory is cerebral infarction at a subacute to chronic stage.
[106] Use according to any of [103] to [105], wherein the penetrating branch infarction, or the cerebral infarction having brain damage in a penetrating branch territory is cerebral infarction at a chronic stage.
[107] Use according to [103], wherein the cerebral infarction at a subacute to chronic stage is cerebral infarction at a chronic stage.
[108] Use according to any of [103] to [107], wherein the polynucleotide encoding the NeuroD1 protein is a polynucleotide comprising a nucleotide sequence encoding a protein consisting of the amino acid sequence as set forth by SEQ ID NO: 8.
[109] Use according to any of [103] to [108], wherein the polynucleotide encoding the NeuroD1 protein comprises a polynucleotide having 70% or higher sequence identity to the nucleotide sequence as set forth by SEQ ID NO: 7 or 10.
[110] Use according to any of [103] to [109], wherein the polynucleotide encoding the NeuroD1 protein is a polynucleotide comprising the nucleotide sequence as set forth by SEQ ID NO: 7 or 10.
[111] Use according to any of [103] to [110], wherein said polynucleotide is mRNA encoding the NeuroD1 protein.
[112] Use according to [111], wherein the mRNA encoding the NeuroD1 protein comprises a nucleotide sequence encoding the NeuroD1 protein of positions 44 to 1114 or positions 44 to 1120 of SEQ ID NO: 11 or 13.
[113] Use according to [111] or [112], wherein the mRNA encoding the NeuroD1 protein comprises 5' UTR and/or 3' UTR.
[114] Use according to any of [111] to [113], wherein the mRNA encoding the NeuroD1 protein is mRNA comprising a polynucleotide consisting of the nucleotide sequence of positions 1 to 1231 of SEQ ID NO: 11 or 13.
[115] Use according to any of [111] to [114], wherein the mRNA encoding the NeuroD1 protein comprises a polyA sequence.
[116] Use according to [115], wherein the polyA sequence is 50 to 200 nucleotides in length.
[117] Use according to any of [111] to [116], wherein the mRNA encoding the NeuroD1 protein has a 5' cap structure.
[118] Use according to any of [111] to [117], wherein the mRNA encoding the NeuroD1 protein is mRNA containing a modified nucleoside.
[119] Use according to [118], wherein the modified nucleoside is N1-methylpseudouridine.
[120] Use according to [119], wherein the mRNA encoding the NeuroD1 protein is a polynucleotide in which some or all uridines are replaced by N1-methylpseudouridine.
[121] Use according to any of [111] to [120], wherein the mRNA encoding the NeuroD1 protein is incorporated in a drug delivery system (DDS).
[122] Use according to any of [111] to [121], wherein the mRNA encoding the NeuroD1 protein is encapsulated into lipid nanoparticles.
[123] Use according to any of [103] to [110], wherein said polynucleotide is DNA.
[124] Use according to any of [103] to [110] and [123], wherein said polynucleotide is an expression vector comprising the polynucleotide encoding the NeuroD1 protein.
[125] Use according to [124], wherein the expression vector is an adeno-associated virus vector, a retrovirus vector, a Sendai virus vector, or a lentivirus vector.
[126] Use according to [125], wherein the expression vector is an adeno-associated virus vector.
[127] The pharmaceutical composition according to [50], wherein the lipid nanoparticle comprises di(heptadecan-9-yl) 8,8'-{[1-(2-hydroxyethyl)piperidin-4-yl]azanediyl}di(octanoate) or a salt thereof as a cationic lipid.
[128] The pharmaceutical composition according to [50], wherein the lipid nanoparticle comprises bis(2-nonylundecyl) 7,7'-[({[1-(N,N-dimethylglycyl)piperidin-4-yl]oxy}carbonyl)azanediyl]di(heptanoate) or a salt thereof as a cationic lipid.
[129] The pharmaceutical composition according to [127] or [128], wherein the lipid nanoparticle further comprises DSPC, cholesterol, and DMG-PEG2000.
[130] The method according to [74], wherein the lipid nanoparticle comprises di(heptadecan-9-yl) 8,8'-{[1-(2-hydroxyethyl)piperidin-4-yl]azanediyl}di(octanoate) or a salt thereof as a cationic lipid.
[131] The method according to [74], wherein the lipid nanoparticle comprises bis(2-nonylundecyl) 7,7'-[({[1-(N,N-dimethylglycyl)piperidin-4-yl]oxy}carbonyl)azanediyl]di(heptanoate) or a salt thereof as a cationic lipid.
[132] The method according to [130] or [131], wherein the lipid nanoparticle further comprises DSPC, cholesterol, and DMG-PEG2000.
[133] The polynucleotide for use according to [98], wherein the lipid nanoparticle comprises di(heptadecan-9-yl) 8,8'-{[1-(2-hydroxyethyl)piperidin-4-yl]azanediyl}di(octanoate) or a salt thereof as a cationic lipid.
[134] The polynucleotide for use according to [98], wherein the lipid nanoparticle comprises bis(2-nonylundecyl) 7,7'-[({[1-(N,N-dimethylglycyl)piperidin-4-yl]oxy}carbonyl)azanediyl]di(heptanoate) or a salt thereof as a cationic lipid.
[135] The polynucleotide for use according to [133] or [134], wherein the lipid nanoparticle further comprises DSPC, cholesterol, and DMG-PEG2000.
[136] Use according to [122], wherein the lipid nanoparticle comprises di(heptadecan-9-yl) 8,8'-{[1-(2-hydroxyethyl)piperidin-4-yl]azanediyl}di(octanoate) or a salt thereof as a cationic lipid.
[137] Use according to [122], wherein the lipid nanoparticle comprises bis(2-nonylundecyl) 7,7'-[({[1-(N,N-dimethylglycyl)piperidin-4-yl]oxy}carbonyl)azanediyl]di(heptanoate) or a salt thereof as a cationic lipid.
[138] Use according to [136] or [137], wherein the lipid nanoparticle further comprises DSPC, cholesterol, and DMG-PEG2000.

The present specification encompasses the contents disclosed in Japanese Patent Applications No. 2022-106308, 2022-211994, and 2023-041345, of which the present application claims priority.

### [Advantageous Effects of Invention]

According to the present invention, a nonhuman primate animal model of cerebral infarction having basal ganglia damage, thalamus damage, and internal capsule damage can be produced. This nonhuman primate animal model of cerebral infarction is particularly useful as a nonhuman primate animal model of cerebral infarction at a subacute to chronic stage, penetrating branch infarction, or cerebral infarction having brain damage in a penetrating branch territory. The present invention can also provide a therapeutic agent, a pharmaceutical composition for treatment, or a treatment method particularly useful in the treatment of penetrating branch infarction, or cerebral infarction having brain damage in a penetrating branch territory.

### [Brief Description of Drawings]

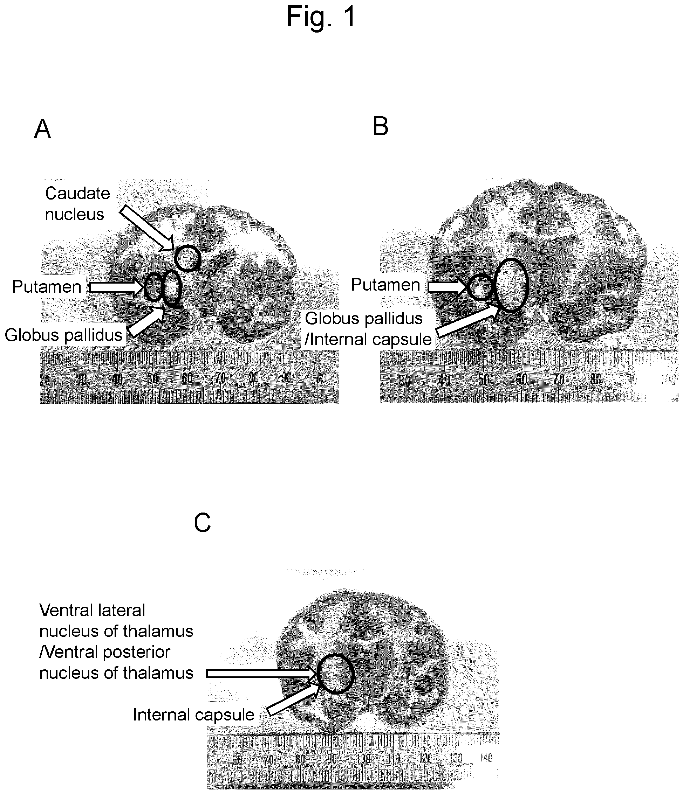

[Figure 1] Figure 1 (A to C) presents photographs showing results of TTC staining of the coronal section of the brain of a cynomolgus macaque individual treated with endothelin-1 in Study Experiment 5 of Example 1. A shows damaged areas in the caudate nucleus, the putamen, and the globus pallidus, B shows damaged areas in the putamen, the globus pallidus, and the internal capsule, and C shows damaged areas in the ventral lateral nucleus of the thalamus and the ventral posterior nucleus of the thalamus, and the internal capsule. The round frames depict the damaged areas.

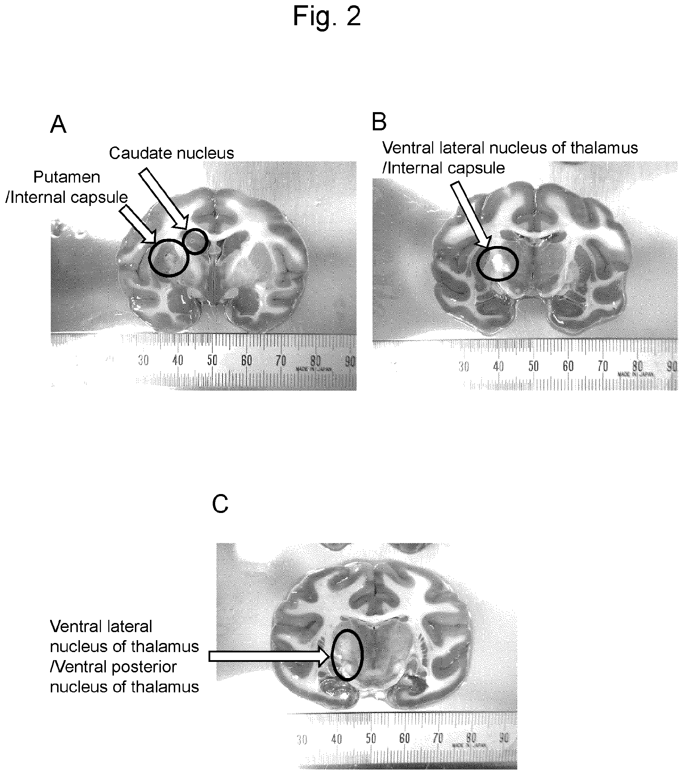

[Figure 2] Figure 2 (A to C) presents photographs showing results of TTC staining of the coronal section of the brain of a cynomolgus macaque individual treated with endothelin-1 in Study Experiment 6 of Example 1. A shows damaged areas in the caudate nucleus, the putamen, and the internal capsule, B shows damaged areas in the internal capsule and the ventral lateral nucleus of the thalamus, and C shows damaged areas in the ventral lateral nucleus of the thalamus and the ventral posterior nucleus of the thalamus. The round frames depict the damaged areas.

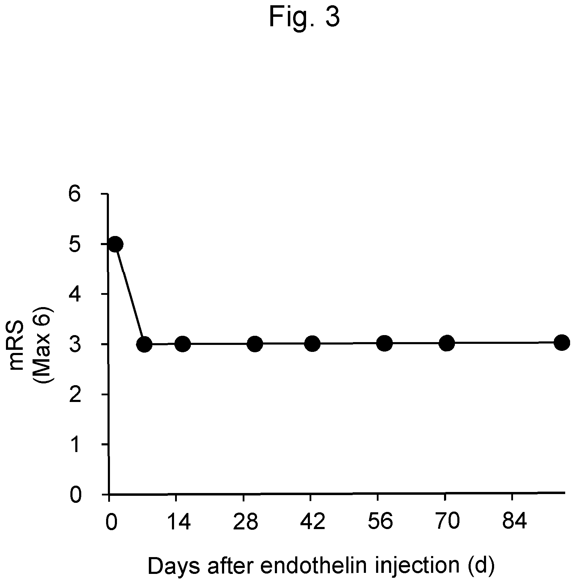

[Figure 3] Figure 3 shows results of mRS evaluation over time of a cynomolgus macaque individual treated with endothelin-1 in Study Experiment 6 of Example 1. The abscissa depicts days after endothelin-1 injection, and the ordinate depicts the value of mRS (maximum value: 6).

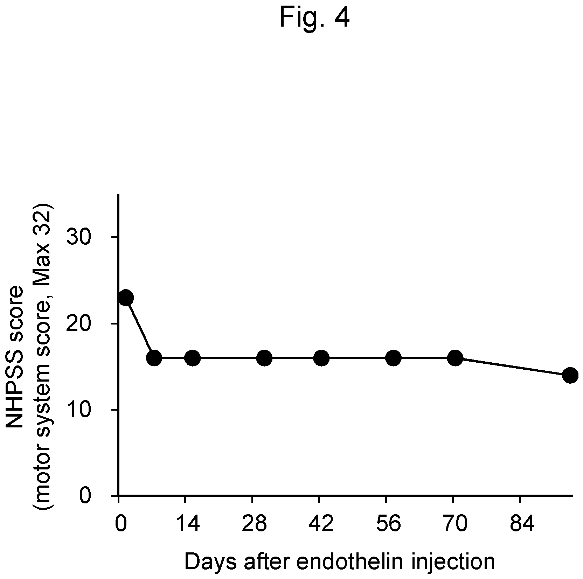

[Figure 4] Figure 4 shows results of NHPSS motor system scoring evaluation over time of a cynomolgus macaque individual treated with endothelin-1 in Study Experiment 6 of Example 1. The abscissa depicts days after endothelin-1 injection, and the ordinate depicts the value of NHPSS (motor system score) (maximum value: 32).

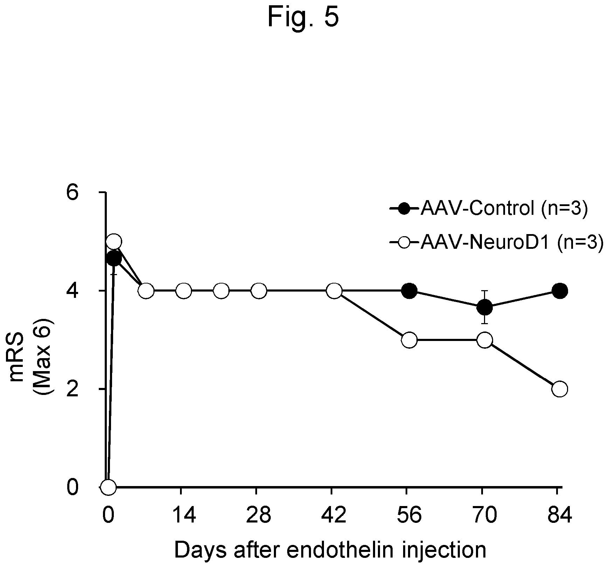

[Figure 5] Figure 5 shows results of mRS evaluation of change over time in motor dysfunction after endothelin-1 treatment of an AAV-NeuroD1 group (AAV-NeuroD1) and a control group (AAV-Control) in Example 5. The abscissa depicts days after endothelin-1 injection, and the ordinate depicts the value of mRS (maximum value: 6). The open circle and the filled circle represent average values of mRS at each time point of evaluation of the AAV-NeuroD1 group and the control group, respectively. The error bar represents standard error of the mean (SEM). The AAV vector was administered on day 21 after endothelin-1 injection.

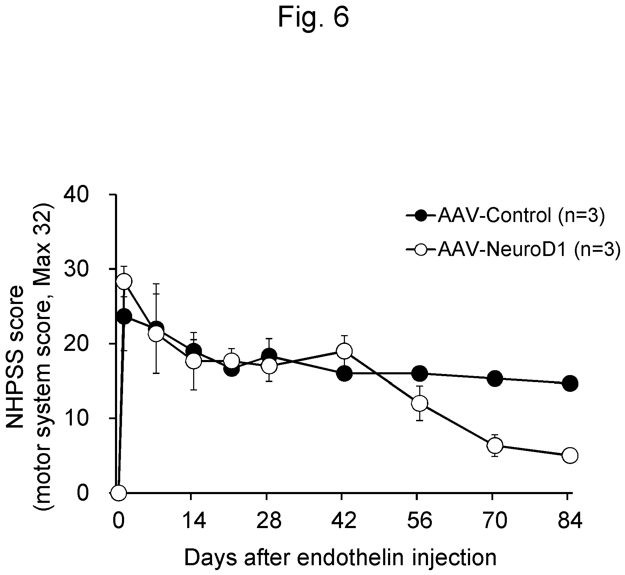

[Figure 6] Figure 6 shows results of NHPSS motor system scoring evaluation of change over time in motor dysfunction after endothelin-1 treatment of an AAV-NeuroD1 group (AAV-NeuroD1) and a control group (AAV-Control) in Example 5. The abscissa depicts days after endothelin-1 injection, and the ordinate depicts the value of NHPSS (motor system score) (maximum value: 32). The open circle and the filled circle represent average values of NHPSS (motor system score) at each time point of evaluation of the AAV-NeuroD1 group and the control group, respectively. The error bar represents standard error of the mean (SEM). The AAV vector was administered on day 21 after endothelin-1 injection.

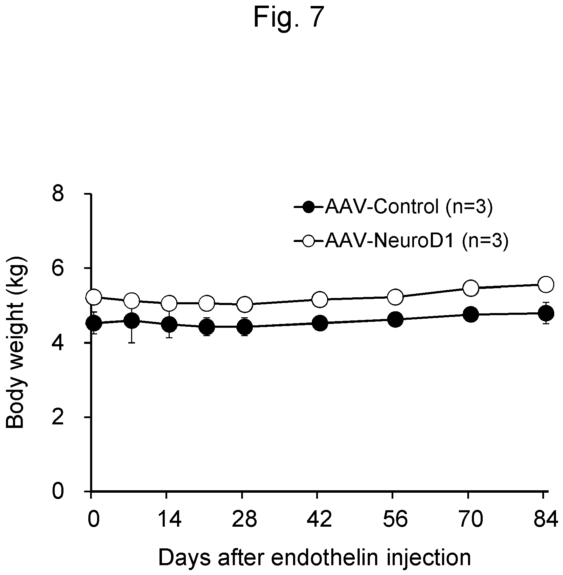

[Figure 7] Figure 7 shows the time course of the body weights of an AAV-NeuroD1 group (AAV-NeuroD1) and a control group (AAV-Control) in Example 5. The abscissa depicts days after endothelin-1 injection, and the ordinate depicts the body weight. The open circle and the filled circle represent average values of the body weights at each time point of evaluation of the AAV-NeuroD1 group and the control group, respectively. The error bar represents standard error of the mean (SEM).

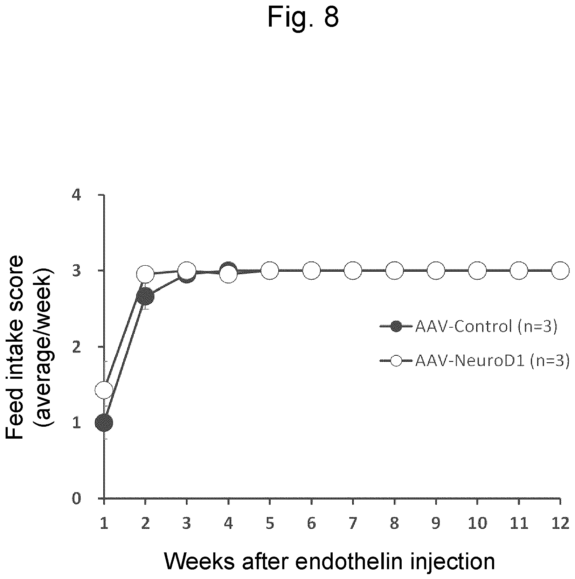

[Figure 8] Figure 8 shows the time course of the feed intake behaviors of an AAV-NeuroD1 group (AAV-NeuroD1) and a control group (AAV-Control) in Example 5. The abscissa depicts weeks after endothelin-1 injection, and the ordinate depicts the feed intake score. The open circle and the filled circle represent average values of the feed intake scores at each time point of evaluation of the AAV-NeuroD1 group and the control group, respectively. The error bar represents standard error of the mean (SEM).

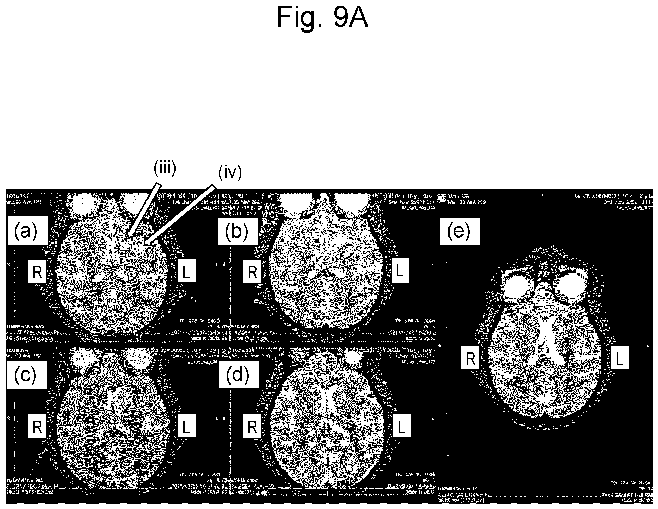

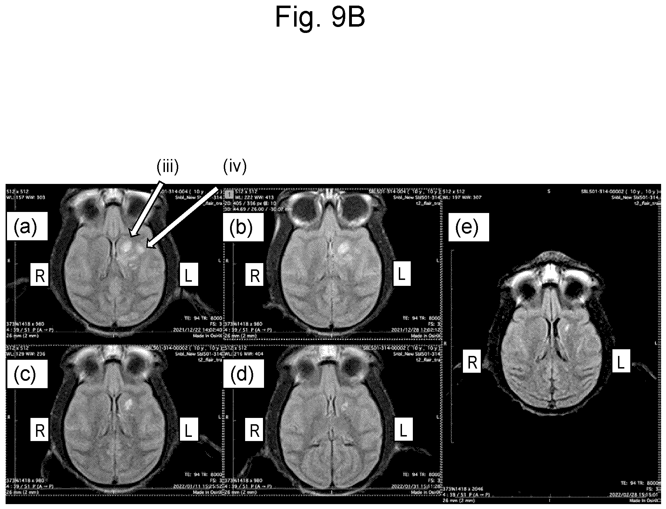

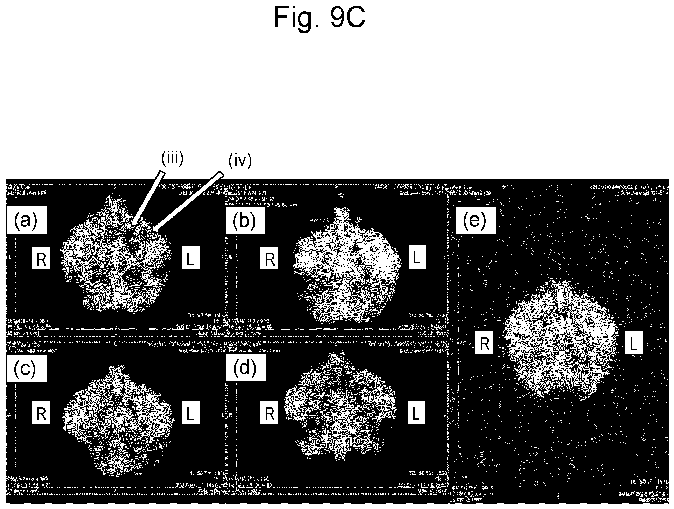

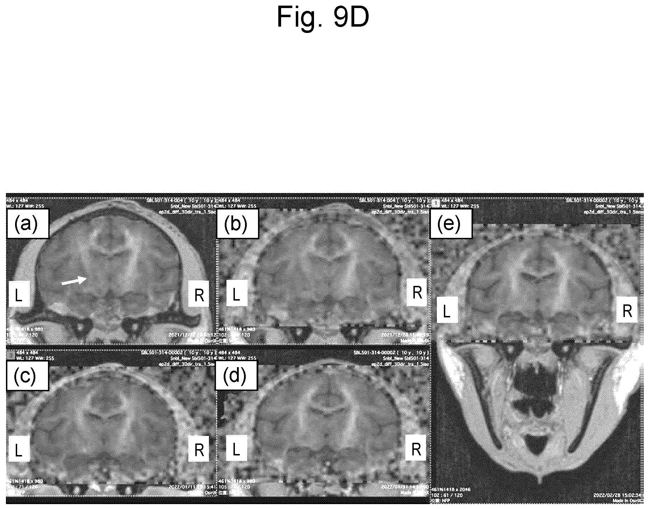

[Figure 9A] Figure 9 shows daily images of the brain of a cynomolgus macaque individual treated with endothelin-1 in Example 6. Figure 9A shows horizontal sectional T2-weighted images of Example 6(a). Figure 9A (a) to (e) show results on day 1, day 7, day 21, day 41, and day 69, respectively, after endothelin-1 injection. L represents the left side of the brain, and R represents the right side of the brain. Endothelin-1 was injected to the left side of the brain. (iii) and (iv) in the figure depict endothelin-1 injection sites (iii) and (iv) described in Example 2.
[Figure 9B] Figure 9 shows daily images of the brain of a cynomolgus macaque individual treated with endothelin-1 in Example 6. Figure 9B shows horizontal sectional FLAIR images of Example 6(a). Figure 9B (a) to (e) show results on day 1, day 7, day 21, day 41, and day 69, respectively, after endothelin-1 injection. L represents the left side of the brain, and R represents the right side of the brain. Endothelin-1 was injected to the left side of the brain. (iii) and (iv) in the figure depict endothelin-1 injection sites (iii) and (iv) described in Example 2.
[Figure 9C] Figure 9 shows daily images of the brain of a cynomolgus macaque individual treated with endothelin-1 in Example 6. Figure 9C shows horizontal sectional perfusion-weighted images of Example 6(b). Figure 9C (a) to (e) show results on day 1, day 7, day 21, day 41, and day 69, respectively, after endothelin-1 injection. L represents the left side of the brain, and R represents the right side of the brain. Endothelin-1 was injected to the left side of the brain. (iii) and (iv) in the figure depict endothelin-1 injection sites (iii) and (iv) described in Example 2.
[Figure 9D] Figure 9 shows daily images of the brain of a cynomolgus macaque individual treated with endothelin-1 in Example 6. Figure 9D shows coronal sectional DTI of Example 6(c). Figure 9D (a) to (e) show results on day 1, day 7, day 21, day 41, and day 69, respectively, after endothelin-1 injection. L represents the left side of the brain, and R represents the right side of the brain. Endothelin-1 was injected to the left side of the brain.

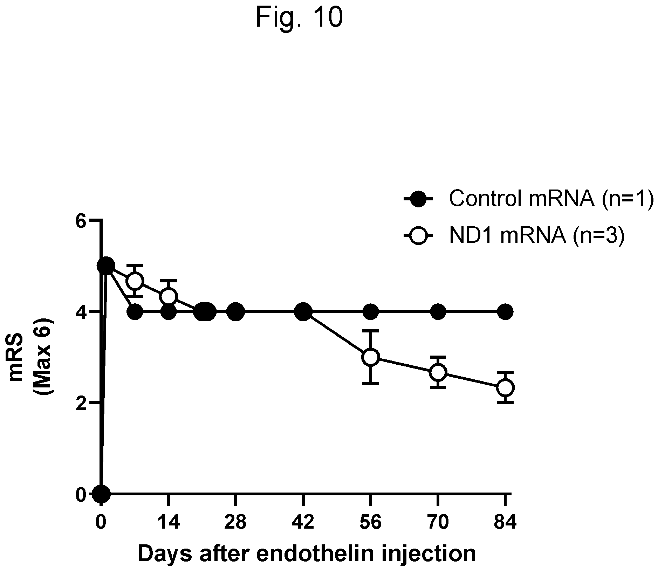

[Figure 10] Figure 10 shows results of mRS evaluation of daily change in motor dysfunction after endothelin-1 treatment in a NeuroD1 mRNA-LNP group (ND1 mRNA) and a control group (Control mRNA) in Example 12(a). The abscissa depicts days after endothelin-1 injection, and the ordinate depicts the value of mRS (maximum value: 6). The open circle represents an average value of mRS at each time point of evaluation of the NeuroD1 mRNA-LNP group. mRS was evaluated every 2 weeks on day 28 or later after endothelin-1 injection, but the result on day 56 indicates an average value of results of evaluating individuals on day 57, 58, or 59. The error bar represents standard error of the mean (SEM). The filled circle represents mRS at each time point of evaluation of one individual of the control group. The result on day 56 indicates the result of performing evaluation on day 58. mRNA-LNP was administered on day 21 after endothelin-1 injection.

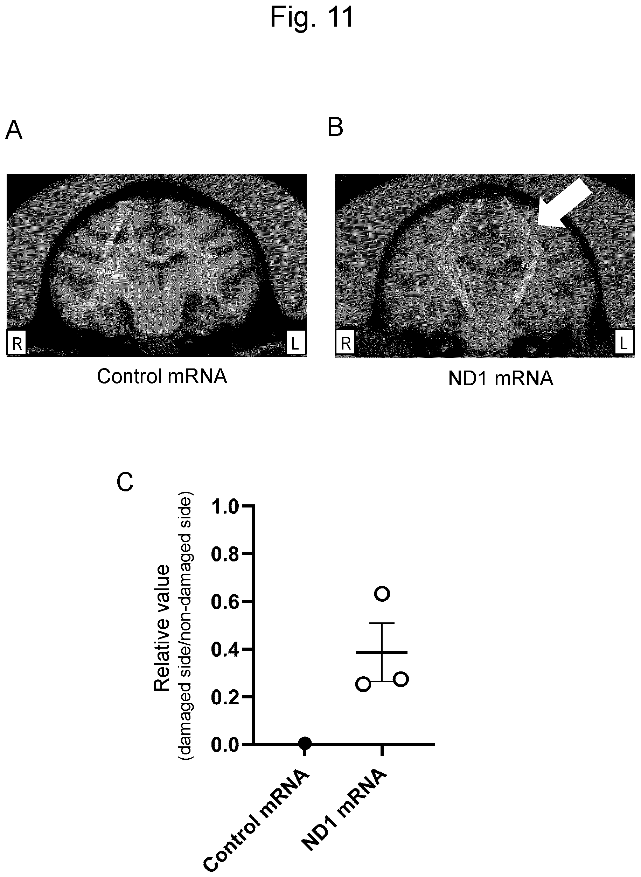

[Figure 11] Figure 11 shows results of DTI analysis in Example 12(b). A and B show diffusion tensor tractography (DTT) obtained by conducting tensor analysis on the basis of DTI images of the brain of a cynomolgus macaque individual treated with mRNA-LNP. In the figure, R represents the right side of the brain (the non-damaged side; mRNA-LNP administration absent), and L represents the left side of the brain (the damaged side; mRNA-LNP administration present). Measured FA values were visualized in the images. A shows the results on day 114 after endothelin-1 injection of a control group (Control mRNA), and B shows representative results among three individuals analyzed for FA values on day 114 to 115 after endothelin-1 injection of a NeuroD1 mRNA-LNP group (ND1 mRNA). C shows results of quantifying the FA value by DTI. The ordinate of the graph of C depicts a value (relative value) obtained by dividing the FA value on the damaged side by the FA value on the non-damaged side. The open circle represents each of the relative values of individuals of the NeuroD1 mRNA-LNP group and the bar represents an average value of them. The error bar represents standard error of the mean (SEM). The filled circle represents the relative value of one individual of the control group.

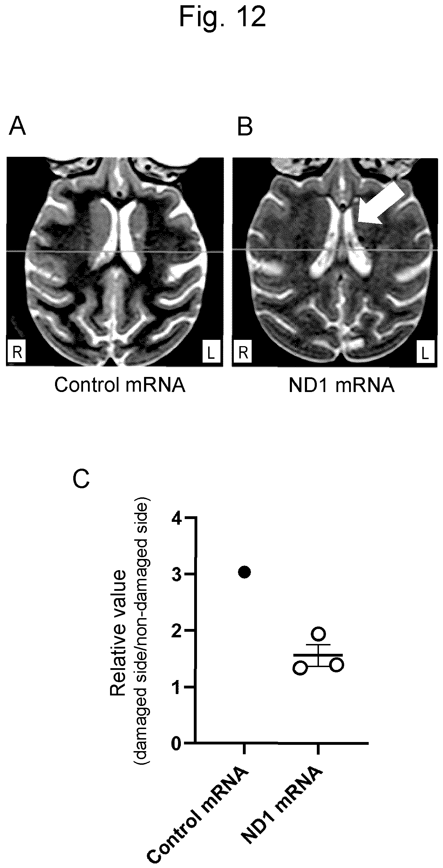

[Figure 12] Figure 12 shows results of analyzing the volume of the lateral ventricle in Example 12(c). A and B show T2-weighted images of the brain of a cynomolgus macaque individual treated with mRNA-LNP. In the figure, R represents the right side of the brain (the non-damaged side; mRNA-LNP administration absent), and L represents the left side of the brain (the damaged side; mRNA-LNP administration present). A shows the results on day 114 after endothelin-1 injection of a control group (Control mRNA), and B shows representative results among three individuals analyzed on day 114 to 115 after endothelin-1 injection of a NeuroD1 mRNA-LNP group (ND1 mRNA). C shows results of quantifying the volume of the lateral ventricle. The ordinate of the graph of C depicts a value (relative value) obtained by dividing the volume of the lateral ventricle on the damaged side by the volume of the lateral ventricle on the non-damaged side. The open circle represents each of the relative values of individuals of the NeuroD1 mRNA-LNP group and the bar represents an average value of them. The error bar represents standard error of the mean (SEM). The filled circle represents the relative value of one individual of the control group.

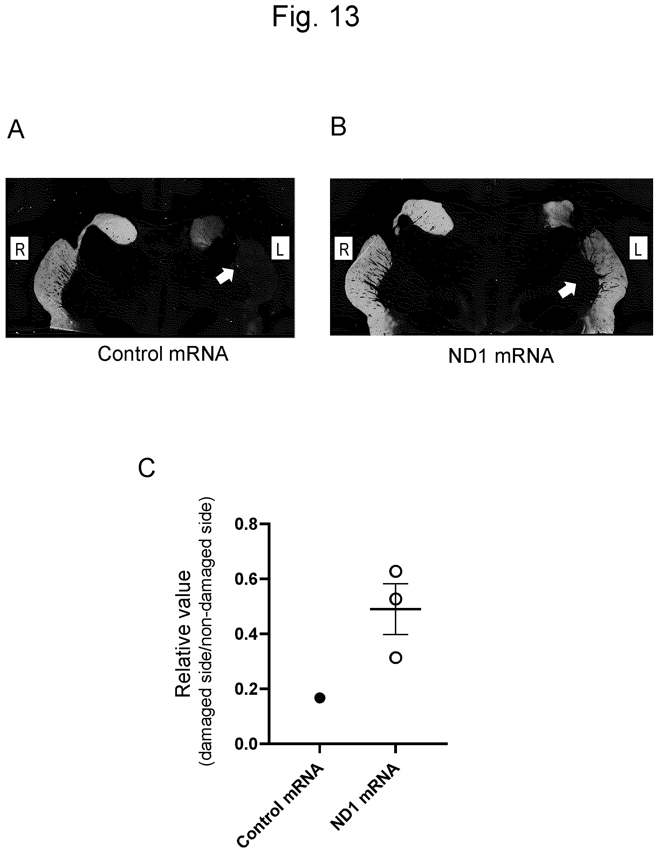

[Figure 13] Figure 13 shows results of expression analysis of dopamine transporter in Example 12 (d). A and B are photographs showing results of immunostaining of dopamine transporter of a coronal slice of brain of a cynomolgus macaque individual treated with mRNA-LNP. In the figure, R represents the right side of the brain (the non-damaged side; mRNA-LNP administration absent), and L represents the left side of the brain (the damaged side; mRNA-LNP administration present). The arrow indicates the corpus striatum. A shows the results on day 118 after endothelin-1 treatment (brain ischemia induction) of a control group (Control mRNA), and B shows representative results among three individuals subjected to immunostaining on day 119 to 122 after endothelin-1 injection of a NeuroD1 mRNA-LNP group (ND1 mRNA). C shows results of analyzing the expression intensity of dopamine transporter. The ordinate of the graph of C depicts a value (relative value) obtained by dividing the fluorescent staining intensity on the damaged side by the fluorescent staining intensity on the non-damaged side. The open circle represents each of the relative values of individuals of the NeuroD1 mRNA-LNP group and the bar represents an average value of them. The error bar represents standard error of the mean (SEM). The filled circle represents the relative value of one individual of the control group.

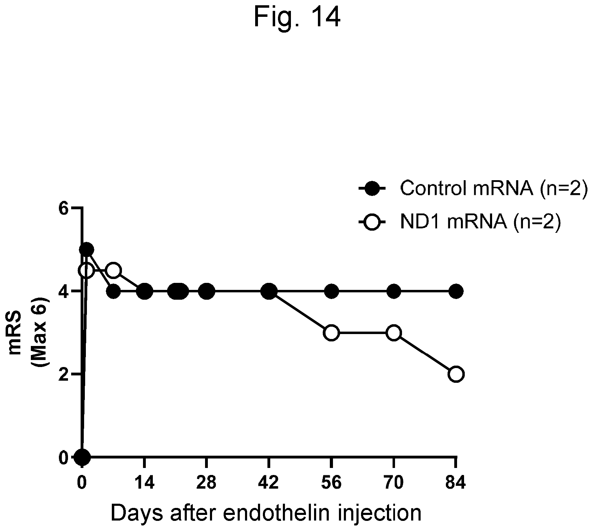

[Figure 14] Figure 14 shows results of mRS evaluation of daily change in motor dysfunction after endothelin-1 treatment in a NeuroD1 mRNA-LNP (ND1-2-L7) group (ND1 mRNA) and a control group (Control mRNA) in Example 12(a). The abscissa depicts days after endothelin-1 injection, and the ordinate depicts the value of mRS (maximum value: 6). The open circle represents an average value of mRS at each time point of evaluation of the NeuroD1 mRNA-LNP group. mRS was evaluated every 2 weeks on day 28 or later after endothelin-1 injection. The filled circle represents mRS at each time point of evaluation of one individual of the control group. The result on day 70 indicates an average value of results of performing evaluation on day 69 or 70. mRNA-LNP was administered on day 21 after endothelin-1 injection.

### [Description of Embodiments]

Hereinafter, the present invention will be described in detail.

### 1. Production of nonhuman primate animal model

The present invention provides a method for producing a nonhuman primate animal model of cerebral infarction, comprising administering endothelin to basal ganglia and thalamic region of a nonhuman primate, and thereby inducing basal ganglia damage, thalamus damage, and internal capsule damage (hereinafter, also referred to as the "animal model production method of the present invention").

### a) Nonhuman primate

In the present specification, the "nonhuman primate" refers to an arbitrary primate (the order *Primates*) animal except for a human. The nonhuman primate (monkey) used in the present invention is preferably a nonhuman primate capable of being evaluated by motor dysfunction scoring using a modified Rankin scale (mRS; see Table 1 mentioned later), a non-human primate stroke scale (NHPSS (Ben R et al., J. Neurol. Res., vol. 25 (1), pp. 68-78, 2003); see Table 2 mentioned later), or the like regarding brain dysfunction including basal ganglia damage, thalamus damage, and internal capsule damage. Examples of the nonhuman primate to which endothelin is administered according to the present invention can include apes, monkeys belonging to the family *Cercopithecidae*, and monkeys belonging to the family *Cebidae.* The "ape" refers to a monkey belonging to the family *Hominoidea* of the order *Primates* (primate). Examples of the ape can include chimpanzees, gorillas, orangutans, bonobos, and gibbons. Examples of the monkey belonging to the family *Cercopithecidae* can include, but are not limited to, monkeys belonging to the genus *Macaca* (macaque monkeys) such as Japanese monkeys, rhesus macaques, cynomolgus macaques, Taiwan monkeys, pigtailed macaques, and bonnet macaques, monkeys belonging to the genus *Papio* (baboons) such as hamadryas baboons and gelada baboons, and monkeys belonging to the genus *Mandrillus* such as mandrills and drills. Examples of the monkey belonging to the family *Cebidae* can include, but are not limited to, monkeys belonging to the genus *Cebus* (capuchins) such as tufted capuchins, monkeys belonging to the genus *Saimiri* (squirrel monkeys) such as common squirrel monkeys, and monkeys belonging to the genus *Callithrix* (marmosets) such as common marmosets. In one embodiment, the nonhuman primate for use in the animal model production method of the present invention is a cynomolgus macaque. In one embodiment, the nonhuman primate for use in the animal model production method of the present invention is a cynomolgus macaque 3 years of age or older. The nonhuman primate for use in the animal model production method of the present invention may be a male or a female. In one embodiment, a male is preferred. In one embodiment, the nonhuman primate for use in the animal model production method of the present invention is a male cynomolgus macaque.

The "cynomolgus macaque" (*Macaca fascicularis*) is a primate that is classified into the genus *Macaca* of the family *Cercopithecidae* of the order *Primates* of the class *Mammalia.* The cynomolgus macaque bipeds, as in a human. Hence, the cynomolgus macaque is very similar in vascular tracking to a human in such a way that the internal carotid artery is largely curved. Furthermore, the cynomolgus macaque has a high-order function of the brain, and not only a motor function but a level of consciousness and a cognitive function, such as wariness over humans, can be observed.

### b) Endothelin

The "endothelin" (mature form) is a peptide that consists of 21 amino acid residues and has two disulfide bonds in the molecule. The endothelin is formed through the processing of a precursor consisting of 203 amino acid residues. The endothelin includes three types of peptide isoforms, endothelin-1, endothelin-2, and endothelin-3, which are encoded by different genes and are similar in amino acid sequence and structure. Most mammals have these three endothelins, and functions of these three endothelins are conserved among organism species. It is further known that the amino acid sequence of endothelin-1 is highly conserved among organism species. The three endothelins have a transient vasodilatory effect and a persistent vasoconstrictive effect subsequent thereto. It is known that ischemia can be induced by the craniotomy of a rat followed by the administration of endothelin to the surface of the middle cerebral artery (Non Patent Literature 1). The endothelin used in the present invention is, for example, endothelin-1 or endothelin-2. In one embodiment, the endothelin to be administered to the nonhuman primate is human endothelin-1 or human endothelin-2. The endothelin used in the present invention is not limited by its sequence as long as the endothelin has endothelin activity (vasoconstrictive effect). For example, the endothelin-1 may be, for example, a peptide comprising an amino acid sequence having 90% or higher, preferably 95% or higher sequence identity to the amino acid sequence as set forth by SEQ ID NO: 9, more preferably the amino acid sequence as set forth by SEQ ID NO: 9.

### c) Administration of endothelin to basal ganglia and thalamic region of nonhuman primate and induction of brain damage

In the present specification, the "basal ganglia and thalamic region" refers to the region consisting of the basal ganglia and the thalamus. The basal ganglia are a cluster of nerve nuclei constituted by the corpus striatum, the globus pallidus, the subthalamic nucleus, and the substantia nigra. The basal ganglia form the loop circuits with the cerebral cortex, together with the cerebellum, and control the activity of the cerebral cortex, particularly, the frontal lobe. The corpus striatum consists of the caudate nucleus and the putamen, and the internal capsule is present therebetween. The globus pallidus is divided to an external segment and an internal segment. The substantia nigra consists of a reticular part and a compact part. Since the putamen and the globus pallidus, which together look like a convex lens, are called the lenticular nucleus. The basal ganglia are divided to the caudate nucleus and the lenticular nucleus (the putamen and the globus pallidus) by the internal capsule, and the thalamus resides on the inner side from the basal ganglia. The basal ganglia are involved in daily life and habit. The thalamus is made up of two symmetric structures positioned near the center of cerebral hemisphere and above the midbrain, and constituted by many nerve nuclei. The left and right halves of the thalamus can each be divided to a lateral part and a medial part. The lateral part consists of the ventral posterior nucleus, the ventral lateral nucleus, the ventral anterior nucleus, the lateral dorsal nucleus, the lateral posterior nucleus, the pulvinar, and the lateral geniculate nucleus of the thalamus. The medial part consists of a pronuclear group, a medial nuclear group, and the medial geniculate nucleus of the thalamus. The thalamus is responsible for relaying sensory input of vision, hearing, somatosensor, and the like to the cerebral cortex. Also, information on motor regulation is also conveyed from the cerebellum or the basal ganglia via the thalamus to a motor-related region.

In the animal model production method of the present invention, endothelin is administered to the basal ganglia and thalamic region of a nonhuman primate, thereby inducing brain damage. The endothelin has a strong vasoconstrictive effect, and the administration of the endothelin can constrict vascular vessels at and near an administration site, induces ischemia, and induces an infarct area (damaged area). In the present invention, the phrase "inducing damage" means that the administration of endothelin induces ischemia at or near an administration site so that neuronal necrosis occurs to injure a function of the necrotized site. In one embodiment, endothelin is administered to the basal ganglia and thalamic region of a nonhuman primate, thereby inducing basal ganglia damage, thalamus damage, and internal capsule damage. In the present invention, the "administration of endothelin to basal ganglia and thalamic region" means the administration of endothelin targeting the basal ganglia and thalamic region (i.e., such that the endothelin reaches the basal ganglia and thalamic region). In the present invention, the phrase "administering endothelin" to, for example, a nerve nucleus, also means administering endothelin targeting the nerve nuclei (i.e., such that the endothelin reaches the nerve nucleus).

In the present specification, the "administration site" refers to a location that is defined by three dimensional coordinates consisting of a position on the surface of the skull (epicranial), in administering (preferably, injecting) endothelin to basal ganglia and thalamic region, and a depth from the pachymeninx immediately beneath the epicranial administration position.

In one embodiment, the animal model production method of the present invention comprises administering endothelin to nerve nuclei selected from the group consisting of the putamen, the caudate nucleus, the globus pallidus, the ventral lateral nucleus of the thalamus, and the ventral posterior nucleus of the thalamus in the basal ganglia and thalamic region. In one embodiment, the animal model production method of the present invention comprises administering endothelin to at least one, at least two, at least three, at least four, or all of five (the putamen, the caudate nucleus, the globus pallidus, the ventral lateral nucleus of the thalamus, and the ventral posterior nucleus of the thalamus) nerve nuclei selected from the group consisting of the putamen, the caudate nucleus, the globus pallidus, the ventral lateral nucleus of the thalamus, and the ventral posterior nucleus of the thalamus in the basal ganglia and thalamic region of the nonhuman primate. In one embodiment, the animal model production method of the present invention comprises administering endothelin to at least one, at least two, at least three, at least four, or at least five administration sites (typically, injection sites) targeting the basal ganglia and thalamic region of the nonhuman primate. In one embodiment, the animal model production method of the present invention comprises administering endothelin to a total of at least one, at least two, at least three, at least four, or at least five administration sites (typically, injection sites) targeting at least one, at least two, at least three, at least four, or all of five nerve nuclei selected from the group consisting of the putamen, the caudate nucleus, the globus pallidus, the ventral lateral nucleus of the thalamus, and the ventral posterior nucleus of the thalamus in the basal ganglia and thalamic region of the nonhuman primate. In one embodiment, endothelin may be administered to the basal ganglia, the ventral lateral nucleus of the thalamus, and the ventral posterior nucleus of the thalamus of the nonhuman primate, thereby inducing brain damage. In one embodiment, endothelin may be administered to the putamen, the globus pallidus, and the thalamus (e.g., the ventral lateral nucleus of the thalamus and the ventral posterior nucleus of the thalamus) of the nonhuman primate, thereby inducing brain damage. In one embodiment, endothelin may be administered to the putamen, the caudate nucleus, the globus pallidus, and the thalamus (e.g., the ventral lateral nucleus of the thalamus and the ventral posterior nucleus of the thalamus) of the nonhuman primate, thereby inducing brain damage.

In one embodiment, the animal model production method of the present invention comprises determining administration sites so as to target the basal ganglia and thalamic region of the nonhuman primate, for example, at least one, at least two, at least three, at least four, or all of five nerve nuclei selected from the group consisting of the putamen, the caudate nucleus, the globus pallidus, the ventral lateral nucleus of the thalamus, and the ventral posterior nucleus of the thalamus, and administering endothelin to the administration sites.

In one embodiment, the animal model production method of the present invention comprises determining administration sites so as to target all of five nerve nuclei of the group consisting of the putamen, the caudate nucleus, the globus pallidus, the ventral lateral nucleus of the thalamus, and the ventral posterior nucleus of the thalamus in the basal ganglia and thalamic region of the nonhuman primate, and administering endothelin to the administration sites.

In one embodiment, the animal model production method of the present invention may be a method for producing a nonhuman primate animal model of cerebral infarction, comprising administering endothelin to basal ganglia and thalamic region of a nonhuman primate, and thereby inducing basal ganglia damage, thalamus damage, and internal capsule damage, the method comprising the steps of:
(a) determining administration sites so as to target at least four nerve nuclei selected from the group consisting of the putamen, the caudate nucleus, the globus pallidus, the ventral lateral nucleus of the thalamus, and the ventral posterior nucleus of the thalamus in the basal ganglia and thalamic region; and
(b) administering endothelin to the administration sites.

In one embodiment, the animal model production method of the present invention may be a method for producing a nonhuman primate animal model of cerebral infarction, comprising administering endothelin to basal ganglia and thalamic region of a nonhuman primate, and thereby inducing basal ganglia damage, thalamus damage, and internal capsule damage, the method comprising the steps of:
(a) determining administration sites so as to target all of five nerve nuclei of the group consisting of the putamen, the caudate nucleus, the globus pallidus, the ventral lateral nucleus of the thalamus, and the ventral posterior nucleus of the thalamus in the basal ganglia and thalamic region; and
(b) administering endothelin to the administration sites.

In one embodiment, the animal model production method of the present invention may be a method for producing a nonhuman primate animal model of cerebral infarction, comprising administering endothelin to basal ganglia and thalamic region of a nonhuman primate, and thereby inducing basal ganglia damage, thalamus damage, and internal capsule damage, the method comprising the steps of:
(a) determining at least three or four administration sites so as to target at least four or all of five nerve nuclei selected from the group consisting of the putamen, the caudate nucleus, the globus pallidus, the ventral lateral nucleus of the thalamus, and the ventral posterior nucleus of the thalamus in the basal ganglia and thalamic region; and
(b) administering endothelin to the administration sites.

The endothelin administration sites (typically, injection sites) can be appropriately determined by those skilled in the art using, for example, known information, an image in the brain obtained by computed tomography (CT) or magnetic resonance imaging (MRI), or a method with Bregma as a base point described in the present specification.

In one embodiment, the positions of the endothelin administration sites (typically, injection sites) to the basal ganglia and thalamic region, for example, nerve nuclei such as the putamen, the caudate nucleus, the globus pallidus, the ventral lateral nucleus of the thalamus, and the ventral posterior nucleus of the thalamus, can be determined with Bregma which is the intersection of the sagittal and coronal sutures on the anterior surface of the skull as a base point (B: 0 mm, ML coordinate: 0 mm; median). More specifically, the positions of the administration sites (typically, injection sites) can be defined by coordinates as set forth by distance B along the anteroposterior axis and distance L (left) or R (right) along the lateral axis on a virtual horizontal plane from Bregma as a base point, which designate an epicranial administration position (which corresponds to a hole in the skull for administration), and a depth (vertical distance) D from the pachymeninx immediately beneath the epicranial administration position. The distance B along the anteroposterior axis represents an anterior distance by a positive numeric value and a posterior distance by a negative numeric value. In the present invention, typically, a needle is inserted vertically downward (i.e., in the direction of gravitational force) from the epicranial administration position (the position of a hole in the skull) designated by the distances along the anteroposterior and lateral axes, and endothelin can be administered into the brain through the needle. Thus, in the present invention, the depth D which defines the administration sites refers to a vertically downward depth (i.e., in the direction of gravitational force) in the brain from the pachymeninx immediately beneath the epicranial administration position. In the present invention, use of the bregma as a base point for the administration sites (typically, injection sites) can reduce variations among individuals and enables the administration sites in predetermined nerve nuclei to be set to more stable positions. In the present invention, the positions of the administration sites are determined with the nonhuman primate head fixed. For example, a stereotaxic instrument can be used in the fixation of the nonhuman primate head. Specifically, ear bars of the stereotaxic instrument are inserted to both the ears of the nonhuman primate to fix the head so as not to move laterally. Then, a bar for the upper jaw and a bar for the orbit are used to fix the head so as not to move up and down. The fixation of the head by the insertion of ear bars allows for reproducible stereotactic placement of the nonhuman primate head. The nonhuman primate head is thereby fixed such that the top of the head faces upward, a straight line that connects both the ears becomes horizontal, and the face faces front.

What region, for example, what nerve nucleus, endothelin is administered to through a predetermined position of an administration site (typically, an injection site) determined with Bregma as a base point (i.e., what region or what nerve nucleus is targeted by administration) may be confirmed in advance. The region or the nerve nucleus to be targeted by administration as to a predetermined position of an administration site can be determined, for example, by injecting a staining solution to the administration site, and examining the stained region in the brain immediately after injection. Examples of the staining solution used include, but are not limited to, Evans Blue (CAS No: 314-13-6). Endothelin may be administered to the administration site confirmed to target the region or the nerve nucleus of interest by administration.

In one embodiment, the endothelin administration sites may include a total of at least three (e.g., three, four, or five) administration sites positioned at (i) B: 0 to 4 mm, L: 7 to 9 mm, and D: 15 to 21 mm, and (ii) B: 4 to 14 mm, L: 3 to 14 mm, and D: 12 to 23 mm from Bregma as a base point. These administration sites are particularly suitable for application to a monkey belonging to the genus *Macaca,* for example, a cynomolgus macaque, but are not limited by the application to the organism species. The administration of endothelin to the respective administration sites may be simultaneously performed, may be sequentially performed, or may be performed in a plurality of portions. The administration of endothelin to one administration site may be performed only once or may be performed a plurality of times. For example, endothelin may be administered to a certain administration site and may then be administered to another administration site and/or the same administration site after a lapse of given time. In one embodiment, endothelin may be administered to a certain administration site and may be further administered to another administration site and/or the same administration site on the next day or later.

In one embodiment, the animal model production method of the present invention is a method comprising administering endothelin to the endothelin administration sites described in the present specification with Bregma as a base point in a cynomolgus macaque, and thereby inducing basal ganglia damage, thalamus damage, and internal capsule damage. In one embodiment, the animal model production method of the present invention comprises designating an epicranial administration position of a cynomolgus macaque, and administering endothelin to an administration site immediately beneath the administration position, wherein the endothelin administration site includes the administration sites described in the present specification with Bregma as a base point. In these embodiments, the animal model production method of the present invention may be a method for producing a nonhuman primate animal model of cerebral infarction at a subacute to chronic stage, for example, at a chronic stage (e.g., penetrating branch infarction, or cerebral infarction having brain damage in a penetrating branch territory).

When a cynomolgus macaque is taken as an example, the administration site positioned at B: 0 to 4 mm, L: 7 to 9 mm, and D: 15 to 21 mm from Bregma as a base point targets the thalamus. Likewise, the administration site positioned at B: 4 to 14 mm, L: 3 to 14 mm, and D: 12 to 23 mm from Bregma as a base point targets the basal ganglia (particularly, the corpus striatum and the globus pallidus).

In one embodiment, the endothelin administration sites may include administration sites (a total of at least three sites, for example, three, four, or five administration sites) respectively positioned at (i) B: 1 to 3 mm (e.g., 2 mm), L: 7 to 9 mm (e.g., 8 mm), and D: 18 to 20 mm (e.g., 19 mm), (ii) B: 4 to 6 mm (e.g., 5 mm), L: 7 to 9 mm (e.g., 8 mm), and D: 18 to 20 mm (e.g., 19 mm), and (iii) B: 8 to 12 mm (e.g., 9 mm, or 11 mm), L: 4 to 13 mm (e.g., 5 mm, 8 mm, 12 mm, or 13 mm), and D: 12 to 20 mm (e.g., 13 mm, 15 mm, or 19 mm) from Bregma as a base point.

In one embodiment, the endothelin administration sites may include administration sites (a total of at least three sites, for example, three or four administration sites) respectively positioned at (i) B: 1 to 3 mm (e.g., 2 mm), L: 7 to 9 mm (e.g., 8 mm), and D: 18 to 20 mm (e.g., 19 mm), (ii) B: 4 to 6 mm (e.g., 5 mm), L: 7 to 9 mm (e.g., 8 mm), and D: 18 to 20 mm (e.g., 19 mm), and (iii) B: 8 to 12 mm (e.g., 9 mm or 11 mm), L: 7 to 14 mm (e.g., 8 mm or 13 mm), and D: 18 to 20 mm (e.g., 19 mm) from Bregma as a base point.

In a more preferred embodiment, the endothelin administration sites may include administration sites (a total of at least four administration sites) respectively positioned at (i) B: 1 to 3 mm (e.g., 2 mm), L: 7 to 9 mm (e.g., 8 mm), and D: 18 to 20 mm (e.g., 19 mm), (ii) B: 4 to 6 mm (e.g., 5 mm), L: 7 to 9 mm (e.g., 8 mm), and D: 18 to 20 mm (e.g., 19 mm), (iii) B: 8 to 10 mm (e.g., 9 mm), L: 4 to 6 mm (e.g., 5 mm), and D: 12 to 14 mm (e.g., 13 mm), and (iv) B: 8 to 10 mm (e.g., 9 mm), L: 11 to 13 mm (e.g., 12 mm), and D: 14 to 16 mm (e.g., 15 mm) from Bregma as a base point.

In one embodiment, the endothelin administration sites may include administration sites (a total of at least three administration sites) respectively positioned at (i) B: 1 to 3 mm (e.g., 2 mm), L: 7 to 9 mm (e.g., 8 mm), and D: 18 to 20 mm (e.g., 19 mm), (ii) B: 4 to 6 mm (e.g., 5 mm), L: 7 to 9 mm (e.g., 8 mm), and D: 18 to 20 mm (e.g., 19 mm), and (iii) B: 8 to 10 mm (e.g., 9 mm), L: 7 to 9 mm (e.g., 8 mm), and D: 18 to 20 mm (e.g., 19 mm) from Bregma as a base point.

In one embodiment, the endothelin administration sites may include administration sites (a total of at least three administration sites) respectively positioned at (i) B: 1 to 3 mm (e.g., 2 mm), L: 7 to 9 mm (e.g., 8 mm), and D: 18 to 20 mm (e.g., 19 mm), (ii) B: 4 to 6 mm (e.g., 5 mm), L: 7 to 9 mm (e.g., 8 mm), and D: 18 to 20 mm (e.g., 19 mm), and (iii) B: 10 to 12 mm (e.g., 11 mm), L: 7 to 9 mm (e.g., 8 mm), and D: 18 to 20 mm (e.g., 19 mm) from Bregma as a base point.

In a more preferred embodiment, the endothelin administration sites may include administration sites (a total of at least four administration sites) respectively positioned at (i) B: 2 mm, L: 8 mm, and D: 19 mm, (ii) B: 5 mm, L: 8 mm, and D: 19 mm, (iii) B: 9 mm, L: 5 mm, and D: 13 mm, and (iv) B: 9 mm, L: 12 mm, and D: 15 mm from Bregma as a base point.

In a more preferred embodiment, the endothelin administration sites may include administration sites (a total of at least three administration sites) respectively positioned at (i) B: 2 mm, L: 8 mm, and D: 19 mm, (ii) B: 5 mm, L: 8 mm, and D: 19 mm, and (iii) B: 9 mm, L: 8 mm, and D: 19 mm from Bregma as a base point.

In a more preferred embodiment, the endothelin administration sites may include administration sites (a total of at least three administration sites) respectively positioned at (i) B: 2 mm, L: 8 mm, and D: 19 mm, (ii) B: 5 mm, L: 8 mm, and D: 19 mm, and (iii) B: 11 mm, L: 8 mm, and D: 19 mm from Bregma as a base point.

In a further preferred embodiment, the endothelin administration sites may include administration sites (a total of at least four administration sites) respectively positioned at (i) B: 2 mm, L: 8 mm, and D: 19 mm, (ii) B: 5 mm, L: 8 mm, and D: 19 mm, (iii) B: 9 mm, L: 5 mm, and D: 13 mm, and (iv) B: 9 mm, L: 12 mm, and D: 15 mm from Bregma as a base point.

In one embodiment, the endothelin administration sites may include administration sites (a total of at least five administration sites) respectively positioned at (i) B: 1 to 3 mm (e.g., 2 mm), L: 7 to 9 mm (e.g., 8 mm), and D: 18 to 20 mm (e.g., 19 mm), (ii) B: 8 to 10 mm (e.g., 9 mm), L: 4 to 6 mm (e.g., 5 mm), and D: 12 to 14 mm (e.g., 13 mm), (iii) B: 8 to 10 mm (e.g., 9 mm), L: 12 to 14 mm (e.g., 13 mm), and D: 18 to 20 mm (e.g., 19 mm), (iv) B: 4 to 6 mm (e.g., 5 mm), L: 7 to 9 mm (e.g., 8 mm), and D: 18 to 20 mm (e.g., 19 mm), and (v) B: 8 to 10 mm (e.g., 9 mm), L: 7 to 9 mm (e.g., 8 mm), and D: 18 to 20 mm (e.g., 19 mm) from Bregma as a base point. In one embodiment, endothelin may be administered to the administration sites of (i), (ii), and (iii) in one individual and then further administered to the administration sites of (iv) and (v) on the next day.

When a cynomolgus macaque is taken as an example, the administration site positioned at B: 1 to 3 mm (e.g., 2 mm), L: 7 to 9 mm (e.g., 8 mm), and D: 18 to 20 mm (e.g., 19 mm) from Bregma as a base point targets the ventral lateral nucleus of the thalamus and the ventral posterior nucleus of the thalamus. Likewise, the administration site positioned at B: 4 to 6 mm (e.g., 5 mm), L: 7 to 9 mm (e.g., 8 mm), and D: 18 to 20 mm (e.g., 19 mm) from Bregma as a base point targets the globus pallidus. The administration site positioned at B: 8 to 10 mm (e.g., 9 mm), L: 4 to 6 mm (e.g., 5 mm), and D: 12 to 14 mm (e.g., 13 mm) from Bregma as a base point targets the caudate nucleus. The administration site positioned at B: 8 to 10 mm (e.g., 9 mm), L: 11 to 13 mm (e.g., 12 mm), and D: 14 to 16 mm (e.g., 15 mm) from Bregma as a base point targets the putamen. Likewise, the administration site positioned at B: 8 to 10 mm (e.g., 9 mm), L: 12 to 14 mm (e.g., 13 mm), and D: 18 to 20 mm (e.g., 19 mm) from Bregma as a base point targets the putamen. Also, the administration site positioned at B: 8 to 10 mm (e.g., 9 mm), L: 7 to 9 mm (e.g., 8 mm), and D: 18 to 20 mm (e.g., 19 mm) from Bregma as a base point targets the putamen (particularly, a deep part of the putamen). Since these administration sites are adjacent to the internal capsule, the administration of endothelin to these administration sites can also bring about damage in the internal capsule.

The administration sites described above are particularly suitable for application to preferably a monkey belonging to the genus *Macaca,* for example, a cynomolgus macaque, but are not limited by the application to the organism species. The administration of endothelin to these administration sites may be simultaneously performed, may be sequentially performed, or may be performed in a plurality of portions.

In the animal model production method of the present invention, endothelin may be administered only to the left side of the brain in order to induce motor dysfunction on the right side of the body, or endothelin may be administered only to the right side of the brain in order to induce motor dysfunction on the left side of the body. The endothelin administration sites with Bregma as a base point mentioned above indicate an example of administering endothelin to the left side of the brain. The present invention also includes a method of administering endothelin to the corresponding bilaterally symmetric sites on the right side of the brain (i.e., administration sites having the same distance B along the anteroposterior axis and depth D, but having a R (right) coordinate determined instead of L (left) as the same distance along the lateral axis, with respect to the endothelin administration sites on the left side of the brain described above). The present invention may also include a method of administering endothelin to both the left side of the brain and the right side of the brain by appropriately adjusting the dose of endothelin, etc. in order to induce motor dysfunction on the left side of the body and the right side of the body.

In the animal model production method of the present invention, the dose (typically, the injection dose) of endothelin per administration site (typically, injection site) may be, for example, at least 0.1 µg, at least 1 µg, at least 4 µg, or at least 10 µg and is preferably at least 20 µg, at least 30 µg, at least 50 µg, at least 55 µg, or at least 60 µg and preferably 200 µg or less, 150 µg or less, 100 µg or less, 90 µg or less, or 70 µg or less. In one embodiment, the dose of endothelin per administration site (typically, injection site) may be 0.1 µg to 200 µg, 10 µg to 200 µg, 20 µg to 200 µg, 30 µg to 200 µg, 0.1 µg to 150 µg, 10 µg to 150 µg, 20 µg to 150 µg, 30 µg to 150 µg, 10 µg to 100 µg, 20 µg to 100 µg, 30 µg to 100 µg, 20 µg to 90 µg, 30 µg to 90 µg, 50 µg to 90 µg, 60 µg to 90 µg, 20 µg to 70 µg, 30 µg to 70 µg, 50 µg to 70 µg, 60 µg to 70 µg, 20 µg to 60 µg, 30 µg to 60 µg, or 50 µg to 60 µg and may be, for example, 60 µg. In one embodiment, the dose of endothelin per administration site (typically, injection site) is preferably 20 µg to 70 µg, 20 µg to 60 µg, or 30 µg to 60 µg, further preferably 50 µg to 60 µg.

These doses (typically, injection doses) of endothelin per administration site (typically, injection site) are particularly suitable for application to preferably a monkey belonging to the genus *Macaca,* for example, a cynomolgus macaque, but are not limited by the application to the organism species. The dose (typically, the injection dose) of endothelin to be administered or injected can be properly determined by those skilled in the art according to the organism species used (e.g., according to the body weight of the organism species).

In the animal model production method of the present invention, endothelin may be administered in the form of a solution (e.g., an aqueous solution) in an aqueous medium (water, saline, a buffer solution, a physiologically acceptable organic solvent, etc.). In one embodiment, endothelin may be administered as a solution of endothelin diluted with an aqueous acetic acid solution (e.g., 0.05 to 5% acetic acid, preferably 0.1 to 1% acetic acid). In the case of administering endothelin in a solution state, the administration can be performed by, but not limited to, injection. The injection can be performed using, but not limited to, an arbitrary injection unit into the brain, for example, an injection syringe such as a microsyringe, a cannula for administration, and/or a microinjection tube.

The amount (volume) of the endothelin solution to be administered or injected may be, but not limited to, 2 µL or more and is preferably 4 µL or more, more preferably 10 µL or more, 12 µL or more, 20 µL or more, or 30 µL or more, per administration site. The amount of the endothelin solution to be administered or injected may also be 100 µL or less, 50 µL or less, 40 µL or less, or 30 µL or less per administration site. In one embodiment, the amount of the endothelin solution to be administered or injected may be 2 µL to 100 µL, 4 µL to 50 µL, 10 µL to 100 µL, 12 µL to 40 µL, 12 µL to 30 µL, 20 µL to 40 µL, 20 µL to 50 µL, 25 µL to 50 µL, 30 µL to 50 µL, or 30 µL to 100 µL and may be, for example, 30 µL, per administration site. In one embodiment, in the case of administering at least 20 µg of endothelin per administration site (typically, injection site), the amount of the endothelin solution to be administered or injected may be 10 µL or more or 12 µL or more, for example, 10 µL to 50 µL, per administration site. In one embodiment, in the case of administering at least 50 µg of endothelin per administration site (typically, injection site), the amount of the endothelin solution to be administered or injected may be 20 µL or more or 30 µL or more, for example, 20 µL to 50 µL, per administration site.

The amount (volume) of the endothelin solution to be administered or injected described above is particularly suitable for application to preferably a monkey belonging to the genus *Macaca,* for example, a cynomolgus macaque, but are not limited by the application to the organism species. The amount (volume) of the endothelin solution to be administered or injected can be properly determined by those skilled in the art according to the organism species used.

The concentration of endothelin in the solution to be administered or injected may be, but not limited to, at least 0.1 µg/µL and is preferably at least 0.5 µg/µL or at least 1 µg/µL, and preferably 10 µg/µL or lower, 5 µg/µL or lower, or 3 µg/µL or lower. In one embodiment, the endothelin concentration may be, for example, 0.1 µg/µL to 10 µg/µL and is preferably 0.5 µg/µL to 5 µg/µL, more preferably 0.5 µg/µL to 3 µg/µL, further preferably 1 µg/µL to 3 µg/µL and may be, for example, 2 µg/µL.

In one embodiment, the animal model production method of the present invention may comprise administering 10 µL or more, preferably 10 µL to 50 µL per administration site of an endothelin solution having a concentration of 0.5 to 3 µg/µL, preferably a concentration of 1 to 3 µg/µL, for example, a concentration of 2 µg/µL, to administration sites (typically, injection sites). In one embodiment, the animal model production method of the present invention may comprise administering 12 µL or more, preferably 12 µL to 40 µL per administration site of an endothelin solution having a concentration of 0.5 to 3 µg/µL, preferably a concentration of 1 to 3 µg/µL, for example, a concentration of 2 µg/µL, to administration sites. In one embodiment, the animal model production method of the present invention may comprise administering 20 µL or more, preferably 20 µL to 50 µL per administration site of an endothelin solution having a concentration of 0.5 to 3 µg/µL, preferably a concentration of 1 to 3 µg/µL, for example, a concentration of 2 µg/µL, to administration sites. In one embodiment, the animal model production method of the present invention may comprise administering 30 µL or more, preferably 30 µL to 50 µL or 30 µL to 100 µL per administration site of an endothelin solution having a concentration of 0.5 to 3 µg/µL, preferably a concentration of 1 to 3 µg/µL, for example, a concentration of 2 µg/µL, to administration sites. In one embodiment, the animal model production method of the present invention may comprise administering 20 µL to 40 µL, preferably 25 µL to 35 µL, for example, 30 µL, per administration site of an endothelin solution having a concentration of 0.5 to 3 µg/µL, preferably a concentration of 1 to 3 µg/µL, for example, a concentration of 2 µg/µL, to administration sites. These approaches can be properly determined by those skilled in the art according to the organism species used.

The concentration and dose (typically, injection dose) of endothelin can be properly selected, thereby facilitating the spread of an effect ascribable to endothelin from the administration sites to their adjacent brain regions (e.g., the internal capsule and the subthalamic nucleus).

In the animal model production method of the present invention, basal ganglia damage, thalamus damage, and internal capsule damage can be induced by administering endothelin as described above to the basal ganglia and thalamic region of a nonhuman primate. In one embodiment, basal ganglia damage, thalamus damage, and internal capsule damage can be induced by administering endothelin as described above to the putamen, the caudate nucleus, the globus pallidus, the ventral lateral nucleus of the thalamus, and/or the ventral posterior nucleus of the thalamus.

In the present invention, the administration of endothelin to the basal ganglia and thalamic region induces cerebral ischemia so that necrosis occurs at or near the administration sites to induce damage.

The "basal ganglia damage" according to the present invention refers to damage developed in the basal ganglia. The damage developed in the basal ganglia makes not only intended activity but everyday natural body movement or action difficult, as seen in Parkinson's disease or the like.

The putamen which constitutes the basal ganglia forms the dorsal striatum, together with the caudate nucleus, and also forms the outermost part of the lenticular nucleus. The putamen is considered to be responsible for reinforcement learning. Two caudate nuclei which constitute the basal ganglia reside to flank the thalamus near the center of the brain. The caudate nucleus has been demonstrated to be strongly involved in learning and memory, particularly, a feedback process. The globus pallidus which constitutes the basal ganglia is divided to an external segment and an internal segment, both of which contain GABAergic large projection neurons. Damage of these nerve nuclei causes basal ganglia damage.

The "thalamus damage" according to the present invention refers to damage developed in the thalamus in the brain. When the administration of endothelin to the basal ganglia and thalamic region includes administration to the thalamus (e.g., the ventral lateral nucleus of the thalamus and/or the ventral posterior nucleus of the thalamus), this administration can induce thalamus damage. In one embodiment, the administration of endothelin to the basal ganglia and thalamic region, which is administration only to the basal ganglia, can also induce thalamus damage. Two (left and right) halves of the thalamus are situated near the center of cerebral hemisphere and above the midbrain and can each be divided to a lateral part and a medial part. The "ventral lateral nucleus of the thalamus" is a region of the lateral part of the thalamus, and this region receives GABA input from the globus pallidus and the substantia nigra and projects it mainly to the motor area of the cerebral cortex. The "ventral posterior nucleus of the thalamus" is a region of the medial part of the thalamus, and this region receives sensory information mainly from ascending sensory tracts and relays and projects it to the sensory area of the cerebral cortex.

In the animal model production method of the present invention, damage at the endothelin administration sites as well as internal capsule damage can be induced by administering endothelin to the basal ganglia and thalamic region of a nonhuman primate.

The "internal capsule damage" according to the present invention refers to damage developed in the internal capsule in the brain. The internal capsule is a cluster of nerve fibers, not the nucleus (neuronal body). The internal capsule is divided to an anterior limb and a posterior limb. Nerve fibers that connect the thalamus to the frontal lobe run in the anterior limb of the internal capsule, and nerve fibers that connect the temporal lobe, the parietal lobe, and the occipital lobe to the brain stem run in the posterior limb of the internal capsule. When internal capsule damage occurs, hemiplegia (partial paralysis) characteristically occurs on the side opposite to a damage site. At an acute stage of internal capsule damage, first, partial spasm of muscle occurs, and motor paralysis appears in the upper and lower extremities. In damage of the posterior limb of the internal capsule, the attenuation or disappearance of perception of the trunk, four extremities, or the head is added.

In one embodiment, in the animal model production method of the present invention, subthalamic nucleus damage can be further induced by administering endothelin to the basal ganglia and thalamic region of a nonhuman primate. The subthalamic nucleus is a nerve nucleus that constitutes the basal ganglia, and receives inhibitory output from the external segment of the globus pallidus, while performing excitatory input to the external segment of the globus pallidus and the internal segment of the globus pallidus/the reticular part of the substantia nigra.

In the animal model production method of the present invention, basal ganglia damage and thalamus damage as well as internal capsule damage is induced by administering endothelin to the basal ganglia and thalamic region of a nonhuman primate, because an effect of the endothelin administered to the basal ganglia and thalamic region extends over the internal capsule. When an effect of the endothelin extends over the internal capsule, for example, the endothelin administered targeting the basal ganglia and thalamic region may also be administered directly to the internal capsule, the endothelin administered to the basal ganglia and thalamic region may be diffused to the internal capsule, or necrosis resulting from the endothelin administered to the basal ganglia and thalamic region may spread to the internal capsule. In the animal model production method of the present invention, an effect of endothelin can extend over the internal capsule directly or indirectly, thereby inducing internal capsule damage. Likewise, the administration of endothelin to the basal ganglia and thalamic region also induces damage in a surrounding region other than the administration sites, such as the subthalamic nucleus, when an effect of the administered endothelin extends over the region.

Damage in the basal ganglia and each nerve nucleus constituting it, the thalamus, the internal capsule, and the like can be confirmed by 2,3,5-triphenyl tetrazolium chloride (TTC; CAS No: 298-96-4) staining. TTC is reduced by hydrogen generated in mitochondrion, thereby forming triphenyl formazan (TPF) which is insoluble in water and exhibits red color. Thus, a non-lesioned tissue is stained red, whereas a lesioned tissue is not stained red.

The present invention also provides a nonhuman primate animal model of cerebral infarction having basal ganglia damage, thalamus damage, and internal capsule damage, the nonhuman primate animal model having been produced by the animal model production method of the present invention described above (hereinafter, also referred to as the "nonhuman primate animal model of the present invention").

The nonhuman primate animal model of the present invention has basal ganglia damage, thalamus damage, and internal capsule damage induced (produced) by the animal model production method of the present invention and is useful as a model (animal model) of cerebral infarction. In one embodiment, the nonhuman primate animal model of the present invention can also be used as a model of cerebral infarction at an acute stage. In one embodiment, the nonhuman primate animal model of the present invention can maintain the damages over a relatively long period (e.g., 2 weeks to 3 months or longer) and can therefore be used as a model of cerebral infarction at a subacute to chronic stage. In one embodiment, the nonhuman primate animal model of the present invention is useful as a model of cerebral infarction at a subacute to chronic stage. In one embodiment, the nonhuman primate animal model of the present invention is particularly useful as a model of cerebral infarction at a chronic stage. In one embodiment, in the animal model production method of the present invention, cerebral infarction at an acute to chronic stage can be induced by administering endothelin to the basal ganglia and thalamic region of a nonhuman primate. In one embodiment, in the animal model production method of the present invention, cerebral infarction at a subacute to chronic stage can be induced by administering endothelin to the basal ganglia and thalamic region of a nonhuman primate. In one embodiment, in the animal model production method of the present invention, cerebral infarction at a chronic stage can be induced by administering endothelin to the basal ganglia and thalamic region of a nonhuman primate.

In the nonhuman primate including a cynomolgus macaque, the acute stage refers to a period having the possibility of worsening symptoms from immediately after the onset of cerebral infarction to less than 72 hours after the onset. The subacute stage refers to a period from 72 hours after the onset of cerebral infarction to less than 2 weeks after the onset, and the chronic stage refers to a period of 2 weeks or later after the onset of cerebral infarction. In the present invention, the subacute to chronic stage at which the evaluation of cerebral infarction in the nonhuman primate animal model or the treatment of the nonhuman primate, etc. is performed may be a period of 72 hours or later after the onset of cerebral infarction, for example, a period of 2 weeks or later after the onset of cerebral infarction, or may be a period of 3 weeks or later or 1 month or later after the onset of cerebral infarction, for example, 3 weeks to 6 months or 1 month to 6 months after the onset of cerebral infarction, or 3 weeks to 3 months or 1 month to 3 months after the onset of cerebral infarction. In the present specification, the "subacute to chronic stage" of cerebral infarction in the nonhuman primate including a cynomolgus macaque refers to a period of 72 hours or later after the onset of cerebral infarction, and the "chronic stage" refers to a period of 2 weeks or later after the onset of cerebral infarction. In the present specification, the term "1 week after" means 7 days later, the term "2 weeks after" means 14 days later, and the term "3 weeks after" means 21 days later.

The nonhuman primate animal model of the present invention is also useful as an animal model of penetrating branch infarction. In one embodiment, in the animal model production method of the present invention, penetrating branch infarction can be induced by administering endothelin to the basal ganglia and thalamic region of a nonhuman primate. The penetrating branch infarction may be penetrating branch infarction having brain damage in a particular region. Examples thereof include i) penetrating branch infarction having brain damage in the basal ganglia and thalamic region, ii) penetrating branch infarction having brain damage in the basal ganglia and/or the thalamus, iii) penetrating branch infarction having brain damage in the basal ganglia and thalamic region and the internal capsule, and iv) penetrating branch infarction having brain damage in the basal ganglia, the thalamus, and the internal capsule. In one embodiment, the nonhuman primate animal model of the present invention can also be used as an animal model of i) penetrating branch infarction having brain damage in the basal ganglia and thalamic region, ii) penetrating branch infarction having brain damage in the basal ganglia and/or the thalamus, iii) penetrating branch infarction having brain damage in the basal ganglia and thalamic region and the internal capsule, or iv) penetrating branch infarction having brain damage in the basal ganglia, the thalamus, and the internal capsule. The penetrating branch infarction having brain damage in a particular region includes not only penetrating branch infarction having brain damage only in the particular region but penetrating branch infarction having brain damage in a region other than the particular region.

In one embodiment, the nonhuman primate animal model of the present invention can be used as an animal model of penetrating branch infarction at a subacute to chronic stage. In one embodiment, the nonhuman primate animal model of the present invention can also be used as an animal model of penetrating branch infarction at a subacute to chronic stage, the penetrating branch infarction i) having brain damage in the basal ganglia and thalamic region, ii) having brain damage in the basal ganglia and/or the thalamus, iii) having brain damage in the basal ganglia and thalamic region and the internal capsule, or iv) having brain damage in the basal ganglia, the thalamus, and the internal capsule. In one embodiment, in the animal model production method of the present invention, penetrating branch infarction at a subacute to chronic stage (e.g., penetrating branch infarction i) having brain damage in the basal ganglia and thalamic region, ii) having brain damage in the basal ganglia and/or the thalamus, iii) having brain damage in the basal ganglia and thalamic region and the internal capsule, or iv) having brain damage in the basal ganglia, the thalamus, and the internal capsule) can be induced by administering endothelin to the basal ganglia and thalamic region of a nonhuman primate.

In one embodiment, the nonhuman primate animal model of the present invention is particularly useful as an animal model of penetrating branch infarction at a chronic stage. In one embodiment, the nonhuman primate animal model of the present invention can also be used as an animal model of penetrating branch infarction at a chronic stage, the penetrating branch infarction i) having brain damage in the basal ganglia and thalamic region, ii) having brain damage in the basal ganglia and/or the thalamus, iii) having brain damage in the basal ganglia and thalamic region and the internal capsule, or iv) having brain damage in the basal ganglia, the thalamus, and the internal capsule. In one embodiment, in the animal model production method of the present invention, penetrating branch infarction at a chronic stage (e.g., penetrating branch infarction i) having brain damage in the basal ganglia and thalamic region, ii) having brain damage in the basal ganglia and/or the thalamus, iii) having brain damage in the basal ganglia and thalamic region and the internal capsule, or iv) having brain damage in the basal ganglia, the thalamus, and the internal capsule) can be induced by administering endothelin to the basal ganglia and thalamic region of a nonhuman primate.

A disease in which the brain is damaged due to the blockage or break of a vascular vessel in the brain is called stroke. Among others, a disease that is caused by the blockage of a vascular vessel that nourishes the brain is cerebral infarction. The cerebral infarction is also referred to as ischemic stroke. The cerebral infarction is broadly classified into four types, cardiogenic brain embolism, atherothrombotic cerebral infarction, lacunar infarction, and infraction that is not classified thereinto (unclassified). The "penetrating branch" is a thin artery given off from the middle cerebral artery which constitutes the major cerebral arteries, and supplies the basal ganglia and thalamic region and the internal capsule as a territory. If a region in the brain is a "territory supplied" by a vascular vessel, this means that blood is supplied to the region in the brain through the vascular vessel. In that case, the region in the brain is within a vascular territory of the vascular vessel. The "penetrating branch infarction" is a disease in which infarction occurs in the thalamus, the caudate nucleus, the putamen, the globus pallidus, and/or the internal capsule as a territory supplied by the penetrating branch so that a function of the site with the infarction is impaired. The putamen and the caudate nucleus are known as nerve nuclei susceptible to infarction due to penetrating branch infarction. Lacunar infarction which is cerebral infarction caused by the blockage of a single deep penetrating branch has been widely known as penetrating branch infarction. Meanwhile, infarction that extends over the whole area of the penetrating branch due to thrombus based on an atheromatous plaque near bifurcation from the parent artery of a large-diameter penetrating branch has been raised in recent years as a type of penetrating branch infarction called branch atheromatous disease (BAD). In addition, striatocapsular infarction, internal capsule infarction, anterior choroidal artery infarction, thalamus infarction, and the like are known as penetrating branch infarction. The penetrating branch infarction is also known as cerebral infarction that occurs in a region as a territory supplied by the penetrating branch (penetrating branch territory). The "penetrating branch territory" is a region being as a territory supplied by the penetrating branch and refers to the basal ganglia and thalamic region and the internal capsule.

The lacunar infarction, as in general cerebral infarction, has weakness on one side of the body (motor paralysis), numbness on one side of the body (sensation disturbance), and difficulty in speaking (dysarthria) as main symptoms, and is generally less than 15 mm in size resulting in necrosis when the penetrating branch is blocked.

The BAD is an infarction of the unclassified type described above. The atherothrombotic cerebral infarction is cerebral infarction that is caused by the blockage of a thick artery due to arteriosclerosis. The BAD is of intermediate type between lacunar infarction and atherothrombotic cerebral infarction and does not narrow a thick vascular vessel, but causes cerebral infarction in a wider region compared with lacunar infarction due to the blockage at the base of the penetrating branch given off therefrom. The BAD varies in symptom depending on a location with cerebral infarction, and has motor paralysis, sensation disturbance, and dysarthria as main symptoms, as in lacunar infarction. The course of symptoms is similar to that of atherothrombotic cerebral infarction, and the region of cerebral infarction gradually spreads over several days after the onset and often progresses.

In one embodiment, the nonhuman primate animal model of the present invention can be used as an animal model of lacunar infarction and/or BAD. In one embodiment, in the animal model production method of the present invention, lacunar infarction and/or BAD can be induced by administering endothelin to the basal ganglia and thalamic region of a nonhuman primate. In one embodiment, the nonhuman primate animal model of the present invention can be used as an animal model of lacunar infarction and/or BAD at a subacute to chronic stage. In one embodiment, in the animal model production method of the present invention, lacunar infarction and/or BAD at a subacute to chronic stage can be induced by administering endothelin to the basal ganglia and thalamic region of a nonhuman primate. In one embodiment, the nonhuman primate animal model of the present invention is particularly useful as an animal model of lacunar infarction and/or BAD at a chronic stage. In one embodiment, in the animal model production method of the present invention, lacunar infarction and/or BAD at a chronic stage can be induced by administering endothelin to the basal ganglia and thalamic region of a nonhuman primate.

The lacunar infarction or BAD may be lacunar infarction or BAD having brain damage in a particular region. For example, the lacunar infarction or BAD may be i) lacunar infarction or BAD having brain damage in the basal ganglia and thalamic region, ii) lacunar infarction or BAD having brain damage in the basal ganglia and/or the thalamus, iii) lacunar infarction or BAD having brain damage in the basal ganglia and thalamic region and the internal capsule, or iv) lacunar infarction or BAD having brain damage in the basal ganglia, the thalamus, and the internal capsule. The penetrating branch infarction having brain damage in a particular region may be lacunar infarction or BAD having brain damage in a particular region.

The nonhuman primate animal model of the present invention can also be used as an animal model of cerebral infarction having brain damage in a penetrating branch territory. In the present specification, the "cerebral infarction having brain damage in a penetrating branch territory" refers to cerebral infarction in which brain damage resulting from infarction exists mainly in a penetrating branch territory. The cerebral infarction having brain damage in a penetrating branch territory includes not only cerebral infarction in which brain damage exists in a penetrating branch territory due to infarction developed in the penetrating branch territory, but cerebral infarction in which brain damage secondarily exists in a penetrating branch territory, for example, as in the case where cell necrosis caused by infarction developed near the penetrating branch territory spreads to the penetrating branch territory so that a function at the site is impaired. In the present specification, the brain damage means functional damage of the brain caused by cell necrosis resulting from infarction. The brain damage usually has cell necrosis in a region having the brain damage. The "cerebral infarction having brain damage in a penetrating branch territory" may be cerebral infarction having cell necrosis in the penetrating branch territory.

The cerebral infarction having brain damage in a penetrating branch territory includes, for example, penetrating branch infarction (such as lacunar infarction, BAD, or penetrating branch infarction having brain damage in a particular region).

The cerebral infarction having brain damage in a penetrating branch territory includes cerebral infarction having a partially or fully damaged penetrating branch territory. Examples thereof include cerebral infarction having brain damage in a penetrating branch territory, wherein the brain damage is brain damage including i) brain damage of the basal ganglia and thalamic region, ii) brain damage of the basal ganglia and/or the thalamus, iii) brain damage of the basal ganglia and thalamic region and the internal capsule, or iv) brain damage of the basal ganglia, the thalamus, and the internal capsule. In one embodiment, the nonhuman primate animal model of the present invention can also be used as an animal model of cerebral infarction having brain damage in a penetrating branch territory, wherein the brain damage is brain damage including i) brain damage of the basal ganglia and thalamic region, ii) brain damage of the basal ganglia and/or the thalamus, iii) brain damage of the basal ganglia and thalamic region and the internal capsule, or iv) brain damage of the basal ganglia, the thalamus, and the internal capsule. In one embodiment, in the animal model production method of the present invention, cerebral infarction having brain damage in a penetrating branch territory (e.g., cerebral infarction having brain damage in a penetrating branch territory, wherein the brain damage is i) brain damage including brain damage of the basal ganglia and thalamic region, ii) brain damage including brain damage of the basal ganglia and/or the thalamus, iii) brain damage including brain damage of the basal ganglia and thalamic region and the internal capsule, or iv) brain damage including brain damage of the basal ganglia, the thalamus, and the internal capsule) can be induced by administering endothelin to the basal ganglia and thalamic region of a nonhuman primate.

In one embodiment, the nonhuman primate animal model of the present invention can be used as an animal model of cerebral infarction at a subacute to chronic stage having brain damage in a penetrating branch territory. In one embodiment, the nonhuman primate animal model of the present invention can also be used as an animal model of cerebral infarction at a subacute to chronic stage having brain damage in a penetrating branch territory, wherein the brain damage is brain damage including i) brain damage of the basal ganglia and thalamic region, ii) brain damage of the basal ganglia and/or the thalamus, iii) brain damage of the basal ganglia and thalamic region and the internal capsule, or iv) brain damage of the basal ganglia, the thalamus, and the internal capsule. In one embodiment, in the animal model production method of the present invention, cerebral infarction at a subacute to chronic stage having brain damage in a penetrating branch territory (e.g., cerebral infarction at a subacute to chronic stage having brain damage in a penetrating branch territory, wherein the brain damage is brain damage including i) brain damage of the basal ganglia and thalamic region, ii) brain damage of the basal ganglia and/or the thalamus, iii) brain damage of the basal ganglia and thalamic region and the internal capsule, or iv) brain damage of the basal ganglia, the thalamus, and the internal capsule) can be induced by administering endothelin to the basal ganglia and thalamic region of a nonhuman primate.

In one embodiment, the nonhuman primate animal model of the present invention can be used as an animal model of cerebral infarction at a chronic stage having brain damage in a penetrating branch territory. In one embodiment, the nonhuman primate animal model of the present invention can also be used as an animal model of cerebral infarction at a chronic stage having brain damage in a penetrating branch territory, wherein the brain damage is brain damage including i) brain damage of the basal ganglia and thalamic region, ii) brain damage of the basal ganglia and/or the thalamus, iii) brain damage of the basal ganglia and thalamic region and the internal capsule, or iv) brain damage of the basal ganglia, the thalamus, and the internal capsule. In one embodiment, in the animal model production method of the present invention, cerebral infarction at a chronic stage having brain damage in a penetrating branch territory (e.g., cerebral infarction at a chronic stage having brain damage in a penetrating branch territory, wherein the brain damage is brain damage including i) brain damage of the basal ganglia and thalamic region, ii) brain damage of the basal ganglia and/or the thalamus, iii) brain damage of the basal ganglia and thalamic region and the internal capsule, or iv) brain damage of the basal ganglia, the thalamus, and the internal capsule) can be induced by administering endothelin to the basal ganglia and thalamic region of a nonhuman primate.

In penetrating branch infarction, or cerebral infarction having brain damage in a penetrating branch territory, damage is often found in the basal ganglia as well as the internal capsule. The basal ganglia and the thalamus are gray matter where the cell bodies of neurons gather, whereas the internal capsule is white matter where nerve fibers mainly accumulate and run, being poor in the cell bodies of neurons. One example of a bundle of nerve fibers that form the internal capsule is a corticospinal tract. The corticospinal tract is a bundle of axons (nerve fibers) from the motor area of the cerebral cortex through the spinal cord to lower motor neurons. The corticospinal tract is mostly constituted by axons of upper motor neurons.

When internal capsule in a penetrating branch territory is damaged, the corticospinal tract is injured. The injured corticospinal tract can be evaluated by, for example, diffusion tensor image (DTI) analysis using magnetic resonance imaging (MRI). In one embodiment, the nonhuman primate animal model of the present invention has corticospinal tract injury.

When the basal ganglia in a penetrating branch territory is damaged, the decreased expression of dopamine transporter is shown mainly in the corpus striatum. The dopamine transporter is present at the terminal of the corpus striatum of nerve (dopamine nerve) that runs from the substantia nigra to the corpus striatum in the brain, and is responsible for the reuptake of dopamine released into the corpus striatum. Abnormalities in the basal ganglia and the dopaminergic system cause motor dysfunction specific to basal ganglia damage, such as abnormalities in voluntary movement, postural muscle tone, and gait. Decreased expression of dopamine transporter can be evaluated, for example, by immunostaining using an anti-dopamine transporter antibody. In one embodiment, the nonhuman primate animal model of the present invention has the decreased expression of dopamine transporter.

The nonhuman primate animal model of the present invention can also be used as an animal model of cerebral infarction having local brain damage. The cerebral infarction having local brain damage does not include cerebral infarction in which brain damage exists over a wide region of the brain, for example, cerebral infarction in which the cerebral cortex, the internal capsule, the basal ganglia, and the like are damaged due to infarction developed in the middle cerebral artery. In one embodiment, the nonhuman primate animal model of the present invention can be used as an animal model of cerebral infarction at a subacute to chronic stage having local brain damage. In one embodiment, the nonhuman primate animal model of the present invention can be used as an animal model of cerebral infarction at a chronic stage having local brain damage.

Cerebral infarction leaves serious aftereffects at a chronic stage because astrocytes react near a lesion in mammalian central nerve to form a scab-like tissue called glial scar which in turn hinders nerve regeneration. A model of cerebral infarction at a chronic stage where glial scar is formed is very useful for the development of a therapeutic agent for cerebral infarction at a chronic stage. In one embodiment, the nonhuman primate animal model of the present invention manifests neuronal loss and astrocyte accumulation at a chronic stage of cerebral infarction.

### d) Methods for confirming and evaluating cerebral infarction

The nonhuman primate animal model of the present invention can be determined as a nonhuman primate animal model of cerebral infarction and/or as a nonhuman primate animal model of penetrating branch infarction by a histological approach, for example, TTC staining or hematoxylin-eosin (HE) staining, or from an infarct area (damaged area) in the nonhuman primate animal model by use of computed tomography (CT) or MRI, as in a method for diagnosing human cerebral infarction, for example, penetrating branch infarction. The nonhuman primate animal model of the present invention can also be determined as an animal model of cerebral infarction having brain damage in a penetrating branch territory by a similar approach. The nonhuman primate animal model of the present invention can be confirmed as a nonhuman primate animal model of cerebral infarction at a subacute to chronic stage on the basis of persistence of damage, in addition to the methods described above. For example, when motor dysfunction persists over at least 4 weeks from the onset of cerebral infarction, the nonhuman primate animal model of cerebral infarction may be determined to be useful as a nonhuman primate animal model of cerebral infarction at a subacute to chronic stage, though the method for confirming the persistence of damage is not limited thereto and can be changed according to an evaluation method.

An index for use in evaluating the condition (symptoms, severity, persistence of damage, etc.) of motor dysfunction in the nonhuman primate animal model of the present invention is not particularly limited as long as the condition of motor dysfunction in the animal can be evaluated. The condition can be evaluated using, for example, NHPSS and/or mRS (for monkeys) described in the present specification. The motor dysfunction induced in the nonhuman primate animal model of the present invention may be impairment of gross motor skills.

The evaluation using mRS can be carried out by scoring based on Table 1 mentioned later. mRS (for human clinical practice) described in Table 1 is an index that is widely used as an index for physical disabilities in the medical examination of stroke. mRS (for monkeys) described in Table 1 which is a modified version of the index for monkeys can be used in the evaluation of the condition (symptoms, severity, persistence of damage, etc.) of motor dysfunction in the nonhuman primate animal model of the present invention. The evaluation using NHPSS can be carried out by scoring in accordance with criteria of Table 2 mentioned later. The evaluation using NHPSS can be conducted on the basis of the scoring of one to four items of motor system, consciousness, skeletal muscle coordination, and sensory system. Preferably, the items include at least the motor system. The sensory system may not be scored because responsiveness easily varies among individuals. Since motor dysfunction is mainly observed at a subacute stage or at a chronic stage, the evaluation using NHPSS in this period may be performed on the basis of only motor system scoring.

In the case of determining the severity of motor dysfunction using mRS, a subject having, for example, mRS of 3 or more can be determined as having moderate to severe motor dysfunction. NHPSS may be used in the determination of severity. A subject having, for example, in an NHPSS motor system score (the total score of item A in Table 2 below; the same holds true for the description below), a score of the impaired side of 12 or more can be determined as having moderate to severe motor dysfunction. In the present specification, the motor dysfunction with high severity means moderate to severe motor dysfunction.

The severity of motor dysfunction in the nonhuman primate animal model of the present invention is, for example, mRS of 2 or more, preferably mRS of 3 to 5.

The severity of motor dysfunction in the nonhuman primate animal model of the present invention is, for example, in an NHPSS motor system score, a score of the impaired side of 4 or more, preferably a score of the damaged side of 12 or more, more preferably 14 or more, particularly preferably 16.

In one embodiment, in the animal model production method of the present invention, the severity of motor dysfunction induced by the administration of endothelin is mRS of 2 or more. In one embodiment, in the animal model production method of the present invention, the severity of motor dysfunction induced by the administration of endothelin is mRS of 3 to 5. In one embodiment, in the animal model production method of the present invention, the severity of motor dysfunction induced by the administration of endothelin is a score of the impaired side of 4 or more in an NHPSS motor system score. In one embodiment, in the animal model production method of the present invention, the severity of motor dysfunction induced by the administration of endothelin is a score of the impaired side of 12 or more in an NHPSS motor system score. In one embodiment, in the animal model production method of the present invention, the severity of motor dysfunction induced by the administration of endothelin is mRS of 3 to 5 and a score of the impairedside of 12 or more in an NHPSS motor system score.

In one embodiment, the severity of motor dysfunction in the nonhuman primate animal model of the present invention is mRS of 2 or more. In one embodiment, the severity of motor dysfunction in the animal model obtained by the production method of the present invention is mRS of 3 to 5. In one embodiment, the severity of motor dysfunction in the nonhuman primate animal model of the present invention is a score of the impaired side of 4 or more in an NHPSS motor system score. In one embodiment, the severity of motor dysfunction in the nonhuman primate animal model of the present invention is a score of the impaired side of 12 or more in an NHPSS motor system score. In one embodiment, the severity of motor dysfunction in the nonhuman primate animal model of the present invention is mRS of 3 to 5 and a score of the impaired side of 12 or more in an NHPSS motor system score.

In the nonhuman primate animal model of the present invention, the score of mRS is relatively stable 2 weeks or later after the onset of cerebral infarction. Accordingly, in the case of determining persistence of motor dysfunction using mRS, the persistence of motor dysfunction may be determined on the basis of the score of mRS, for example, over an arbitrarily set period from 2 weeks or later after the onset of cerebral infarction. Likewise, in the case of determining persistence of motor dysfunction using an NHPSS motor system score, the persistence of motor dysfunction may be determined on the basis of the motor system score of NHPSS, for example, over an arbitrarily set period from 2 weeks or later after the onset of cerebral infarction.

**Table 1**

| Score | mRS (for human clinical practice) | mRS (for monkeys) |
|---|---|---|
| 0 | No symptoms at all | No symptoms at all |
| 1 | No significant disability: | Mild paralysis¹ of a forelimb or hindlimb on impaired side |
| | despite symptoms, able to carry out all usual duties and activities | |
| 2 | Slight disability: | Slight decline in locomotor activity³, or mild paralysis of a forelimb and hindlimb on impaired side |
| | unable to perform all previous activities but able to look after own affairs without assistance | |
| 3 | Moderate disability: | (i) Decline in locomotor activity⁴, or (ii) seated posture (including incomplete standing-up motion), or (iii) paralysis² of a forelimb and hindlimb on impaired side. No decreased level of consciousness⁵ |
| | requiring some help but able to walk without assistance | |
| 4 | Moderately severe disability: | (i) Decline in locomotor activity and (ii) seated posture/recumbent posture⁶ (including incomplete standing-up motion), and (iii) paralysis of a forelimb and hindlimb on impaired side or a slightly decreased level of consciousness⁷ |
| | unable to walk without assistance and unable to attend to own bodily needs without assistance | |
| 5 | Severe disability: | Recumbent posture (including incomplete standing-up motion), and paralysis of a forelimb and hindlimb on impaired side, and a decreased level of consciousness⁸ |
| | bedridden, incontinent and requiring constant nursing care and attention | |
| 6 | Death | Death |

| | | |
|---|---|---|
| ¹Mild paralysis: no significant paralysis is found, but a slight sign of paralysis is found. ²Paralysis: significant paralysis is found. ³Slight decline in locomotor activity: no significant decline in locomotor activity is found, but a slight sign of decline in locomotor activity is found. ⁴Decline in locomotor activity: significant decline in locomotor activity is found. ⁵Level of consciousness (monkeys): determined on the level of response to human. ⁶Seated posture/recumbent posture: at least one of seated posture and recumbent posture. ⁷Slightly decreased level of consciousness: no significantly decreased level of consciousness is found, but a slight sign of a decreased level of consciousness is found. ⁸Decreased level of consciousness: a significantly decreased level of consciousness is found. | | |

**Table 2**

| <NHPSS> | |
|---|---|
| A. Motor system | |
| Select an appropriate score (within parentheses) as to each of limbs on impaired and unimpaired sides, and calculate a total score of 1 to 4. | |
| 1. Upper limb (grip and movement, impaired/unimpaired side) | |
| - Normal (0/0) | |
| - Reduced grip strength and skilled movement (2/2) | |
| - Paralysed and useless (4/4) | |
| 2. Lower limb (grip and movement, impaired/unimpaired side) | |
| - Normal (0/0) | |
| - Raise with flexion of knee against gravity (2/2) | |
| - Movement possible, but not against gravity (4/4) | |
| - Paralysed and useless (6/6) | |
| 3. Upper limb tone (impaired/unimpaired side) | |
| - Normal (0/0) | |
| - Overtly spastic or flaccid (3/3) | |
| 4. Lower limb tone (impaired/unimpaired side) | |
| - Normal (0/0) | |
| - Overtly spastic or flaccid (3/3) | |
| | Total score (A) |

| B. Consciousness | |
|---|---|
| Select one appropriate score (within parentheses). | |
| - Normal, consistently alert (0) | |
| - Conscious and aggressive (4) | |
| - Conscious and evasive(6) | |
| - Conscious but clouded and tolerant (8) | |
| - Drowsiness, arounsed with stimulation (10) | |
| - Lethargia, eyes opened by intense stimulation (16) | |
| - Stupor, aroused with persistent stimulation (20) | |
| - Light coma, reflex movement only (24) | |
| - Deep coma, no movement (28) | |
| | Total score (B) |

| C. Musculoskeletal coordination | |
|---|---|
| Select one appropriate score (within parentheses). | |
| - Normal, walks normally (0) | |
| - Minimal incoordination, walks with some gait impairment (4) | |
| - Incoordinated, unable to climb the perch (6) | |
| - Stands independently, difficulty in walking (10) | |
| - Sit on floor, able to circle with stimulation (12) | |
| - Pose with recumbency (16) | |
| - No movement (18) | |
| | Total score (C) |

| D. Sensory system | |
|---|---|
| Select an appropriate score (within parentheses) as to each of impaired and unimpaired sides, and calculate a total score of 1 to 3. | |
| 1. Facial sensation (impaired/unimpaired side) | |
| - React consistently to stimulation of face (0/0) | |
| - Absent, does not react to stimulation of face (3/3) | |
| 2. Pinna reflex (impaired/unimpaired side) | |
| - Reacts in response to pull of ear (0/0) | |
| - Absent, does not react in response to pull of ear (3/3) | |
| 3. Pain reflex (lower limb, impaired/unimpaired side) | |
| - Quick withdrawal from toe pinch (0/0) | |
| - Slow withdrawal from toe pinch (3/3) | |
| - Absent, no withdrawal from toe pinch (5/5) | |
| | Total score (D) |
| Total score (total sum of the scores of the evaluated items A to D) | |

### 2. Method for screening for therapeutic agent for cerebral infarction

The present invention also provides a method for screening for a therapeutic agent for cerebral infarction using the nonhuman primate animal model of the present invention (hereinafter, also referred to as the "screening method for a therapeutic agent for cerebral infarction of the present invention"). More specifically, the screening method for a therapeutic agent for cerebral infarction of the present invention comprises the steps of: administering a test substance to the nonhuman primate animal model of the present invention; and determining an effect of the test substance on cerebral infarction in the nonhuman primate animal model.

In one embodiment, the screening method for a therapeutic agent for cerebral infarction of the present invention is a method for screening for a therapeutic agent for cerebral infarction, preferably cerebral infarction at a subacute to chronic stage. In one embodiment, the screening method for a therapeutic agent for cerebral infarction of the present invention is a method for screening for a therapeutic agent for cerebral infarction which is penetrating branch infarction (e.g., penetrating branch infarction having brain damage in the basal ganglia and thalamic region, penetrating branch infarction having brain damage in the basal ganglia and/or the thalamus, penetrating branch infarction having brain damage in the basal ganglia and thalamic region and the internal capsule, or penetrating branch infarction having brain damage in the basal ganglia, the thalamus, and the internal capsule, and/or lacunar infarction and/or BAD) or cerebral infarction having brain damage in a penetrating branch territory (e.g., cerebral infarction having brain damage in a penetrating branch territory, wherein the brain damage is brain damage including i) brain damage of the basal ganglia and thalamic region, ii) brain damage of the basal ganglia and/or the thalamus, iii) brain damage of the basal ganglia and thalamic region and the internal capsule, or iv) brain damage of the basal ganglia, the thalamus, and the internal capsule). In one embodiment, the screening method for a therapeutic agent for cerebral infarction of the present invention is a method for screening for a therapeutic agent for cerebral infarction which is penetrating branch infarction at a subacute to chronic stage (particularly, at a chronic stage) (e.g., penetrating branch infarction i) having brain damage in the basal ganglia and thalamic region, ii) having brain damage in the basal ganglia and/or the thalamus, iii) having brain damage in the basal ganglia and thalamic region and the internal capsule, or iv) having brain damage in the basal ganglia, the thalamus, and the internal capsule, and/or lacunar infarction and/or BAD) or cerebral infarction at a subacute to chronic stage (particularly, at a chronic stage) having brain damage in a penetrating branch territory (e.g., cerebral infarction having brain damage in a penetrating branch territory, wherein the brain damage is brain damage including: i) brain damage of the basal ganglia and thalamic region, ii) brain damage of the basal ganglia and/or the thalamus, iii) brain damage of the basal ganglia and thalamic region and the internal capsule, or iv) brain damage of the basal ganglia, the thalamus, and the internal capsule). In one embodiment, the screening method for a therapeutic agent for cerebral infarction of the present invention is a method for screening for a therapeutic agent for cerebral infarction which is cerebral infarction at a subacute to chronic stage (particularly, at a chronic stage) other than penetrating branch infarction and cerebral infarction having brain damage in a penetrating branch territory. The screening method of the present invention may be a method for screening for a therapeutic agent for cerebral infarction which is cerebral infarction in the same subject as that to which the treatment method of the present invention, etc. mentioned later is applied.

The screening method for a therapeutic agent for cerebral infarction of the present invention is particularly useful in the evaluation or screening of a therapeutic agent for cerebral infarction which is penetrating branch infarction at a subacute to chronic stage (particularly, at a chronic stage) in a human (e.g., penetrating branch infarction i) having brain damage in the basal ganglia and thalamic region, ii) having brain damage in the basal ganglia and/or the thalamus, iii) having brain damage in the basal ganglia and thalamic region and the internal capsule, or iv) having brain damage in the basal ganglia, the thalamus, and the internal capsule, and/or lacunar infarction and/or BAD) or cerebral infarction at a subacute to chronic stage (particularly, at a chronic stage) having brain damage in a penetrating branch territory in a human (e.g., cerebral infarction having brain damage in a penetrating branch territory, wherein the brain damage is brain damage including i) brain damage of the basal ganglia and thalamic region, ii) brain damage of the basal ganglia and/or the thalamus, iii) brain damage of the basal ganglia and thalamic region and the internal capsule, or iv) brain damage of the basal ganglia, the thalamus, and the internal capsule). Likewise, the screening method for a therapeutic agent for cerebral infarction of the present invention is also useful in the evaluation or screening of a therapeutic agent for cerebral infarction which is cerebral infarction at a subacute to chronic stage (particularly, at a chronic stage) other than penetrating branch infarction in a human (i.e., cardiogenic brain embolism and atherothrombotic cerebral infarction).

Cerebral infarction can be classified depending on its site of onset into cerebral cortex infarction, brain stem infarction, subcortical infarction (e.g., penetrating branch infarction), and cerebellar infarction. In the present specification, the cerebral infarction may be cerebral cortex infarction, brain stem infarction, subcortical infarction (e.g., penetrating branch infarction), and/or cerebellar infarction.

The screening method for a therapeutic agent for cerebral infarction of the present invention can also be used in the screening of a therapeutic agent for cerebral infarction which is cerebral infarction having a high severity. In one embodiment, the screening method for a therapeutic agent for cerebral infarction of the present invention is a method for screening for a therapeutic agent for cerebral infarction which is cerebral infarction with mRS of 2 or more. In one embodiment, the screening method for a therapeutic agent for cerebral infarction of the present invention is a method for screening for a therapeutic agent for cerebral infarction which is cerebral infarction with mRS of 3 to 5. In one embodiment, the screening method for a therapeutic agent for cerebral infarction of the present invention is a method for screening for a therapeutic agent for cerebral infarction which is cerebral infarction with a score of the impaired side of 4 or more in an NHPSS motor system score. In one embodiment, the screening method for a therapeutic agent for cerebral infarction of the present invention is a method for screening for a therapeutic agent for cerebral infarction which is cerebral infarction with a score of the impaired side of 12 or more in an NHPSS motor system score. In one embodiment, the screening method for a therapeutic agent for cerebral infarction of the present invention is a method for screening for a therapeutic agent for cerebral infarction which is cerebral infarction with mRS of 3 to 5 and a score of the impaired side of 12 or more in an NHPSS motor system score.

The screening method for a therapeutic agent for cerebral infarction of the present invention can also be used in the screening of a therapeutic agent for cerebral infarction which is cerebral infarction having local brain damage. In one embodiment, the screening method for a therapeutic agent for cerebral infarction of the present invention is a method for screening for a therapeutic agent for cerebral infarction which is cerebral infarction having local brain damage. In one embodiment, the screening method for a therapeutic agent for cerebral infarction of the present invention is a method for screening for a therapeutic agent for cerebral infarction which is cerebral infarction at a subacute to chronic stage having local brain damage. In one embodiment, the screening method for a therapeutic agent for cerebral infarction of the present invention is a method for screening for a therapeutic agent for cerebral infarction which is cerebral infarction at a chronic stage having local brain damage.

In the screening method for a therapeutic agent for cerebral infarction of the present invention, the test substance to be administered to the nonhuman primate animal model of the present invention may be a nucleic acid (DNA or RNA (e.g., messenger RNA (mRNA), small interfering RNA (siRNA), short hairpin RNA (shRNA), microRNA (miRNA), and an antisense oligonucleotide). The nucleic acid may be a natural nucleic acid or an artificial nucleic acid (e.g., a nucleic acid containing a natural nucleobase and/or an artificial nucleobase). Further, the test substance may be any substance such as a peptide, a protein, a cell, a low-molecular compound, and a middle-molecular compound. Examples thereof include substances having nerve regeneration activity, various transcriptional factors, various neurotrophic factors, neuronal growth factors, neuronal growth promoters, direct reprograming factors, neural stem cells, neurons, and mesenchymal stem cells. In the screening method for a therapeutic agent for cerebral infarction of the present invention, examples of the approach of administering the test substance include, but are not limited to, oral administration, injection (intravenous injection, subcutaneous injection, intramuscular injection, etc.), direct administration to a brain damage site (e.g., a damaged area), and transplantation of the test substance. The test substance can be administered to the nonhuman primate animal model of the present invention at an arbitrary timing. For the purpose of screening for a therapeutic agent for cerebral infarction which is cerebral infarction at a subacute to chronic stage, it is preferred to administer the test substance to the nonhuman primate animal model of the present invention at a subacute to chronic stage (e.g., 3 weeks after the onset of cerebral infarction). Likewise, for the purpose of screening for a therapeutic agent for cerebral infarction which is cerebral infarction at a chronic stage, it is preferred to administer the test substance to the nonhuman primate animal model of the present invention at a chronic stage (e.g., 2 weeks or 3 weeks after the onset of cerebral infarction).

The screening method for a therapeutic agent for cerebral infarction of the present invention comprises the step of determining an effect of the test substance administered to the nonhuman primate animal model of the present invention. The determination of an effect of the administered test substance on cerebral infarction in the nonhuman primate animal model of the present invention can be carried out, for example, by conducting the observation of a motor function such as mobility capability, rotational movement capability, retention of a sitting position, standing-up action, jumping movement capability, the ability to grab for an object, the ability to feed, or the ability to grip an object in an animal model individual, diagnosis by CT or MRI, an electromyogram, or evaluation based on NHPSS and/or mRS, and determining the presence or absence of improvement as compared with before administration of the test substance. The test substance determined to attain the improvement as compared with before administration of the test substance can be identified as a therapeutic agent for cerebral infarction. The test substance determined to attain the improvement as compared with before administration of the test substance as a result of being administered to the nonhuman primate animal model of the present invention at a subacute to chronic stage can be identified as a therapeutic agent for cerebral infarction at a subacute to chronic stage. The test substance determined to attain the improvement as compared with before administration of the test substance as a result of being administered to the nonhuman primate animal model of penetrating branch infarction (e.g., lacunar infarction and/or BAD) of the present invention can be identified as a therapeutic agent for cerebral infarction which is penetrating branch infarction (e.g., lacunar infarction and/or BAD). The test substance determined to attain the improvement as compared with before administration of the test substance as a result of being administered to the nonhuman primate animal model of penetrating branch infarction (e.g., lacunar infarction and/or BAD) at a subacute to chronic stage of the present invention can be identified as a therapeutic agent for cerebral infarction which is penetrating branch infarction (e.g., lacunar infarction and/or BAD) at a subacute to chronic stage. The test substance determined to attain the improvement as compared with before administration of the test substance as a result of being administered to the nonhuman primate animal model of cerebral infarction having brain damage in a penetrating branch territory of the present invention can be identified as a therapeutic agent for cerebral infarction which is cerebral infarction having brain damage in a penetrating branch territory.

### 3. Pharmaceutical composition and treatment method, etc.

The present invention provides a pharmaceutical composition for the treatment of cerebral infarction (a pharmaceutical composition for use in the treatment of cerebral infarction), comprising a therapeutic agent for cerebral infarction, or a therapeutic agent for cerebral infarction which is penetrating branch infarction, cerebral infarction having brain damage in a penetrating branch territory, or cerebral infarction at a subacute to chronic stage, identified by the screening method for a therapeutic agent for cerebral infarction of the present invention. The present invention also provides a pharmaceutical composition for the treatment of penetrating branch infarction (e.g., for the treatment of penetrating branch infarction at a subacute to chronic stage, or for the treatment of penetrating branch infarction at a chronic stage), a pharmaceutical composition for the treatment of cerebral infarction at a subacute to chronic stage (e.g., for the treatment of cerebral infarction at a chronic stage), a pharmaceutical composition for the treatment of cerebral infarction having brain damage in a penetrating branch territory (e.g., for the treatment of cerebral infarction at a subacute to chronic stage, or for the treatment of cerebral infarction at a chronic stage), a pharmaceutical composition for the treatment of cerebral infarction having local brain damage (e.g., for the treatment of cerebral infarction at a subacute to chronic stage, or for the treatment of cerebral infarction at a chronic stage), a pharmaceutical composition for the treatment of cerebral infarction with mRS of 2 or more (e.g., for the treatment of cerebral infarction at a subacute to chronic stage, or for the treatment of cerebral infarction at a chronic stage), or a pharmaceutical composition for the treatment of cerebral infarction with NIHSS of 5 or more (e.g., for the treatment of cerebral infarction at a subacute to chronic stage, or for the treatment of cerebral infarction at a chronic stage), comprising a NeuroD1 protein or a polynucleotide encoding the NeuroD1 protein. In the present specification, the "polynucleotide encoding the NeuroD1 protein" means a polynucleotide comprising a nucleotide sequence encoding the NeuroD1 protein, though the term is described below in detail. In the present specification, the "nucleotide sequence encoding the NeuroD1 protein" means a nucleotide sequence translatable into the NeuroD1 protein. The "nucleotide sequence encoding the NeuroD1 protein" may be, for example, CDS (cording sequence; a sequence from a start codon to a stop codon (the start codon and the stop codon inclusive)) encoding the NeuroD1 protein. In a typical embodiment, the polynucleotide encoding the NeuroD1 protein encodes the NeuroD1 protein in an expressible state. In the present invention, the phrase "encoding ... in an expressible state" means that a nucleotide sequence encoding the NeuroD1 protein is placed in an operable state (e.g., in frame) under the control of a sequence(s) that enables or promotes transcription into or translation of mRNA (e.g., a promoter, an enhancer, a Kozak sequence, a transcriptional response sequence, a terminator, a polyadenylation signal, a 5'-terminal cap structure of mRNA, and a 3'-terminal polyA sequence of mRNA). In the present specification, for example, the pharmaceutical composition for the treatment of penetrating branch infarction, for the treatment of cerebral infarction at a subacute to chronic stage, for the treatment of cerebral infarction having brain damage in a penetrating branch territory, for the treatment of cerebral infarction having local brain damage, for the treatment of cerebral infarction with mRS of 2 or more, or for the treatment of cerebral infarction with NIHSS of 5 or more, comprising a NeuroD1 protein or a polynucleotide encoding the NeuroD1 protein is also referred to as the "pharmaceutical composition of the present invention". The present invention further provides a method for producing a pharmaceutical composition for the treatment of penetrating branch infarction, a pharmaceutical composition for the treatment of cerebral infarction at a subacute to chronic stage, a pharmaceutical composition for the treatment of cerebral infarction having brain damage in a penetrating branch territory, a pharmaceutical composition for the treatment of cerebral infarction having local brain damage, a pharmaceutical composition for the treatment of cerebral infarction with mRS of 2 or more, and/or a pharmaceutical composition for the treatment of cerebral infarction with NIHSS of 5 or more, comprising producing the pharmaceutical composition using a NeuroD1 protein or a polynucleotide encoding the NeuroD1 protein.

The "NeuroD1" (neuronal differentiation 1) is a basic helix-loop-helix (bHLH) transcriptional factor that belongs to the NeuroD family. The NeuroD1 protein activates the transcription of a gene comprising a specific DNA sequence known as E-box. Specifically, the NeuroD1 protein has transcriptional factor activity. The NeuroD1 has a central function of inducing the differentiation of neural stem cells into neurons. It is known that glial cells such as astrocytes or NG2 cells can be converted into the nerve by the ectopic expression of NeuroD1 in the glial cells (Patent Literature 2). The glial cells are classified into four types of cells, astrocytes, oligodendrocytes, ependymal cells, and microglia.

The NeuroD1 protein may be a protein of the following (i) or (ii):
(i) a protein consisting of the amino acid sequence as set forth by SEQ ID NO: 6 or 8 or an amino acid sequence having 90% or higher, preferably 95% or higher, more preferably 98% or higher, further preferably 99% or higher, for example, 99.4% or higher or 99.5% or higher sequence identity to the amino acid sequence as set forth by SEQ ID NO: 6 or 8, and
(ii) a protein consisting of an amino acid sequence derived from the amino acid sequence as set forth by SEQ ID NO: 6 or 8 by the insertion, deletion, substitution, and/or addition of 1 to 50 (e.g., 1 or 2, 1 to 3, 1 to 5, 1 to 7, 1 to 10, or 1 to 35) amino acids.

The NeuroD1 protein to be used in the pharmaceutical composition of the present invention preferably has transcriptional factor activity. SEQ ID NOs: 6 and 8 are the amino acid sequences of mouse NeuroD1 protein and human NeuroD1 protein, respectively, and are polypeptide sequences encoded by CDS of mouse NeuroD1 gene and CDS of human NeuroD1 gene, respectively.

In one embodiment, the NeuroD1 is human NeuroD1. The human NeuroD1 protein is a protein of the following (i) or (ii):
(i) a protein that consists of the amino acid sequence as set forth by SEQ ID NO: 8 or an amino acid sequence having 98% or higher, preferably 99% or higher, more preferably 99.4% or higher or 99.5% or higher sequence identity to the amino acid sequence as set forth by SEQ ID NO: 8, and has transcriptional factor activity, and
(ii) a protein that consists of an amino acid sequence derived from the amino acid sequence as set forth by SEQ ID NO: 8 by the insertion, deletion, substitution, and/or addition of 1 to 10 (e.g., 1 or 2, 1 to 3, 1 to 5, or 1 to 7) amino acids, and has transcriptional factor activity.

In one embodiment, the NeuroD1 is mouse NeuroD1. The mouse NeuroD1 protein is a protein of the following (i) or (ii):
(i) a protein that consists of the amino acid sequence as set forth by SEQ ID NO: 6 or an amino acid sequence having 98% or higher, preferably 99% or higher, more preferably 99.4% or higher or 99.5% or higher sequence identity to the amino acid sequence as set forth by SEQ ID NO: 6, and has transcriptional factor activity, and
(ii) a protein that consists of an amino acid sequence derived from the amino acid sequence as set forth by SEQ ID NO: 6 by the insertion, deletion, substitution, and/or addition of 1 to 10 (e.g., 1 or 2, 1 to 3, 1 to 5, or 1 to 7) amino acids, and has transcriptional factor activity.

Whether or not a certain protein has transcriptional factor activity can be readily determined by those skilled in the art, for example, by integrating a polynucleotide encoding the protein into a vector having an arbitrary promoter to produce a recombinant vector, transferring the vector to cells, expressing the protein, measuring the expression level of a gene whose expression (transcription) is regulated by the protein functioning as a transcriptional factor (in the present specification, also referred to as a regulated gene), and evaluating the presence or absence of change (increase or decrease) in the expression level of the regulated gene as compared with cells without the vector introduced. For example, when the expression level of the regulated gene whose expression is known to be upregulated by the NeuroD1 functioning as a transcriptional factor is increased in cells harboring a vector comprising a polynucleotide encoding NeuroD1, the NeuroD1 protein can be determined as having transcriptional factor activity.

The NeuroD1 protein contained in the pharmaceutical composition of the present invention may be incorporated in any drug delivery system (DDS) as long as the protein is delivered to the cells of interest. In one embodiment, the pharmaceutical composition of the present invention comprises the NeuroD1 protein, and the protein is incorporated in DDS. In one embodiment, the pharmaceutical composition of the present invention comprises the NeuroD1 protein, and the protein may be encapsulated into a drug delivery carrier. In one embodiment, the pharmaceutical composition of the present invention comprises the NeuroD1 protein, and the protein may be encapsulated into, but not limited to, a nanoparticle such as a lipid nanoparticle or exosome.

In one embodiment, the polynucleotide encoding the NeuroD1 protein is a polynucleotide selected from the group consisting of the following (i) to (iv):
(i) a polynucleotide comprising a nucleotide sequence encoding a protein consisting of the amino acid sequence as set forth by SEQ ID NO: 6 or 8 or an amino acid sequence having 90% or higher, preferably 95% or higher, more preferably 98% or higher, further preferably 99% or higher, for example, 99.4% or higher or 99.5% or higher sequence identity to the amino acid sequence as set forth by SEQ ID NO: 6 or 8,
(ii) a polynucleotide comprising a nucleotide sequence encoding a protein consisting of an amino acid sequence derived from the amino acid sequence as set forth by SEQ ID NO: 6 or 8 by the insertion, deletion, substitution, and/or addition of 1 to 50 (e.g., 1 or 2, 1 to 3, 1 to 5, 1 to 7, 1 to 10, or 1 to 35) amino acids,
(iii) a polynucleotide comprising the nucleotide sequence as set forth by SEQ ID NO: 5, 7, or 10 or comprising a nucleotide sequence having 90% or higher, preferably 95% or higher, more preferably 98% or higher, further preferably 99% or higher, for example, 99.4% or higher or 99.5% or higher sequence identity to the nucleotide sequence as set forth by SEQ ID NO: 5, 7, or 10, and
(iv) a polynucleotide comprising a nucleotide sequence derived from the nucleotide sequence as set forth by SEQ ID NO: 5, 7, or 10 by the insertion, deletion, substitution, and/or addition of 1 to 50 (e.g., 1 or 2, 1 to 3, 1 to 5, 1 to 7, 1 to 10, or 1 to 35) bases.

The NeuroD1 protein encoded by any of these polynucleotides preferably has transcriptional factor activity. The nucleotide sequence as set forth by SEQ ID NO: 5 is CDS of mouse NeuroD1 gene, and the nucleotide sequence as set forth by SEQ ID NO: 7 or 10 is CDS of human NeuroD1 gene. The nucleotide sequence as set forth by SEQ ID NO: 10 is a nucleotide sequence that differs only in a stop codon from the nucleotide sequence as set forth by SEQ ID NO: 7.

In one embodiment, the polynucleotide encoding the human NeuroD1 protein is a polynucleotide selected from the group consisting of the following (i) to (iv):
(i) a polynucleotide comprising a nucleotide sequence encoding a protein that consists of the amino acid sequence as set forth by SEQ ID NO: 8 or an amino acid sequence having 98% or higher, preferably 99% or higher, more preferably 99.4% or higher or 99.5% or higher sequence identity to the amino acid sequence as set forth by SEQ ID NO: 8, and has transcriptional factor activity,
(ii) a polynucleotide comprising a nucleotide sequence encoding a protein that consists of an amino acid sequence derived from the amino acid sequence as set forth by SEQ ID NO: 8 by the insertion, deletion, substitution, and/or addition of 1 to 10 (e.g., 1 or 2, 1 to 3, 1 to 5, or 1 to 7) amino acids, and has transcriptional factor activity,
(iii) a polynucleotide comprising the nucleotide sequence as set forth by SEQ ID NO: 7 or 10 or a nucleotide sequence having 98% or higher, preferably 99% or higher, more preferably 99.4% or higher or 99.5% or higher sequence identity to the nucleotide sequence as set forth by SEQ ID NO: 7 or 10, and encoding a protein having transcriptional factor activity, and
(iv) a polynucleotide comprising a nucleotide sequence derived from the nucleotide sequence as set forth by SEQ ID NO: 7 or 10 by the insertion, deletion, substitution, and/or addition of 1 to 10 (e.g., 1 or 2, 1 to 3, 1 to 5, or 1 to 7) bases, and encoding a protein having transcriptional factor activity.

In one embodiment, the polynucleotide encoding the mouse NeuroD1 protein is a polynucleotide selected from the group consisting of the following (i) to (iv):
(i) a polynucleotide comprising a nucleotide sequence encoding a protein that consists of the amino acid sequence as set forth by SEQ ID NO: 6 or an amino acid sequence having 98% or higher, preferably 99% or higher, more preferably 99.4% or higher or 99.5% or higher sequence identity to the amino acid sequence as set forth by SEQ ID NO: 6, and has transcriptional factor activity,
(ii) a polynucleotide comprising a nucleotide sequence encoding a protein that consists of an amino acid sequence derived from the amino acid sequence as set forth by SEQ ID NO: 6 by the insertion, deletion, substitution, and/or addition of 1 to 10 (e.g., 1 or 2, 1 to 3, 1 to 5, or 1 to 7) amino acids, and has transcriptional factor activity,
(iii) a polynucleotide comprising the nucleotide sequence as set forth by SEQ ID NO: 5 or a nucleotide sequence having 98% or higher, preferably 99% or higher, more preferably 99.4% or higher or 99.5% or higher sequence identity to the nucleotide sequence as set forth by SEQ ID NO: 5, and encoding a protein having transcriptional factor activity, and
(iv) a polynucleotide comprising a nucleotide sequence derived from the nucleotide sequence as set forth by SEQ ID NO: 5 by the insertion, deletion, substitution, and/or addition of 1 to 10 (e.g., 1 or 2, 1 to 3, 1 to 5, or 1 to 7) bases, and encoding a protein having transcriptional factor activity.

The polynucleotide encoding the NeuroD1 protein, for example, the polynucleotide encoding the NeuroD1 protein in an expressible state, may be subjected to codon optimization. For the codon optimization, a method known in the art can be used.

In one embodiment, the polynucleotide encoding the NeuroD1 protein is a polynucleotide selected from the group consisting of the following (i) to (iii):
(i) a polynucleotide comprising a nucleotide sequence having 60% or higher, preferably 70% or higher, more preferably 80% or higher, further preferably 90% or higher or 95% or higher sequence identity to the nucleotide sequence as set forth by SEQ ID NO: 10, and encoding a protein consisting of the amino acid sequence as set forth by SEQ ID NO: 8,
(ii) a polynucleotide comprising a nucleotide sequence having 60% or higher, preferably 70% or higher, more preferably 80% or higher, further preferably 90% or higher or 95% or higher sequence identity to the nucleotide sequence as set forth by SEQ ID NO: 7, and encoding a protein consisting of the amino acid sequence as set forth by SEQ ID NO: 8, and
(iii) a polynucleotide comprising a nucleotide sequence having 60% or higher, preferably 70% or higher, more preferably 80% or higher, further preferably 90% or higher or 95% or higher sequence identity to the nucleotide sequence as set forth by SEQ ID NO: 5, and encoding a protein consisting of the amino acid sequence as set forth by SEQ ID NO: 6.

In the present specification, the "sequence identity" means the ratio (%) of identical residues between sequences (usually, the ratio % of identical residues to the full length of the sequence of interest) when a reference biological sequence (a nucleotide sequence or an amino acid sequence, etc.) is aligned with the biological sequence of interest. The "sequence identity" can be calculated as the value of identity obtained from parameters prepared in default using, for example, EMBOSS Needle (Weizhong, L. et al., Nucleic Acids Res., vol. 43, pp. W580-W584, 2015). The parameters are as follows.
Gap Open Penalty = 10
Gap Extend Penalty = 0.5
Matrix = EBLOSUM62
End Gap Penalty = false

The pharmaceutical composition of the present invention comprises a NeuroD1 protein or a polynucleotide encoding the NeuroD1 protein. In one embodiment, the pharmaceutical composition of the present invention comprises a polynucleotide selected from the group consisting of the following (i) to (xiii):
(i) a polynucleotide comprising a nucleotide sequence encoding a protein that consists of an amino acid sequence having 98% or higher, preferably 99% or higher, more preferably 99.4% or higher or 99.5% or higher sequence identity to the amino acid sequence as set forth by SEQ ID NO: 6 or 8, and has transcriptional factor activity,
(ii) a polynucleotide comprising a nucleotide sequence encoding a protein that consists of an amino acid sequence derived from the amino acid sequence as set forth by SEQ ID NO: 6 or 8 by the insertion, deletion, substitution, and/or addition of 1 to 10 (e.g., 1 or 2, 1 to 3, 1 to 5, or 1 to 7) amino acids, and has transcriptional factor activity,
(iii) a polynucleotide comprising a nucleotide sequence encoding a protein consisting of the amino acid sequence as set forth by SEQ ID NO: 6 or 8,
(iv) a polynucleotide comprising the nucleotide sequence as set forth by SEQ ID NO: 5, 7, or 10 or a nucleotide sequence having 98% or higher, preferably 99% or higher, more preferably 99.4% or higher or 99.5% or higher sequence identity to the nucleotide sequence as set forth by SEQ ID NO: 5, 7 or 10, and encoding a protein having transcriptional factor activity,
(v) a polynucleotide comprising a nucleotide sequence derived from the nucleotide sequence as set forth by SEQ ID NO: 5,7, or 10 by the insertion, deletion, substitution, and/or addition of 1 to 10 (e.g., 1 or 2, 1 to 3, 1 to 5, or 1 to 7) bases, and encoding a protein having transcriptional factor activity,
(vi) a polynucleotide comprising a nucleotide sequence having 70% or higher sequence identity to the nucleotide sequence as set forth by SEQ ID NO: 7 or 10,
(vii) a polynucleotide comprising a nucleotide sequence having 70% or higher sequence identity to the nucleotide sequence as set forth by SEQ ID NO: 7 or 10, and encoding a protein consisting of the amino acid sequence as set forth by SEQ ID NO: 8,
(viii) a polynucleotide comprising a nucleotide sequence having 70% or higher sequence identity to the nucleotide sequence as set forth by SEQ ID NO: 7, and encoding a protein consisting of the amino acid sequence as set forth by SEQ ID NO: 8,
(ix) a polynucleotide comprising a nucleotide sequence having 70% or higher sequence identity to the nucleotide sequence as set forth by SEQ ID NO: 10, and encoding a protein consisting of the amino acid sequence as set forth by SEQ ID NO: 8,
(x) a polynucleotide comprising the nucleotide sequence as set forth by SEQ ID NO: 7 or 10,
(xi) a polynucleotide comprising the nucleotide sequence as set forth by SEQ ID NO: 7,
(xii) a polynucleotide comprising the nucleotide sequence as set forth by SEQ ID NO: 10, and
(xiii) a polynucleotide comprising or consisting of a nucleotide sequence having 70% or higher (e.g., 80% or higher, 90% or higher, or 95% or higher) sequence identity to the nucleotide sequence of positions 44 to 1114, the nucleotide sequence of positions 44 to 1120, or the nucleotide sequence of positions 1 to 1231, of SEQ ID NO: 11 or 13, and preferably, the polynucleotide encoding a protein (preferably having transcriptional factor activity) consisting of an amino acid sequence having 90% or higher (e.g., 95% or higher) sequence identity to the amino acid sequence as set forth by SEQ ID NO: 8.

The polynucleotide encoding the NeuroD1 protein, for example, the polynucleotide encoding the NeuroD1 protein in an expressible state, may be, but not limited to, DNA or RNA such as mRNA, or a combination thereof (a chimeric nucleic acid). The polynucleotide may be a natural nucleic acid or an artificial nucleic acid (e.g., a nucleic acid containing a natural nucleobase and/or an artificial nucleobase).

In one embodiment, the polynucleotide encoding the NeuroD1 protein in an expressible state may be contained in an expression vector. The expression vector comprising the polynucleotide encoding the NeuroD1 protein according to the present invention comprises a polynucleotide (e.g., DNA or RNA) comprising a nucleotide sequence encoding the NeuroD1 protein in an operable state under the control of a sequence that enables or promotes transcription into or translation of mRNA, i.e., the expression vector comprises the polynucleotide encoding the NeuroD1 protein in an expressible state. In the present invention, the sequence that enables or promotes transcription into or translation of mRNA (e.g., a promoter, an enhancer, a Kozak sequence, a transcriptional response sequence, a terminator, a polyA sequence addition signal, a 5'-terminal cap structure of mRNA, or a 3'-terminal polyA sequence of mRNA) is not particularly limited as long as the sequence is capable of functioning *in vivo* in an organism to be treated. The promoter may be an arbitrary promoter such as a constitutive promoter, a cell-specific promoter, a tissue-specific promoter, a transient promoter, or an inducible promoter according to intended use. Examples of the promoter capable of functioning *in vivo* in a human include, but are not limited to, human GFAP promoter, CMV promoter, EF-1α promoter, PGK promoter, and MSCV promoter. The human GFAP promoter may comprise the nucleotide sequence as set forth by SEQ ID NO: 14. For delivering the polynucleotide encoding the NeuroD1 protein to astrocytes, it is particularly preferred to use GFAP promoter.

In the present invention, the expression vector may be an arbitrary vector such as a virus vector or a plasmid vector. The expression vector used in the present invention may be, but not limited to, an adeno-associated virus (AAV) vector, an adenovirus vector, a retrovirus vector, a Sendai virus vector, or a lentivirus vector and is particularly preferably an AAV vector. When the expression vector is a virus vector, the expression vector may be a nucleic acid vector in which a polynucleotide comprising a nucleotide sequence encoding the NeuroD1 protein is packaged in a virus particle under the control of a sequence that enables or promotes transcription or translation of mRNA (a promoter, etc.).

In the present invention, the serotype of the AAV vector is not particularly limited as long as host cells are capable of expressing NeuroD1. For example, AAV1, AAV2, AAV3, AAV4, AAV5, AAV6, AAV7, AAV8, AAV9, or AAV10 can be used. In one embodiment, the serotype of AAV may be AAV9.

The expression vector can be produced by use of a method known in the art. As one example of the method for producing the AAV vector, an arbitrary promoter sequence (e.g., a human GFAP promoter sequence) and an arbitrary nucleic acid molecule sequence (e.g., a nucleotide sequence encoding the NeuroD1 protein) are inserted to a commercially available vector plasmid (e.g., pAAV-CMV Vector (Takara Bio Inc.)) to produce an AAV vector plasmid. Also, a plasmid containing AAV Rep gene and Cap gene which complement an AAV helper function, and a plasmid containing a helper virus-derived gene (e.g., regions VA, E2A, and E4) which promotes AAV replication are produced. Packaging cells are cotransfected with these three plasmids so that recombinant AAV (i.e., an AAV vector) is produced in the cells. Then, the packaging cells are cultured to produce an AAV vector. The AAV vector is purified by use of a standard technique known in the art. Examples of the packaging cells used include, but are not limited to, mammalian cells, specifically, 293T cells, A549 cells, HeLa cells, and HEK293 cells. For example, cesium chloride density gradient centrifugation, DNA and RNA digestion using Benzonase, sucrose gradient centrifugation, iodixanol density gradient centrifugation, ultrafiltration, diafiltration, affinity chromatography, ion-exchange chromatography, polyethylene glycol precipitation, or ammonium sulfate precipitation can be used as a purification method.

In one embodiment, the pharmaceutical composition of the present invention comprises an AAV vector, a retrovirus vector, a Sendai virus vector, or a lentivirus vector comprising the polynucleotide encoding the NeuroD1 protein. In one embodiment, the pharmaceutical composition of the present invention comprises an AAV vector comprising the polynucleotide encoding the NeuroD1 protein. In one embodiment, the pharmaceutical composition of the present invention comprises an AAV9 vector comprising the polynucleotide encoding the NeuroD1 protein.

The "mRNA" refers to RNA comprising a nucleotide sequence encoding (at least one) polypeptide (e.g., a naturally occurring polypeptide, a polypeptide derived from the naturally occurring polypeptide by the insertion, deletion, substitution, and/or addition of an amino acid, or a non-naturally occurring polypeptide) under the control of a sequence that enables or promotes translation of mRNA (e.g., a Kozak sequence, a start codon, a 5'-terminal cap structure of mRNA, a 5' untranslated region (UTR), 3' UTR, or a 3'-terminal polyA sequence of mRNA). The mRNA is capable of being translated to produce the encoded polypeptide, for example, *in vitro* or *in vivo.* In one embodiment, the mRNA is translated *in vivo* to generate a polypeptide encoded thereby.

In one embodiment, the polynucleotide encoding the NeuroD1 protein may be mRNA encoding the NeuroD1 protein. In the present invention, the "mRNA encoding the NeuroD1 protein" means RNA comprising a polynucleotide comprising a nucleotide sequence encoding the NeuroD1 protein under the control of a sequence that enables or promotes translation of mRNA. The mRNA encoding the NeuroD1 protein according to the present invention typically comprises a polynucleotide comprising a nucleotide sequence encoding the NeuroD1 protein in an operable state under the control of a sequence that enables or promotes translation of mRNA, i.e., comprises the polynucleotide encoding the NeuroD1 protein in an expressible state. In one embodiment, the mRNA encoding the NeuroD1 protein according to the present invention is a polynucleotide encoding the NeuroD1 protein, comprising a functional sequence for expressing the NeuroD1 protein (examples of the functional sequence include, but are not limited to, CDS, 5' UTR, 3' UTR, a 5' cap structure, and a polyA sequence).

Each polynucleotide described in the present specification may be described as a polynucleotide defined by a nucleotide sequence containing "T" as a representative DNA sequence. When the polynucleotide is RNA (e.g., mRNA), "T" is replaced with "U". Thus, for example, even if a nucleotide sequence defined by a particular SEQ ID NO represents a DNA sequence, the nucleotide sequence of RNA (e.g., mRNA) comprising the nucleotide sequence defined by the particular SEQ ID NO is understood as an RNA sequence in which each "T" in the DNA sequence is replaced with "U". Bases "adenine (A)", "thymine (T)", "guanine (G)", "cytosine (C)", and "uracil (U)" which constitute nucleotide sequences (e.g., the nucleotide sequences as set forth by SEQ ID NOs: 1 to 3, 5, 7, and 10 to 14) as well as nucleosides and nucleotides containing these bases may be in a natural form or a modified form corresponding thereto and/or may have other modifications (methylation, etc.) on an individual base, nucleoside, or nucleotide basis, unless otherwise specified in the sequence listing.

The mRNA may comprise CDS, 5' UTR, 3' UTR, a 5' cap structure, and a polyA sequence. The CDS contained in the mRNA encoding the NeuroD1 protein may be subjected to codon optimization. A stop codon positioned at the 3' end of the CDS is not particularly limited as long as the translation of the mRNA into the NeuroD1 protein can be terminated. Any stop codon of TAA, TGA, and TAG may be used. A plurality of stop codons may be further used continuously to the stop codon contained in the CDS (e.g., TGATAG may be used in continuously to the stop codon TAA contained in the CDS). The UTR contained in the mRNA encoding the NeuroD1 protein is not particularly limited as long as the NeuroD1 protein can be expressed. The UTR may be, for example, NeuroD1 gene-derived 5' UTR and/or 3' UTR or may be 5' UTR and/or 3' UTR derived from a heterologous gene (e.g., human α globin gene or human β globin gene). The 5' UTR and the 3' UTR may be derived from the same heterologous gene or may be derived from different heterologous genes. The UTR may be in a natural form or may be in a variant form in which the sequence of the natural form of UTR is altered by the insertion, deletion, substitution, and/or addition of one or several (e.g., 2, 3, 4, 5, or 6) nucleotides. In the present specification, the "gene-derived UTR" includes not only the natural form of UTR but such a variant form of UTR. The 5' UTR and/or the 3' UTR may be a plurality of UTRs linked directly or via a spacer sequence. The 5' UTR may comprise a partial Kozak sequence (e.g., a sequence on the 5' terminal side from the start codon, within Kozak sequence). Examples of the 5' cap structure include Cap-0, Cap-1, and Cap-2 (Anand R et al., Nucleic Acids Res., vol. 44 (16), pp. 7511-7526, 2016). The Cap-0 has a structure where guanosine is bound with the 5' end of mRNA via a triphosphate bond (5'-5' bond) and position 7 of guanine base of the guanosine is methylated, and is also called 7-methylguanylate cap. The Cap-1 has a structure where, in addition to Cap-0, position 2 of ribose of the first 5'-terminal nucleotide (nucleotide bound with the guanosine of Cap-0 through a 5'-5' bond) of mRNA is methylated. The Cap-2 has a structure where, in addition to Cap-0, position 2 of each ribose of the first and second 5'-terminal nucleotides of mRNA is methylated. The length of the polyA sequence is not particularly limited as long as the sequence has functions of mRNA stabilization, nuclear export, translation, and the like. The length may be, for example, 20 to 1000 nucleotides (e.g., 30 to 500, 50 to 200, 60 to 150, 70 to 130, 70 to 120, 70 to 90, 70 to 85, 70 to 80, or 75 to 85; or at least 70, at least 75, or 79 nucleotides). The polyA sequence may be a plurality of polyA sequences linked via a spacer sequence consisting of a nucleotide other than A (International Publication No. WO2016/005004, etc.).

In one embodiment, the mRNA encoding the NeuroD1 protein is mRNA of the following (i) or (ii):
(i) mRNA comprising a nucleotide sequence encoding a protein consisting of the amino acid sequence as set forth by SEQ ID NO: 6 or 8 or an amino acid sequence having 90% or higher, preferably 95% or higher, more preferably 98% or higher, further preferably 99% or higher, for example, 99.4% or higher or 99.5% or higher sequence identity to the amino acid sequence as set forth by SEQ ID NO: 6 or 8; and
(ii) mRNA comprising a nucleotide sequence encoding a protein consisting of an amino acid sequence derived from the amino acid sequence as set forth by SEQ ID NO: 6 or 8 by the insertion, deletion, substitution, and/or addition of 1 to 50 (e.g., 1 or 2, 1 to 3, 1 to 5, 1 to 7, 1 to 10, or 1 to 35) amino acids. The protein (NeuroD1 protein) encoded by the polynucleotide preferably has transcriptional factor activity.

In one embodiment, the mRNA encoding the NeuroD1 protein is mRNA comprising a nucleotide sequence encoding a protein consisting of the amino acid sequence as set forth by SEQ ID NO: 8. In one embodiment, the mRNA encoding the NeuroD1 protein is mRNA comprising a nucleotide sequence encoding a polypeptide having 90% or higher (e.g., 95% or higher, 98% or higher, 99% or higher, 99.4% or higher, or 99.5% or higher) sequence identity to the protein consisting of the amino acid sequence as set forth by SEQ ID NO: 8. The NeuroD1 protein encoded by the mRNA preferably has transcriptional factor activity.

In one embodiment, the mRNA encoding the NeuroD1 protein is mRNA comprising a nucleotide sequence having 70% or higher (e.g., 80% or higher, 90% or higher, or 95% or higher) sequence identity to the nucleotide sequence as set forth by SEQ ID NO: 7 or 10, and encoding a protein consisting of the amino acid sequence as set forth by SEQ ID NO: 8. In one embodiment, the mRNA encoding the NeuroD1 protein is mRNA comprising a nucleotide sequence having 70% or higher (e.g., 80% or higher, 90% or higher, or 95% or higher) sequence identity to the nucleotide sequence as set forth by SEQ ID NO: 7 or 10, and encoding a polypeptide having 90% or higher (e.g., 95% or higher, 98% or higher, 99% or higher, 99.4% or higher, or 99.5% or higher) sequence identity to the protein consisting of the amino acid sequence as set forth by SEQ ID NO: 8. In one embodiment, the mRNA encoding the NeuroD1 protein is mRNA comprising a nucleotide sequence having 70% or higher (e.g., 80% or higher, 90% or higher, or 95% or higher) sequence identity to the nucleotide sequence as set forth by SEQ ID NO: 10, and encoding a protein consisting of the amino acid sequence as set forth by SEQ ID NO: 8. In one embodiment, the mRNA encoding the NeuroD1 protein is mRNA comprising a nucleotide sequence having 70% or higher (e.g., 80% or higher, 90% or higher, or 95% or higher) sequence identity to the nucleotide sequence as set forth by SEQ ID NO: 10, and encoding a polypeptide having 90% or higher (e.g., 95% or higher, 98% or higher, 99% or higher, 99.4% or higher, or 99.5% or higher) sequence identity to the protein consisting of the amino acid sequence as set forth by SEQ ID NO: 8. In one embodiment, the mRNA encoding the NeuroD1 protein is mRNA comprising the nucleotide sequence as set forth by SEQ ID NO: 10. In one embodiment, the mRNA encoding the NeuroD1 protein is mRNA comprising a nucleotide sequence having 70% or higher (e.g., 80% or higher, 90% or higher, or 95% or higher) sequence identity to the nucleotide sequence as set forth by SEQ ID NO: 7, and encoding a protein consisting of the amino acid sequence as set forth by SEQ ID NO: 8. In one embodiment, the mRNA encoding the NeuroD1 protein is mRNA comprising a nucleotide sequence having 70% or higher (e.g., 80% or higher, 90% or higher, or 95% or higher) sequence identity to the nucleotide sequence as set forth by SEQ ID NO: 7, and encoding a polypeptide having 90% or higher (e.g., 95% or higher, 98% or higher, 99% or higher, 99.4% or higher, or 99.5% or higher) sequence identity to the protein consisting of the amino acid sequence as set forth by SEQ ID NO: 8. In one embodiment, the mRNA encoding the NeuroD1 protein is mRNA comprising the nucleotide sequence as set forth by SEQ ID NO: 7. The NeuroD1 protein encoded by the mRNA preferably has transcriptional factor activity.

In one embodiment, the mRNA encoding the NeuroD1 protein is mRNA comprising a nucleotide sequence having 70% or higher (e.g., 80% or higher, 90% or higher, or 95% or higher) sequence identity to the nucleotide sequence of positions 44 to 1114 or positions 44 to 1120 of SEQ ID NO: 11 or 13, and encoding a polypeptide having 90% or higher (e.g., 95% or higher, 98% or higher, 99% or higher, 99.4% or higher, or 99.5% or higher) sequence identity to a protein consisting of the amino acid sequence as set forth by SEQ ID NO: 8. The protein preferably has transcriptional factor activity. In one embodiment, the mRNA encoding the NeuroD1 protein is mRNA comprising a nucleotide sequence having 70% or higher (e.g., 80% or higher, 90% or higher, or 95% or higher) sequence identity to the nucleotide sequence of positions 44 to 1114 or positions 44 to 1120 of SEQ ID NO: 11 or 13, and encoding the protein consisting of the amino acid sequence as set forth by SEQ ID NO: 8. In one embodiment, the mRNA encoding the NeuroD1 protein is mRNA comprising the nucleotide sequence of positions 44 to 1114 or positions 44 to 1120 of SEQ ID NO: 11 or 13. The nucleotide sequence of positions 44 to 1114 of SEQ ID NO: 11 or 13 is CDS of human NeuroD1 gene, and the nucleotide sequence of positions 44 to 1120 of SEQ ID NO: 11 or 13 comprises CDS of human NeuroD1 gene and a plurality of stop codons.

In one embodiment, the mRNA encoding the NeuroD1 protein comprises 5' UTR and/or 3' UTR. In one embodiment, the mRNA encoding the NeuroD1 protein is mRNA comprising 5' UTR and/or 3' UTR derived from a heterologous gene (e.g., human α globin gene or human β globin gene). In one embodiment, the mRNA encoding the NeuroD1 protein is mRNA comprising 5' UTR and/or 3' UTR derived from human α globin gene. In one embodiment, the mRNA encoding the NeuroD1 protein is mRNA comprising a nucleotide sequence encoding a protein consisting of the amino acid sequence as set forth by SEQ ID NO: 8, the mRNA further comprising 5' UTR and/or 3' UTR derived from human α globin gene. In one embodiment, the mRNA encoding the NeuroD1 protein is mRNA comprising a nucleotide sequence having 70% or higher (e.g., 80% or higher, 90% or higher, or 95% or higher) sequence identity to the nucleotide sequence of positions 44 to 1114 or positions 44 to 1120 of SEQ ID NO: 11 or 13, and encoding the protein consisting of the amino acid sequence as set forth by SEQ ID NO: 8, the mRNA further comprising 5' UTR and/or 3' UTR derived from human α globin gene. In one embodiment, the mRNA encoding the NeuroD1 protein is mRNA comprising the nucleotide sequence of positions 44 to 1114 or positions 44 to 1120 of SEQ ID NO: 11 or 13, the mRNA further comprising 5' UTR and/or 3' UTR derived from human α globin gene. In one embodiment, the mRNA encoding the NeuroD1 protein is mRNA comprising 5' UTR consisting of a nucleotide sequence of positions 4 to 43 of SEQ ID NO: 11 or 13 and/or 3' UTR consisting of a nucleotide sequence of positions 1121 to 1231 of SEQ ID NO: 11 or 13. In one embodiment, the mRNA encoding the NeuroD1 protein comprises a polynucleotide consisting of a nucleotide sequence of positions 4 to 1231 of SEQ ID NO: 11 or 13. In one embodiment, the mRNA encoding the NeuroD1 protein is mRNA comprising a nucleotide sequence having 70% or higher (e.g., 80% or higher, 90% or higher, or 95% or higher) sequence identity to the nucleotide sequence of positions 4 to 1231 of SEQ ID NO: 11 or 13, and encoding the protein consisting of the amino acid sequence as set forth by SEQ ID NO: 8.

In one embodiment, the mRNA encoding the NeuroD1 protein comprises a polyA sequence. In one embodiment, the mRNA encoding the NeuroD1 protein is mRNA comprising a polyA sequence consisting of 50 to 200 nucleotides. In one embodiment, the mRNA encoding the NeuroD1 protein is mRNA comprising a polyA sequence consisting of 70 to 90 nucleotides. In one embodiment, the mRNA encoding the NeuroD1 protein is mRNA comprising a polyA sequence consisting of 75 to 85 nucleotides. In one embodiment, the mRNA encoding the NeuroD1 protein is mRNA comprising a polyA sequence consisting of 120 nucleotides. In one embodiment, the mRNA encoding the NeuroD1 protein is mRNA comprising a polyA sequence consisting of 79 nucleotides.

In one embodiment, the mRNA encoding the NeuroD1 protein comprises a polyA sequence of 30 to 500 nucleotides in length (e.g., 40 to 200, 50 to 200, 50 to 150, 50 to 100, 50 to 90, 60 to 150, 60 to 100, 60 to 90, 70 to 130, 70 to 120, 70 to 100, 70 to 90, 70 to 85, 70 to 80, 75 to 130, 75 to 120, 75 to 100, or 75 to 90; preferably, 74 to 84, 75 to 85, 75 to 83, or 76 to 82 nucleotides in length). In one embodiment, the mRNA encoding the NeuroD1 protein comprises a polyA sequence of 74 to 84 nucleotides in length. In one embodiment, the mRNA encoding the NeuroD1 protein comprises a polyA sequence of 75 to 85 nucleotides in length.

In one embodiment, the mRNA encoding the NeuroD1 protein comprises a nucleotide sequence encoding a protein consisting of the amino acid sequence as set forth by SEQ ID NO: 8, the mRNA further comprising 5' UTR and/or 3' UTR, and a polyA sequence. In one embodiment, the mRNA encoding the NeuroD1 protein comprises a nucleotide sequence having 70% or higher (e.g., 80% or higher, 90% or higher, or 95% or higher) sequence identity to the nucleotide sequence of positions 44 to 1114 or positions 44 to 1120 of SEQ ID NO: 11 or 13, and encoding the protein consisting of the amino acid sequence as set forth by SEQ ID NO: 8, and the mRNA further comprising 5' UTR and/or 3' UTR, and a polyA sequence. In one embodiment, the mRNA encoding the NeuroD1 protein comprises the nucleotide sequence of positions 44 to 1114 or positions 44 to 1120 of SEQ ID NO: 11 or 13, and the mRNA further comprising 5' UTR and/or 3' UTR, and a polyA sequence.

In one embodiment, the mRNA encoding the NeuroD1 protein is mRNA comprising a nucleotide sequence encoding a protein consisting of the amino acid sequence as set forth by SEQ ID NO: 8, the mRNA further comprising 5' UTR and/or 3' UTR derived from human α globin gene and a polyA sequence. In one embodiment, the mRNA encoding the NeuroD1 protein is mRNA comprising a nucleotide sequence having 70% or higher (e.g., 80% or higher, 90% or higher, or 95% or higher) sequence identity to the nucleotide sequence of positions 44 to 1114 or positions 44 to 1120 of SEQ ID NO: 11 or 13, and encoding the protein consisting of the amino acid sequence as set forth by SEQ ID NO: 8, the mRNA further comprising 5' UTR and/or 3' UTR derived from human α globin gene and a polyA sequence. In one embodiment, the mRNA encoding the NeuroD1 protein is mRNA comprising the nucleotide sequence of positions 44 to 1114 or positions 44 to 1120 of SEQ ID NO: 11 or 13, the mRNA further comprising 5' UTR and/or 3' UTR derived from human α globin gene and a polyA sequence. In one embodiment, the mRNA encoding the NeuroD1 protein is mRNA comprising 5' UTR consisting of a nucleotide sequence of positions 4 to 43 of SEQ ID NO: 11 or 13 and/or 3' UTR consisting of a nucleotide sequence of positions 1121 to 1231 of SEQ ID NO: 11 or 13 and a polyA sequence. In one embodiment, the mRNA encoding the NeuroD1 protein is mRNA comprising a polynucleotide consisting of a nucleotide sequence of positions 4 to 1231 of SEQ ID NO: 11 or 13, and a polyA sequence. In one embodiment, the mRNA encoding the NeuroD1 protein is mRNA comprising a nucleotide sequence having 70% or higher (e.g., 80% or higher, 90% or higher, or 95% or higher) sequence identity to the nucleotide sequence of positions 4 to 1231 of SEQ ID NO: 11 or 13, and encoding the protein consisting of the amino acid sequence as set forth by SEQ ID NO: 8, and a polyA sequence.

In one embodiment, the mRNA encoding the NeuroD1 protein is mRNA comprising the nucleotide sequence as set forth by SEQ ID NO: 7 or 10, the mRNA further comprising 5' UTR consisting of a nucleotide sequence of positions 4 to 43 of SEQ ID NO: 11 or 13 and/or 3' UTR consisting of a nucleotide sequence of positions 1121 to 1231 of SEQ ID NO: 11 or 13, and a polyA sequence. In one embodiment, the mRNA encoding the NeuroD1 protein is mRNA comprising a polynucleotide consisting of a nucleotide sequence of positions 4 to 1231 of SEQ ID NO: 11 or 13, and a polyA sequence. In one embodiment, the mRNA encoding the NeuroD1 protein is mRNA comprising a nucleotide sequence having 70% or higher (e.g., 80% or higher, 90% or higher, or 95% or higher) sequence identity to a nucleotide sequence of positions 4 to 1231 of SEQ ID NO: 11 or 13, and encoding a protein consisting of the amino acid sequence as set forth by SEQ ID NO: 8, and a polyA sequence.

The mRNA can be produced by use of a method known in the art. As one example of the method for producing the mRNA, arbitrary UTR (e.g., human α globin 5' UTR and 3' UTR) and a nucleotide sequence of an arbitrary nucleic acid molecule (e.g., a nucleotide sequence encoding the NeuroD1 protein) are inserted to a commercially available plasmid (e.g., a high copy plasmid for *E. coli* (e.g., pUC18 or pCU18)) to produce a plasmid. The produced plasmid can be allowed to proliferate using, for example, *E. coli,* and purified by use of a method known in the art. The purified plasmid can be linearized by use of a method known in the art, for example, but not limited to, a method using restriction enzymes and a buffer solution. The linearization reaction product can be purified by a method using, for example, PureLink^{(R)} (Thermo Fisher Scientific Inc.). The purification method may vary depending on the size of the linearization reaction product. The purified linear reaction product may be used directly as a DNA template, or the linear reaction product may be subjected to polymerase chain reaction (PCR) to form a DNA template. The formed DNA template can be transcribed using an *in vitro* transcription (IVT) system. This system typically contains a transcription buffer solution, nucleoside triphosphate (NTP), an RNase inhibitor, and polymerase (e.g., T7 RNA polymerase). In the present invention, the NTP may be in a natural form or a non-natural form (modified form). The DNA template can be removed by use of a method known in the art, for example, but not limited to, treatment with deoxyribonuclease I (DNase I). For example, a silica column may be used in the purification of RNA thus obtained by transcription. When the RNA comprises a polyA sequence, the RNA may be purified using, for example, Oligo dT column. The mRNA may be prepared through capping reaction and/or polyA addition reaction. The capping reaction can be performed by use of a method known in the art which involves adding a 5' cap structure to the 5' end of a sequence transcribed by IVT or adding a 5' cap structure to the 5' end during IVT. The method for capping may be, for example, a method using a vaccinia capping enzyme and 2'O-methyl transferase or a method using CleanCap^{(R)} (TriLink BioTechnologies, Inc.), and can be performed by use of a method known in the art which is not limited thereto. When the sequence transcribed by IVT comprises no polyA sequence, polyA addition reaction may be performed. The constructed mRNA can be purified by use of a method known in the art.

In one embodiment, the mRNA encoding the NeuroD1 protein may have a cap structure and may comprise a 5'-terminal sequence suitable for a capping method. In the case of using, for example, a capping method using CleanCap^{(R)} Reagent AG (TriLink BioTechnologies, Inc.), the mRNA may comprise a nucleotide sequence AGG at the 5' end. In one embodiment, the mRNA encoding the NeuroD1 protein may be mRNA comprising a nucleotide sequence (AGG) of positions 1 to 3 of SEQ ID NO: 11 or 13, 5' UTR, a nucleotide sequence encoding a protein consisting of the amino acid sequence as set forth by SEQ ID NO: 8, 3' UTR, and a polyA sequence, in order from the 5' terminal side. In one embodiment, the mRNA encoding the NeuroD1 protein may be mRNA comprising a nucleotide sequence (AGG) of positions 1 to 3 of SEQ ID NO: 11 or 13, 5' UTR, a nucleotide sequence having 70% or higher (e.g., 80% or higher, 90% or higher, or 95% or higher) sequence identity to the nucleotide sequence of positions 44 to 1114 or positions 44 to 1120 of SEQ ID NO: 11 or 13, and encoding a protein consisting of the amino acid sequence as set forth by SEQ ID NO: 8, 3' UTR, and a polyA sequence, in order from the 5' terminal side. In one embodiment, the mRNA encoding the NeuroD1 protein may be mRNA comprising a nucleotide sequence (AGG) of positions 1 to 3 of SEQ ID NO: 11 or 13, 5' UTR, the nucleotide sequence of positions 44 to 1114 or positions 44 to 1120 of SEQ ID NO: 11 or 13, 3' UTR, and a polyA sequence, in order from the 5' terminal side.

In one embodiment, the mRNA encoding the NeuroD1 protein is mRNA comprising a nucleotide sequence (AGG) of positions 1 to 3 of SEQ ID NO: 11 or 13, 5' UTR derived from human α globin gene, a nucleotide sequence encoding a protein consisting of the amino acid sequence as set forth by SEQ ID NO: 8, 3' UTR derived from human α globin gene, and a polyA sequence in order from the 5' terminal side. In one embodiment, the mRNA encoding the NeuroD1 protein is mRNA comprising a nucleotide sequence (AGG) of positions 1 to 3 of SEQ ID NO: 11 or 13, 5' UTR derived from human α globin gene, a nucleotide sequence having 70% or higher (e.g., 80% or higher, 90% or higher, or 95% or higher) sequence identity to the nucleotide sequence of positions 44 to 1114 or positions 44 to 1120 of SEQ ID NO: 11 or 13, and encoding the protein consisting of the amino acid sequence as set forth by SEQ ID NO: 8, 3' UTR derived from human α globin gene, and a polyA sequence in order from the 5' terminal side. In one embodiment, the mRNA encoding the NeuroD1 protein is mRNA comprising a nucleotide sequence (AGG) of positions 1 to 3 of SEQ ID NO: 11 or 13, 5' UTR derived from human α globin gene, the nucleotide sequence of positions 44 to 1114 or positions 44 to 1120 of SEQ ID NO: 11 or 13, 3' UTR derived from human α globin gene, and a polyA sequence in order from the 5' terminal side. In one embodiment, the mRNA encoding the NeuroD1 protein is mRNA comprising a nucleotide sequence (AGG) of positions 1 to 3 of SEQ ID NO: 11 or 13, 5' UTR consisting of a nucleotide sequence of positions 4 to 43 of SEQ ID NO: 11 or 13, the nucleotide sequence of positions 44 to 1114 or positions 44 to 1120 of SEQ ID NO: 11 or 13, 3' UTR consisting of a nucleotide sequence of positions 1121 to 1231 of SEQ ID NO: 11 or 13, and a polyA sequence in order from the 5' terminal side. In one embodiment, the mRNA encoding the NeuroD1 protein is mRNA comprising a polynucleotide consisting of a nucleotide sequence of positions 1 to 1231 of SEQ ID NO: 11 or 13 and a polyA sequence. In one embodiment, the mRNA encoding the NeuroD1 protein is mRNA comprising a nucleotide sequence (AGG) from positions 1 to 3 of SEQ ID NO: 11 or 13, a nucleotide sequence having 70% or higher (e.g., 80% or higher, 90% or higher, or 95% or higher) sequence identity to a nucleotide sequence of positions 4 to 1231 of SEQ ID NO: 11 or 13, and encoding the protein consisting of the amino acid sequence as set forth by SEQ ID NO: 8, and a polyA sequence in order from the 5' terminal side.

In one embodiment, the mRNA encoding the NeuroD1 protein is mRNA comprising the nucleotide sequence as set forth by SEQ ID NO: 11 or 13 or consisting of this nucleotide sequence.

In the present invention, the mRNA encoding the NeuroD1 protein may be modified mRNA. The modified mRNA can be produced, for example, by using a modified form of NTP in mRNA production. The modified mRNA may have a modification (e.g., a chemical modification) on a nucleobase of a nucleotide, on a sugar moiety, and/or on phosphate. The modified mRNA contains, for example, a modified nucleoside. The "modified nucleoside" is a nucleoside that has been modified. The modified nucleoside is a nucleoside that has undergone a modification, for example, methylation, atomic exchange, double bond saturation, deamination, or the replacement of an oxygen atom or the like with a sulfur atom. The modified nucleoside may be, but not limited to, pseudouridine, N1-methylpseudouridine (also referred to as 1-methylpseudouridine), 5-methylcytidine, 7-methylguanosine, N6-methyladenosine, 1-methyladenosine, dihydrouridine, inosine, 4-thiouridine, or the like. A polynucleotide in which "some or all" nucleosides of particular type are replaced by a modified nucleoside encompasses a polynucleotide in which some nucleosides of the type in the original polynucleotide sequence (specifically, mRNA encoding the NeuroD1 protein) are replaced by a modified nucleoside and the other nucleosides are not replaced by a modified nucleoside, and a polynucleotide in which all nucleosides of the type contained in the original polynucleotide sequence are replaced by a modified nucleoside. The term "some" regarding the ratio of replaced nucleosides of particular type in the sequence of a polynucleotide containing a modified nucleoside by the modified nucleoside includes one or more nucleosides and may be, for example, but not limited to, 60 to 99%, 70 to 99%, 80 to 99%, 90 to 99%, 95 to 99%, 60 to 99.9%, 70 to 99.9%, 80 to 99.9%, 90 to 99.9%, or 95 to 99.9% in terms of a substitution ratio to the total number of nucleosides of the particular type. In one embodiment, the mRNA encoding the NeuroD1 protein according to the present invention may contain a modified nucleoside. In one embodiment, the mRNA encoding the NeuroD1 protein according to the present invention may contain N1-methylpseudouridine. In one embodiment, in the mRNA encoding the NeuroD1 protein according to the present invention, some or all uridines may be replaced by N1-methylpseudouridine. In one embodiment, in the mRNA encoding the NeuroD1 protein according to the present invention, all uridines may be replaced by N1-methylpseudouridine.

In one embodiment, the mRNA encoding the NeuroD1 protein is mRNA comprising a nucleotide sequence encoding a protein consisting of the amino acid sequence as set forth by SEQ ID NO: 8, the mRNA containing a modified nucleoside, preferably N1-methylpseudouridine. In one embodiment, the mRNA encoding the NeuroD1 protein is mRNA comprising a nucleotide sequence having 70% or higher (e.g., 80% or higher, 90% or higher, or 95% or higher) sequence identity to a nucleotide sequence of positions 44 to 1114 or positions 44 to 1120 of SEQ ID NO: 11 or 13, and encoding the protein consisting of the amino acid sequence as set forth by SEQ ID NO: 8, the mRNA containing a modified nucleoside, preferably N1-methylpseudouridine. In one embodiment, the mRNA encoding the NeuroD1 protein is mRNA comprising the nucleotide sequence of positions 44 to 1114 or positions 44 to 1120 of SEQ ID NO: 11, the mRNA containing a modified nucleoside, preferably N1-methylpseudouridine.

In one embodiment, the mRNA encoding the NeuroD1 protein is mRNA comprising a nucleotide sequence encoding a protein consisting of the amino acid sequence as set forth by SEQ ID NO: 8, wherein some or all, preferably all uridines are replaced by N1-methylpseudouridine. In one embodiment, the mRNA encoding the NeuroD1 protein is mRNA comprising a nucleotide sequence having 70% or higher (e.g., 80% or higher, 90% or higher, or 95% or higher) sequence identity to the nucleotide sequence of positions 44 to 1114 or positions 44 to 1120 of SEQ ID NO: 11 or 13, and encoding the protein consisting of the amino acid sequence as set forth by SEQ ID NO: 8, wherein some or all, preferably all uridines are replaced by N1-methylpseudouridine. In one embodiment, the mRNA encoding the NeuroD1 protein is mRNA comprising the nucleotide sequence of positions 44 to 1114 or positions 44 to 1120 of SEQ ID NO: 11, wherein some or all, preferably all uridines are replaced by N1-methylpseudouridine.

In one embodiment, the mRNA encoding the NeuroD1 protein is mRNA comprising a nucleotide sequence encoding a protein consisting of the amino acid sequence as set forth by SEQ ID NO: 8 and further comprsing 5' UTR and/or 3' UTR, wherein the mRNA contains a modified nucleoside, preferably, N1-methylpseudouridine. In one embodiment, the mRNA encoding the NeuroD1 protein is mRNA comprising a nucleotide sequence having 70% or higher (e.g., 80% or higher, 90% or higher, or 95% or higher) sequence identity to the nucleotide sequence of positions 44 to 1114 or positions 44 to 1120 of SEQ ID NO: 11 or 13, and encoding the protein consisting of the amino acid sequence as set forth by SEQ ID NO: 8, and further comprsing 5' UTR and/or 3' UTR, wherein the mRNA contains a modified nucleoside, preferably, N1-methylpseudouridine. In one embodiment, the mRNA encoding the NeuroD1 protein is mRNA comprising the nucleotide sequence of positions 44 to 1114 or positions 44 to 1120 of SEQ ID NO: 11 and further comprsing 5' UTR and/or 3' UTR, wherein the mRNA contains a modified nucleoside, preferably, N1-methylpseudouridine.

In one embodiment, the mRNA encoding the NeuroD1 protein is mRNA comprising a nucleotide sequence encoding a protein consisting of the amino acid sequence as set forth by SEQ ID NO: 8 and further comprsing 5' UTR and/or 3' UTR, wherein some or all, preferably all uridines are replaced by N1-methylpseudouridine. In one embodiment, the mRNA encoding the NeuroD1 protein is mRNA comprising a nucleotide sequence having 70% or higher (e.g., 80% or higher, 90% or higher, or 95% or higher) sequence identity to the nucleotide sequence of positions 44 to 1114 or positions 44 to 1120 of SEQ ID NO: 11 or 13, and encoding the protein consisting of the amino acid sequence as set forth by SEQ ID NO: 8, and further comprsing 5' UTR and/or 3' UTR, wherein some or all, preferably all uridines are replaced by N1-methylpseudouridine. In one embodiment, the mRNA encoding the NeuroD1 protein is mRNA comprising the nucleotide sequence of positions 44 to 1114 or positions 44 to 1120 of SEQ ID NO: 11 and further comprsing 5' UTR and/or 3' UTR, wherein some or all, preferably all uridines are replaced by N1-methylpseudouridine.

In one embodiment, the mRNA encoding the NeuroD1 protein is mRNA comprising a nucleotide sequence encoding a protein consisting of the amino acid sequence as set forth by SEQ ID NO: 8 and further comprsing 5' UTR and/or 3' UTR, and a polyA sequence, wherein the mRNA contains a modified nucleoside, preferably, N1-methylpseudouridine. In one embodiment, the mRNA encoding the NeuroD1 protein is mRNA comprising a nucleotide sequence having 70% or higher (e.g., 80% or higher, 90% or higher, or 95% or higher) sequence identity to the nucleotide sequence of positions 44 to 1114 or positions 44 to 1120 of SEQ ID NO: 11 or 13, and encoding the protein consisting of the amino acid sequence as set forth by SEQ ID NO: 8, and further comprsing 5' UTR and/or 3' UTR, and a polyA sequence, wherein the mRNA contains a modified nucleoside, preferably, N1-methylpseudouridine. In one embodiment, the mRNA encoding the NeuroD1 protein is mRNA comprising the nucleotide sequence of positions 44 to 1114 or positions 44 to 1120 of SEQ ID NO: 11 and further comprsing 5' UTR and/or 3' UTR, and a polyA sequence, wherein the mRNA contains a modified nucleoside, preferably, N1-methylpseudouridine.

In one embodiment, the mRNA encoding the NeuroD1 protein is mRNA comprising a nucleotide sequence encoding a protein consisting of the amino acid sequence as set forth by SEQ ID NO: 8 and further comprsing 5' UTR and/or 3' UTR, and a polyA sequence, wherein some or all, preferably all uridines are replaced by N1-methylpseudouridine. In one embodiment, the mRNA encoding the NeuroD1 protein is mRNA comprising a nucleotide sequence having 70% or higher (e.g., 80% or higher, 90% or higher, or 95% or higher) sequence identity to the nucleotide sequence of positions 44 to 1114 or positions 44 to 1120 of SEQ ID NO: 11 or 13, and encoding the protein consisting of the amino acid sequence as set forth by SEQ ID NO: 8, and further comprsing 5' UTR and/or 3' UTR, and a polyA sequence, wherein some or all, preferably all uridines are replaced by N1-methylpseudouridine. In one embodiment, the mRNA encoding the NeuroD1 protein is mRNA comprising the nucleotide sequence of positions 44 to 1114 or positions 44 to 1120 of SEQ ID NO: 11 and further comprsing 5' UTR and/or 3' UTR, and a polyA sequence, wherein some or all, preferably all uridines are replaced by N1-methylpseudouridine.

In one embodiment, the mRNA encoding the NeuroD1 protein is mRNA comprising a nucleotide sequence encoding a protein consisting of the amino acid sequence as set forth by SEQ ID NO: 8 and further comprsing 5' UTR and/or 3' UTR derived from human α globin gene, wherein the mRNA contains a modified nucleoside, preferably, N1-methylpseudouridine. In one embodiment, the mRNA encoding the NeuroD1 protein is mRNA comprising a nucleotide sequence having 70% or higher (e.g., 80% or higher, 90% or higher, or 95% or higher) sequence identity to the nucleotide sequence of positions 44 to 1114 or positions 44 to 1120 of SEQ ID NO: 11 or 13, and encoding the protein consisting of the amino acid sequence as set forth by SEQ ID NO: 8, and further comprsing 5' UTR and/or 3' UTR derived from human α globin gene, wherein the mRNA contains a modified nucleoside, preferably, N1-methylpseudouridine. In one embodiment, the mRNA encoding the NeuroD1 protein is mRNA comprising the nucleotide sequence of positions 44 to 1114 or positions 44 to 1120 of SEQ ID NO: 11 and further comprsing 5' UTR and/or 3' UTR derived from human α globin gene, wherein the mRNA contains a modified nucleoside, preferably, N1-methylpseudouridine.

In one embodiment, the mRNA encoding the NeuroD1 protein is mRNA comprising a nucleotide sequence encoding a protein consisting of the amino acid sequence as set forth by SEQ ID NO: 8 and further comprsing 5' UTR and/or 3' UTR derived from human α globin gene, wherein some or all, preferably all uridines are replaced by N1-methylpseudouridine. In one embodiment, the mRNA encoding the NeuroD1 protein is mRNA comprising a nucleotide sequence having 70% or higher (e.g., 80% or higher, 90% or higher, or 95% or higher) sequence identity to the nucleotide sequence of positions 44 to 1114 or positions 44 to 1120 of SEQ ID NO: 11 or 13, and encoding the protein consisting of the amino acid sequence as set forth by SEQ ID NO: 8, and further comprsing 5' UTR and/or 3' UTR derived from human α globin gene, wherein some or all, preferably all uridines are replaced by N1-methylpseudouridine. In one embodiment, the mRNA encoding the NeuroD1 protein is mRNA comprising the nucleotide sequence of positions 44 to 1114 or positions 44 to 1120 of SEQ ID NO: 11 and further comprsing 5' UTR and/or 3' UTR derived from human α globin gene, wherein some or all, preferably all uridines are replaced by N1-methylpseudouridine.

In one embodiment, the mRNA encoding the NeuroD1 protein is mRNA comprising a nucleotide sequence encoding a protein consisting of the amino acid sequence as set forth by SEQ ID NO: 8 and further comprsing 5' UTR and/or 3' UTR derived from human α globin gene, and a polyA sequence, wherein the mRNA contains a modified nucleoside, preferably, N1-methylpseudouridine. In one embodiment, the mRNA encoding the NeuroD1 protein is mRNA comprising a nucleotide sequence having 70% or higher (e.g., 80% or higher, 90% or higher, or 95% or higher) sequence identity to the nucleotide sequence of positions 44 to 1114 or positions 44 to 1120 of SEQ ID NO: 11 or 13, and encoding the protein consisting of the amino acid sequence as set forth by SEQ ID NO: 8, and further comprsing 5' UTR and/or 3' UTR derived from human α globin gene, and a polyA sequence, wherein the mRNA contains a modified nucleoside, preferably, N1-methylpseudouridine. In one embodiment, the mRNA encoding the NeuroD1 protein is mRNA comprising the nucleotide sequence of positions 44 to 1114 or positions 44 to 1120 of SEQ ID NO: 11 and further comprsing 5' UTR and/or 3' UTR derived from human α globin gene, and a polyA sequence, wherein the mRNA contains a modified nucleoside, preferably, N1-methylpseudouridine.

In one embodiment, the mRNA encoding the NeuroD1 protein is mRNA comprising a nucleotide sequence encoding a protein consisting of the amino acid sequence as set forth by SEQ ID NO: 8 and further comprsing 5' UTR and/or 3' UTR derived from human α globin gene, and a polyA sequence, wherein some or all, preferably all uridines are replaced by N1-methylpseudouridine. In one embodiment, the mRNA encoding the NeuroD1 protein is mRNA comprising a nucleotide sequence having 70% or higher (e.g., 80% or higher, 90% or higher, or 95% or higher) sequence identity to the nucleotide sequence of positions 44 to 1114 or positions 44 to 1120 of SEQ ID NO: 11 or 13, and encoding the protein consisting of the amino acid sequence as set forth by SEQ ID NO: 8, and further comprsing 5' UTR and/or 3' UTR derived from human α globin gene, and a polyA sequence, wherein some or all, preferably all uridines are replaced by N1-methylpseudouridine. In one embodiment, the mRNA encoding the NeuroD1 protein is mRNA comprising the nucleotide sequence of positions 44 to 1114 or positions 44 to 1120 of SEQ ID NO: 11 and further comprsing 5' UTR and/or 3' UTR derived from human α globin gene, and a polyA sequence, wherein some or all, preferably all uridines are replaced by N1-methylpseudouridine.

In one embodiment, the mRNA encoding the NeuroD1 protein is mRNA comprising the nucleotide sequence as set forth by SEQ ID NO: 11, wherein the mRNA contains a modified nucleoside. In one embodiment, the mRNA encoding the NeuroD1 protein is mRNA comprising the nucleotide sequence as set forth by SEQ ID NO: 11, wherein the mRNA contains N1-methylpseudouridine. In one embodiment, the mRNA encoding the NeuroD1 protein is mRNA comprising the nucleotide sequence as set forth by SEQ ID NO: 11, wherein some or all uridines are replaced by N1-methylpseudouridine. In one embodiment, the mRNA encoding the NeuroD1 protein is mRNA comprising the nucleotide sequence as set forth by SEQ ID NO: 11, wherein all uridines are replaced by N1-methylpseudouridine. The mRNA encoding the NeuroD1 protein, wherein all uridines are replaced by N1-methylpseudouridine may be, for example, a polynucleotide comprising the nucleotide sequence as set forth by SEQ ID NO: 13.

In one embodiment, the mRNA encoding the NeuroD1 protein is mRNA comprising a nucleotide sequence having 70% or higher (e.g., 80% or higher, 90% or higher, or 95% or higher) sequence identity to a nucleotide sequence of positions 4 to 1231 of SEQ ID NO: 11 or 13, and encoding a protein consisting of the amino acid sequence as set forth by SEQ ID NO: 8, and a polyA sequence, the mRNA containing a modified nucleoside. In one embodiment, the mRNA encoding the NeuroD1 protein is mRNA comprising a nucleotide sequence having 70% or higher (e.g., 80% or higher, 90% or higher, or 95% or higher) sequence identity to a nucleotide sequence of positions 4 to 1231 of SEQ ID NO: 11 or 13, and encoding a protein consisting of the amino acid sequence as set forth by SEQ ID NO: 8, and a polyA sequence, the mRNA containing N1-methylpseudouridine. In one embodiment, the mRNA encoding the NeuroD1 protein is mRNA comprising a nucleotide sequence having 70% or higher (e.g., 80% or higher, 90% or higher, or 95% or higher) sequence identity to a nucleotide sequence of positions 4 to 1231 of SEQ ID NO: 11, and encoding a protein consisting of the amino acid sequence as set forth by SEQ ID NO: 8, and a polyA sequence, wherein some or all uridines are replaced by N1-methylpseudouridine. In one embodiment, the mRNA encoding the NeuroD1 protein is mRNA comprising a nucleotide sequence having 70% or higher (e.g., 80% or higher, 90% or higher, or 95% or higher) sequence identity to a nucleotide sequence of positions 4 to 1231 of SEQ ID NO: 11, and encoding a protein consisting of the amino acid sequence as set forth by SEQ ID NO: 8, and a polyA sequence, wherein all uridines are replaced by N1-methylpseudouridine.

In one embodiment, the mRNA encoding the NeuroD1 protein has a 5' cap structure (e.g., Cap-0, Cap-1, or Cap-2). In one embodiment, the 5' cap structure contained in the mRNA encoding the NeuroD1 protein is Cap-1.

In one embodiment, the mRNA encoding the NeuroD1 protein is mRNA comprising a nucleotide sequence encoding a protein consisting of the amino acid sequence as set forth by SEQ ID NO: 8 (e.g., the nucleotide sequence of positions 44 to 1114 or positions 44 to 1120 of SEQ ID NO: 11 or 13, or a nucleotide sequence having 70% or higher (e.g., 80% or higher, 90% or higher, or 95% or higher) sequence identity to the nucleotide sequence of positions 44 to 1114 or positions 44 to 1120 of SEQ ID NO: 11 or 13), wherein the mRNA has a 5' cap structure. In one embodiment, the mRNA encoding the NeuroD1 protein is mRNA comprising a nucleotide sequence encoding a protein consisting of the amino acid sequence as set forth by SEQ ID NO: 8 (e.g., the nucleotide sequence of positions 44 to 1114 or positions 44 to 1120 of SEQ ID NO: 11 or 13, or a nucleotide sequence having 70% or higher (e.g., 80% or higher, 90% or higher, or 95% or higher) sequence identity to the nucleotide sequence of positions 44 to 1114 or positions 44 to 1120 of SEQ ID NO: 11 or 13) and further comprsing 5' UTR and/or 3' UTR, wherein the mRNA has a 5' cap structure. In one embodiment, the mRNA encoding the NeuroD1 protein is mRNA comprising a nucleotide sequence encoding a protein consisting of the amino acid sequence as set forth by SEQ ID NO: 8 (e.g., the nucleotide sequence of positions 44 to 1114 or positions 44 to 1120 of SEQ ID NO: 11 or 13, or a nucleotide sequence having 70% or higher (e.g., 80% or higher, 90% or higher, or 95% or higher) sequence identity to the nucleotide sequence of positions 44 to 1114 or positions 44 to 1120 of SEQ ID NO: 11 or 13) and further comprsing 5' UTR and/or 3' UTR, and a polyA sequence, wherein the mRNA has a 5' cap structure. In one embodiment, the mRNA encoding the NeuroD1 protein is mRNA comprising a nucleotide sequence encoding a protein consisting of the amino acid sequence as set forth by SEQ ID NO: 8 (e.g., the nucleotide sequence of positions 44 to 1114 or positions 44 to 1120 of SEQ ID NO: 11 or 13, or a nucleotide sequence having 70% or higher (e.g., 80% or higher, 90% or higher, or 95% or higher) sequence identity to the nucleotide sequence of positions 44 to 1114 or positions 44 to 1120 of SEQ ID NO: 11 or 13) and further comprsing 5' UTR and/or 3' UTR derived from human α globin gene, wherein the mRNA has a 5' cap structure. In one embodiment, the mRNA encoding the NeuroD1 protein is mRNA comprising a nucleotide sequence encoding a protein consisting of the amino acid sequence as set forth by SEQ ID NO: 8 (e.g., the nucleotide sequence of positions 44 to 1114 or positions 44 to 1120 of SEQ ID NO: 11 or 13, or a nucleotide sequence having 70% or higher (e.g., 80% or higher, 90% or higher, or 95% or higher) sequence identity to the nucleotide sequence of positions 44 to 1114 or positions 44 to 1120 of SEQ ID NO: 11 or 13) and further comprsing 5' UTR and/or 3' UTR derived from human α globin gene, and a polyA sequence, wherein the mRNA has a 5' cap structure.

In one embodiment, the mRNA encoding the NeuroD1 protein is mRNA comprising a nucleotide sequence encoding a protein consisting of the amino acid sequence as set forth by SEQ ID NO: 8 (e.g., the nucleotide sequence of positions 44 to 1114 or positions 44 to 1120 of SEQ ID NO: 11 or 13, or a nucleotide sequence having 70% or higher (e.g., 80% or higher, 90% or higher, or 95% or higher) sequence identity to the nucleotide sequence of positions 44 to 1114 or positions 44 to 1120 of SEQ ID NO: 11 or 13), wherein the mRNA contains a modified nucleoside, preferably, N1-methylpseudouridine, and has a 5' cap structure. In one embodiment, the mRNA encoding the NeuroD1 protein is mRNA comprising a nucleotide sequence encoding a protein consisting of the amino acid sequence as set forth by SEQ ID NO: 8 (e.g., the nucleotide sequence of positions 44 to 1114 or positions 44 to 1120 of SEQ ID NO: 11 or 13, or a nucleotide sequence having 70% or higher (e.g., 80% or higher, 90% or higher, or 95% or higher) sequence identity to the nucleotide sequence of positions 44 to 1114 or positions 44 to 1120 of SEQ ID NO: 11 or 13) and further comprsing 5' UTR and/or 3' UTR, wherein the mRNA contains a modified nucleoside, preferably, N1-methylpseudouridine, and has a 5' cap structure. In one embodiment, the mRNA encoding the NeuroD1 protein is mRNA comprising a nucleotide sequence encoding a protein consisting of the amino acid sequence as set forth by SEQ ID NO: 8 (e.g., the nucleotide sequence of positions 44 to 1114 or positions 44 to 1120 of SEQ ID NO: 11 or 13, or a nucleotide sequence having 70% or higher (e.g., 80% or higher, 90% or higher, or 95% or higher) sequence identity to the nucleotide sequence of positions 44 to 1114 or positions 44 to 1120 of SEQ ID NO: 11 or 13) and further comprsing 5' UTR and/or 3' UTR, and a polyA sequence, wherein the mRNA contains a modified nucleoside, preferably, N1-methylpseudouridine, and has a 5' cap structure. In one embodiment, the mRNA encoding the NeuroD1 protein is mRNA comprising a nucleotide sequence encoding a protein consisting of the amino acid sequence as set forth by SEQ ID NO: 8 (e.g., the nucleotide sequence of positions 44 to 1114 or positions 44 to 1120 of SEQ ID NO: 11 or 13, or a nucleotide sequence having 70% or higher (e.g., 80% or higher, 90% or higher, or 95% or higher) sequence identity to the nucleotide sequence of positions 44 to 1114 or positions 44 to 1120 of SEQ ID NO: 11 or 13) and further comprsing 5' UTR and/or 3' UTR derived from human α globin gene, wherein the mRNA contains a modified nucleoside, preferably, N1-methylpseudouridine, and has a 5' cap structure. In one embodiment, the mRNA encoding the NeuroD1 protein is mRNA comprising a nucleotide sequence encoding a protein consisting of the amino acid sequence as set forth by SEQ ID NO: 8 (e.g., the nucleotide sequence of positions 44 to 1114 or positions 44 to 1120 of SEQ ID NO: 11 or 13, or a nucleotide sequence having 70% or higher (e.g., 80% or higher, 90% or higher, or 95% or higher) sequence identity to the nucleotide sequence of positions 44 to 1114 or positions 44 to 1120 of SEQ ID NO: 11 or 13) and further comprsing 5' UTR and/or 3' UTR derived from human α globin gene, and a polyA sequence, wherein the mRNA contains a modified nucleoside, preferably, N1-methylpseudouridine, and has a 5' cap structure.

In one embodiment, the mRNA encoding the NeuroD1 protein is mRNA comprising a polynucleotide consisting of a nucleotide sequence of positions 1 to 1231 of SEQ ID NO: 11 or 13 and a polyA sequence, wherein the mRNA has Cap-1 as a 5' cap structure. In one embodiment, the mRNA encoding the NeuroD1 protein is mRNA comprising a polynucleotide consisting of a nucleotide sequence of positions 4 to 1231 of SEQ ID NO: 11 or 13 and a polyA sequence, wherein the mRNA has Cap-1 as a 5' cap structure. In one embodiment, the mRNA encoding the NeuroD1 protein is mRNA comprising a nucleotide sequence having 70% or higher (e.g., 80% or higher, 90% or higher, or 95% or higher) sequence identity to the nucleotide sequence of positions 4 to 1231 of SEQ ID NO: 11 or 13, and encoding a protein consisting of the amino acid sequence as set forth by SEQ ID NO: 8, and a polyA sequence, wherein the mRNA has Cap-1 as a 5' cap structure.

In one embodiment, the mRNA encoding the NeuroD1 protein is mRNA comprising a polynucleotide consisting of a nucleotide sequence of positions 1 to 1231 of SEQ ID NO: 11 or 13 and a polyA sequence, wherein the mRNA contains a modified nucleoside, preferably, N1-methylpseudouridine, and has Cap-1 as a 5' cap structure. In one embodiment, the mRNA encoding the NeuroD1 protein is mRNA comprising a polynucleotide consisting of a nucleotide sequence of positions 4 to 1231 of SEQ ID NO: 11 or 13 and a polyA sequence, wherein the mRNA contains a modified nucleoside, preferably, N1-methylpseudouridine, and has Cap-1 as a 5' cap structure. In one embodiment, the mRNA encoding the NeuroD1 protein is mRNA comprising a nucleotide sequence having 70% or higher (e.g., 80% or higher, 90% or higher, or 95% or higher) sequence identity to the nucleotide sequence of positions 4 to 1231 of SEQ ID NO: 11 or 13, and encoding a protein consisting of the amino acid sequence as set forth by SEQ ID NO: 8, and a polyA sequence, wherein the mRNA contains a modified nucleoside, preferably, N1-methylpseudouridine, and has Cap-1 as a 5' cap structure.

In one embodiment, the mRNA encoding the NeuroD1 protein is mRNA comprising a polynucleotide consisting of a nucleotide sequence of positions 1 to 1231 of SEQ ID NO: 11 or 13 and a polyA sequence, wherein some or all, preferably all uridines are replaced by N1-methylpseudouridine, and the mRNA has Cap-1 as a 5' cap structure. In one embodiment, the mRNA encoding the NeuroD1 protein is mRNA comprising a polynucleotide consisting of a nucleotide sequence of positions 4 to 1231 of SEQ ID NO: 11 or 13 and a polyA sequence, wherein some or all, preferably all uridines are replaced by N1-methylpseudouridine, and the mRNA has Cap-1 as a 5' cap structure. In one embodiment, the mRNA encoding the NeuroD1 protein is mRNA comprising a nucleotide sequence having 70% or higher (e.g., 80% or higher, 90% or higher, or 95% or higher) sequence identity to the nucleotide sequence of positions 4 to 1231 of SEQ ID NO: 11 or 13, and encoding a protein consisting of the amino acid sequence as set forth by SEQ ID NO: 8, and a polyA sequence, wherein some or all, preferably all uridines are replaced by N1-methylpseudouridine, and the mRNA has Cap-1 as a 5' cap structure.

In one embodiment, the mRNA encoding the NeuroD1 protein is mRNA comprising a polynucleotide consisting of a nucleotide sequence of positions 1 to 1231 of SEQ ID NO: 11 and a polyA sequence, wherein:
(i) 7-methylguanosine is bound with the 5' end of the nucleotide sequence of positions 1 to 1231 of SEQ ID NO: 11 via a triphosphate bond (5'-5' bond),
(ii) adenosine at position 1 of SEQ ID NO: 11 is 2'-O-methyladenosine, and
(iii) some or all uridines are replaced by N1-methylpseudouridine.

In one embodiment, the mRNA encoding the NeuroD1 protein is mRNA comprising a polynucleotide consisting of a nucleotide sequence of positions 1 to 1231 of SEQ ID NO: 13 and a polyA sequence, wherein:
(i) 7-methylguanosine is bound with the 5' end of the nucleotide sequence of positions 1 to 1231 of SEQ ID NO: 13 via a triphosphate bond (5'-5' bond), and
(ii) adenosine at position 1 of SEQ ID NO: 13 is 2'-O-methyladenosine.

In one embodiment, the mRNA encoding the NeuroD1 protein is mRNA comprising the nucleotide sequence as set forth by SEQ ID NO: 11, wherein some or all uridines are replaced by N1-methylpseudouridine, and the mRNA has Cap-1 as a 5' cap structure. In one embodiment, the mRNA encoding the NeuroD1 protein is mRNA comprising the nucleotide sequence as set forth by SEQ ID NO: 11, wherein all uridines are replaced by N1-methylpseudouridine, and the mRNA has Cap-1 as a 5' cap structure. In one embodiment, the mRNA encoding the NeuroD1 protein is mRNA comprising the nucleotide sequence as set forth by SEQ ID NO: 13, the mRNA having Cap-1 as a 5' cap structure.

In one embodiment, the mRNA encoding the NeuroD1 protein is mRNA comprising the nucleotide sequence as set forth by SEQ ID NO: 11, wherein:
(i) 7-methylguanosine is bound with the 5' end of the nucleotide sequence as set forth by SEQ ID NO: 11 via a triphosphate bond (5'-5' bond),
(ii) adenosine at position 1 of SEQ ID NO: 11 is 2'-O-methyladenosine, and
(iii) some or all uridines are replaced by N1-methylpseudouridine.

In one embodiment, the mRNA encoding the NeuroD1 protein is mRNA comprising the nucleotide sequence as set forth by SEQ ID NO: 13, wherein:
(i) 7-methylguanosine is bound with the 5' end of the nucleotide sequence as set forth by SEQ ID NO: 13 via a triphosphate bond (5'-5' bond), and
(ii) adenosine at position 1 of SEQ ID NO: 13 is 2'-O-methyladenosine.

In one embodiment, the mRNA encoding the NeuroD1 protein may be mRNA having no 5' cap structure. In one embodiment, the mRNA encoding the NeuroD1 protein is mRNA consisting of the nucleotide sequence as set forth by SEQ ID NO: 11, wherein the mRNA contains a modified nucleoside. In one embodiment, the mRNA encoding the NeuroD1 protein is mRNA consisting of the nucleotide sequence as set forth by SEQ ID NO: 11, wherein the mRNA contains N1-methylpseudouridine. In one embodiment, the mRNA encoding the NeuroD1 protein is mRNA consisting of the nucleotide sequence as set forth by SEQ ID NO: 11, wherein some or all uridines are replaced by N1-methylpseudouridine. In one embodiment, the mRNA encoding the NeuroD1 protein is mRNA consisting of the nucleotide sequence as set forth by SEQ ID NO: 11, wherein all uridines are replaced by N1-methylpseudouridine. The mRNA encoding the NeuroD1 protein, wherein all uridines are replaced by N1-methylpseudouridine may be, for example, a polynucleotide consisting of the nucleotide sequence as set forth by SEQ ID NO: 13.

In one embodiment, in the pharmaceutical composition of the present invention comprising the mRNA encoding the NeuroD1 protein, the mRNA may be incorporated in DDS. In one embodiment, the pharmaceutical composition of the present invention may be a pharmaceutical composition comprising mRNA encoding the NeuroD1 protein, wherein the mRNA is incorporated in a "drug delivery system" (DDS).

The DDS that may be used in the present invention preferably controls the distribution of the mRNA encoding the NeuroD1 protein in the body, enhances an effect of the mRNA, and suppresses an adverse reaction. In one embodiment, the DDS may be a drug delivery carrier. Examples of the drug delivery carrier include nanoparticles such as lipid nanoparticles and polymer particles (e.g., a nanomicelle carrier consisting of a block copolymer of polyethylene glycol and a biocompatible cationic polymer linked to each other). In one embodiment, the pharmaceutical composition of the present invention comprises mRNA encoding the NeuroD1 protein, and the mRNA is encapsulated into a drug delivery carrier. In one embodiment, the pharmaceutical composition of the present invention comprises mRNA encoding the NeuroD1 protein, and the mRNA is encapsulated into nanoparticles.

The "lipid nanoparticle" (LNP) is a nanoparticle constituted by a lipid with a diameter within the range of 10 nm to 1000 nm (10 nm or larger and smaller than 1000 nm) and is used as DDS to incorporate therein a nucleic acid medicine or the like. For example, an RNA vaccine encapsulated into LNP (e.g., L.R. Baden et al., The New England Journal of Medicine, vol 384, No. 5, pp. 403-416, 2021; and Fernando et al., The New England Journal of Medicine, vol 383, No. 27, pp 2603-2615, 2020) is known as a pharmaceutical composition comprising mRNA encoding a protein, wherein the mRNA is encapsulated into lipid nanoparticles. The lipid nanoparticle comprises an arbitrary lipid such as a cationic lipid, a PEGylated lipid, sterol, or a neutral phospholipid. In one embodiment, the lipid nanoparticle comprises a cationic lipid. In one embodiment, the lipid nanoparticle comprises a neutral phospholipid, sterol, and a PEGylated lipid. In one embodiment, the lipid nanoparticle comprises a cationic lipid, a neutral phospholipid, sterol, and a PEGylated lipid. Examples of the PEGylated lipid include DSPE-mPEG, ALC-0159, DMG-mPEG, and DMG-PEG2000. Examples of the sterol include cholesterol. Examples of the neutral phospholipid include distearoylphosphatidylcholine

(DSPC), dipalmitoylphosphatidylcholine (DPPC), and 1,2-dioleoyl-3-sn-phosphatidylethanolamine (DOPE). Examples of the cationic lipid (or an ionizable lipid) include DLin-MC3-DMA for use in a lipid nanoparticle to enclose therein siRNA. In one embodiment, the lipid nanoparticle comprises DSPC, cholesterol and DMG-PEG2000. As one example, the lipid nanoparticle containing RNA can comprise: an outer lipid layer constituted by a PEGylated lipid (e.g., DSPE-mPEG, ALC-0159, DMG-mPEG, or DMG-PEG2000), sterol (e.g., cholesterol), a neutral phospholipid (e.g., distearoylphosphatidylcholine (DSPC), dipalmitoylphosphatidylcholine (DPPC), or 1,2-dioleoyl-3-sn-phosphatidylethanolamine (DOPE)), and a cationic lipid (or an ionizable lipid) (e.g., DLin-MC3-DMA for use in a lipid nanoparticle to enclose therein siRNA); and a core of a cationic lipid surrounded by the lipid layer, the core surrounding the RNA. Such a lipid nanoparticle containing RNA can typically be produced by rapidly mixing (e.g., microfluidic mixing) ethanol containing the lipid with a low-pH buffer containing the RNA, with a mixer, thereby promoting RNA encapsulation, and further adjusting pH so as to gradually elevate the pH to or near a neutral region (e.g., pH 7.4), followed by the removal of ethanol to form a lipid nanoparticle. The PEGylated lipid, the sterol, the neutral phospholipid, and the cationic lipid constituting the lipid nanoparticle can be produced by use of a method known in the art.

Other examples of the cationic lipid that can be used in the lipid nanoparticle include Compound 1 (Ex1) (that is, di(heptadecan-9-yl) 8,8'-{[1-(2-hydroxyethyl)piperidin-4-yl]azanediyl}di(octanoate)) (see Example 15) represented by the formula (I), or a salt thereof; or Compound 2 (Ex2) (that is, bis(2-nonylundecyl) 7,7'-[({[1-(N,N-dimethylglycyl)piperidin-4-yl]oxy}carbonyl)azanediyl]di(heptanoate)) (see Example 16) represented by the formula (II), or a salt thereof.

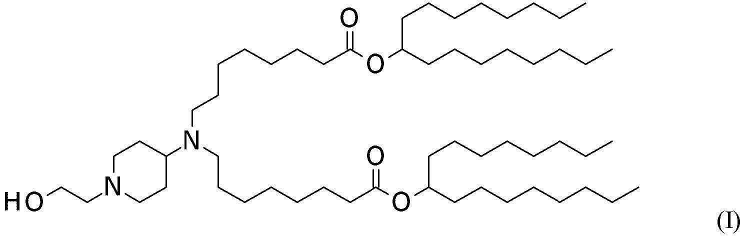

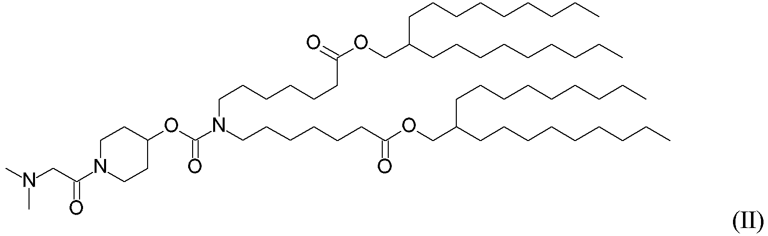

In the present invention, the salt of a compound is a pharmaceutically acceptable salt and may form an acid-addition salt depending on the type of a substituent. Specific examples thereof include acid-addition salts with inorganic acids such as hydrochloric acid, hydrobromic acid, hydroiodic acid, sulfuric acid, nitric acid, and phosphoric acid, or organic acids such as formic acid, acetic acid, propionic acid, oxalic acid, malonic acid, succinic acid, fumaric acid, maleic acid, lactic acid, malic acid, mandelic acid, tartaric acid, dibenzoyltartaric acid, ditoluoyltartaric acid, citric acid, methanesulfonic acid, ethanesulfonic acid, benzenesulfonic acid, p-toluenesulfonic acid, aspartic acid, and glutamic acid.

In one embodiment, the pharmaceutical composition of the present invention comprises mRNA encoding the NeuroD1 protein, and the mRNA is encapsulated into lipid nanoparticles.

In one embodiment, the pharmaceutical composition of the present invention comprises mRNA selected from the group consisting of the following (i) to (xx), and the mRNA is encapsulated into lipid nanoparticles:
(i) mRNA comprising a nucleotide sequence encoding a NeuroD1 protein consisting of the amino acid sequence as set forth by SEQ ID NO: 8,
(ii) mRNA comprising a nucleotide sequence having 70% or higher (e.g., 80% or higher, 90% or higher, or 95% or higher) sequence identity to the nucleotide sequence as set forth by SEQ ID NO: 7 or 10, and encoding a NeuroD1 protein consisting of the amino acid sequence as set forth by SEQ ID NO: 8,
(iii) mRNA comprising a nucleotide sequence having 70% or higher (e.g., 80% or higher, 90% or higher, or 95% or higher) sequence identity to the nucleotide sequence of positions 44 to 1114 or positions 44 to 1120 of SEQ ID NO: 11 or 13, and encoding a NeuroD1 protein consisting of the amino acid sequence as set forth by SEQ ID NO: 8,
(iv) mRNA comprising the nucleotide sequence as set forth by SEQ ID NO: 7 or 10,
(v) mRNA comprising the nucleotide sequence of positions 44 to 1114 or positions 44 to 1120 of SEQ ID NO: 11 or 13,
(vi) mRNA comprising the nucleotide sequence as set forth by SEQ ID NO: 11 or 13 or consisting of this nucleotide sequence,
(vii) mRNA comprising the nucleotide sequence as set forth by SEQ ID NO: 11 or consisting of this nucleotide sequence, wherein some or all uridine residues are replaced by N1-methyl pseudouridine,
(viii) mRNA comprising the nucleotide sequence as set forth by SEQ ID NO: 11, wherein some or all uridines are replaced by N1-methylpseudouridine, and the mRNA has Cap-1 as a 5' cap structure,
(ix) mRNA comprising the nucleotide sequence as set forth by SEQ ID NO: 11, wherein all uridines are replaced by N1-methylpseudouridine, and the mRNA has Cap-1 as a 5' cap structure (mRNA comprising the nucleotide sequence as set forth by SEQ ID NO: 13, the mRNA having Cap-1 as a 5' cap structure),
(x) mRNA of any of (i) to (v) above, wherein the mRNA further comprses 5' UTR and/or 3' UTR,
(xi) mRNA of any of (i) to (v) above, wherein the mRNA further comprses a polyA sequence,
(xii) mRNA of any of (i) to (v) above, wherein the mRNA further comprses 5' UTR and/or 3' UTR, and a polyA sequence,
(xiii) mRNA of (x) or (xii) above, wherein the 5' UTR and/or 3' UTR is 5' UTR and/or 3' UTR derived from human α globin gene,
(xiv) mRNA of any of (i) to (v) above, wherein the mRNA further comprses 5' UTR consisting of the nucleotide sequence of positions 4 to 43 of SEQ ID NO: 11 or 13, and/or 3' UTR consisting of the nucleotide sequence of positions 1121 to 1231 of SEQ ID NO: 11 or 13, and a polyA sequence,
(xv) mRNA comprising a nucleotide sequence having 70% or higher (e.g., 80% or higher, 90% or higher, or 95% or higher) sequence identity to the nucleotide sequence of positions 4 to 1231 of SEQ ID NO: 11 or 13, and encoding a protein consisting of the amino acid sequence as set forth by SEQ ID NO: 8, and a polyA sequence,
(xvi) mRNA comprising a polynucleotide consisting of a nucleotide sequence of positions 1 to 1231 of SEQ ID NO: 11 or 13, and a polyA sequence,
(xvii) mRNA of any of (xi) to (xvi) above, wherein the polyA sequence is 50 to 200 nucleotides in length,
(xix) mRNA of any of (i) to (v) and (x) to (xviii) above, wherein the mRNA contains a modified nucleoside, preferably, N1-methylpseudouridine, and
(xx) mRNA of any of (i) to (v) and (x) to (xix) above, wherein some or all, preferably all uridines are replaced by N1-methylpseudouridine.

The lipid nanoparticle is not particularly limited as long as the lipid nanoparticle is capable of delivering mRNA to the cells of interest. For example, in the case of delivering mRNA to glial cells (e.g., astrocytes or microglia), a lipid nanoparticle capable of delivering the mRNA to glial cells (e.g., astrocytes) may be selected. Whether a lipid nanoparticle is capable of delivering mRNA to glial cells can be evaluated using, for example, CleanCap^{(R)} eGFP mRNA (TriLink BioTechnologies, Inc.). Specifically, the mRNA is encapsulated into the lipid nanoparticles to be evaluated, and the mRNA encapsulated into the lipid nanoparticle is transferred to glial cells. Subsequently, the expression intensity of eGFP expressed in the glial cells can be measured and compared with cells harboring PBS instead of the mRNA encapsulated into the lipid nanoparticle to study the presence or absence of change (increase or decrease) in the expression intensity of eGFP, thereby determining the capability of delivery. For example, when the expression intensity of eGFP is increased by the transfer of the mRNA encapsulated into the lipid nanoparticle, this lipid nanoparticle can be determined to be capable of delivering mRNA to glial cells. Such evaluation may be conducted, for example, by using the number of cells expressing eGFP as an index instead of the expression intensity of eGFP as an index.

In one embodiment, the mRNA contained in the pharmaceutical composition of the present invention may be encapsulated into lipid nanoparticles capable of delivery to glial cells, and delivered to glial cells. In one embodiment, the mRNA contained in the pharmaceutical composition of the present invention may be encapsulated into lipid nanoparticles capable of delivery to astrocytes, and delivered to astrocytes. In one embodiment, the mRNA contained in the pharmaceutical composition of the present invention may be encapsulated into lipid nanoparticles capable of delivery to microglia, and delivered to microglia.

Examples of the cationic lipid contained in the lipid nanoparticle to enclose the mRNA therein, for example, the cationic lipid contained in the lipid nanoparticle capable of delivering the mRNA to astrocytes, include Compound 1 (Ex1; Example 15) or a salt thereof, or Compound 2 (Ex2; Example 16) or a salt thereof. In one embodiment, the lipid nanoparticle capable of delivering the mRNA to astrocytes comprises a cationic lipid. In one embodiment, the lipid nanoparticle capable of delivering the mRNA to astrocytes comprises Compound 1 (Ex1) or a salt thereof, and/or Compound 2 (Ex2) or a salt thereof as a cationic lipid.

In one embodiment, in the pharmaceutical composition of the present invention comprising mRNA encoding the NeuroD1 protein, wherein the mRNA is encapsulated into lipid nanoparticles, the lipid nanoparticle comprises a cationic lipid. In one embodiment, in the pharmaceutical composition of the present invention comprising mRNA encoding the NeuroD1 protein, wherein the mRNA is encapsulated into lipid nanoparticles, the lipid nanoparticle may comprise Compound 1 (Ex1) or a salt thereof, and/or Compound 2 (Ex2) or a salt thereof. In one embodiment, in the pharmaceutical composition of the present invention comprising mRNA encoding the NeuroD1 protein, wherein the mRNA is encapsulated into lipid nanoparticles, the cationic lipid in the lipid nanoparticle may be Compound 1 (Ex1) or a salt thereof. In one embodiment, in the pharmaceutical composition of the present invention comprising mRNA encoding the NeuroD1 protein, wherein the mRNA is encapsulated into lipid nanoparticles, the cationic lipid in the lipid nanoparticle may be Compound 2 (Ex2) or a salt thereof.

In one embodiment, in the pharmaceutical composition of the present invention comprising mRNA encoding the NeuroD1 protein, wherein the mRNA is encapsulated into lipid nanoparticles, the lipid nanoparticle may comprise di(heptadecan-9-yl) 8,8'-{[1-(2-hydroxyethyl)piperidin-4-yl]azanediyl}di(octanoate) or a salt thereof, and/or bis(2-nonylundecyl) 7,7'-[({[1-(N,N-dimethylglycyl)piperidin-4-yl]oxy}carbonyl)azanediyl]di(heptanoate) or a salt thereof, as a cationic lipid.

In one embodiment, in the pharmaceutical composition of the present invention comprising mRNA encoding the NeuroD1 protein, wherein the mRNA is encapsulated into lipid nanoparticles, the lipid nanoparticle may comprise di(heptadecan-9-yl) 8,8'-{[1-(2-hydroxyethyl)piperidin-4-yl]azanediyl}di(octanoate) or a salt thereof as a cationic lipid.

In one embodiment, in the pharmaceutical composition of the present invention comprising mRNA encoding the NeuroD1 protein, wherein the mRNA is encapsulated into lipid nanoparticles, the lipid nanoparticle may comprise bis(2-nonylundecyl) 7,7'-[({[1-(N,N-dimethylglycyl)piperidin-4-yl]oxy}carbonyl)azanediyl]di(heptanoate) or a salt thereof, as a cationic lipid.

The present invention also includes a pharmaceutical composition comprising mRNA encoding the NeuroD1 protein, wherein the mRNA is encapsulated into lipid nanoparticles. The present invention also includes a pharmaceutical composition comprising mRNA encoding the NeuroD1 protein, wherein the mRNA is encapsulated into lipid nanoparticles comprising Compound 1 (Ex1) or a salt thereof, and/or Compound 2 (Ex2) or a salt thereof. The present invention also includes a pharmaceutical composition comprising mRNA encoding the NeuroD1 protein, wherein the mRNA is encapsulated into lipid nanoparticles comprising Compound 1 (Ex1) or a salt thereof. The present invention also includes a pharmaceutical composition comprising mRNA encoding the NeuroD1 protein, wherein the mRNA is encapsulated into lipid nanoparticles comprising Compound 2 (Ex2) or a salt thereof. The present invention also includes a pharmaceutical composition comprising mRNA encoding the NeuroD1 protein and Compound 1 (Ex1) or a salt thereof. The present invention also includes a pharmaceutical composition comprising mRNA encoding the NeuroD1 protein and Compound 2 (Ex2) or a salt thereof.

In one embodiment, the pharmaceutical composition of the present invention comprises mRNA encoding the NeuroD1 protein, and the mRNA may be encapsulated into lipid nanoparticles comprising Compound 1 (Ex1) or a salt thereof, the lipid nanoparticle further comprising DMG-PEG2000, cholesterol, and DSPC. In one embodiment, the pharmaceutical composition of the present invention comprises mRNA encoding the NeuroD1 protein, and the mRNA may be encapsulated into lipid nanoparticles comprising Compound 1 (Ex1) or a salt thereof, the lipid nanoparticle further comprising DMG-PEG2000, cholesterol, and DSPC, wherein the cationic lipid, the DSPC, the cholesterol and the DMG-PEG2000 are contained at a ratio of 40:12:46.5:1.5.

In one embodiment, the pharmaceutical composition of the present invention comprises mRNA encoding the NeuroD1 protein, and the mRNA may be encapsulated into lipid nanoparticles comprising Compound 2 (Ex2) or a salt thereof, the lipid nanoparticle further comprising DMG-PEG2000, cholesterol, and DSPC. In one embodiment, the pharmaceutical composition of the present invention comprises mRNA encoding the NeuroD1 protein, and the mRNA may be encapsulated into lipid nanoparticles comprising Compound 2 (Ex2) or a salt thereof, the lipid nanoparticle further comprising DMG-PEG2000, cholesterol, and DSPC, wherein the cationic lipid, the DSPC, the cholesterol and the DMG-PEG2000 are contained at a ratio of 50:10:38.5:1.5.

The pharmaceutical composition of the present invention may comprise a plurality of mRNAs encoding the NeuroD1 protein. The present invention also includes, for example, a pharmaceutical composition comprising mRNA in which some uridines are replaced by N1-methylpseudouridine, mRNA in which all uridines are replaced by N1-methylpseudouridine, mRNA having a 5' cap structure, and/or mRNA having no 5' cap structure.

The present invention also includes a pharmaceutical composition comprising two or more mRNAs encoding the NeuroD1 protein among the following (i) to (iv) as the pharmaceutical composition of the present invention containing the mRNA encoding the NeuroD1 protein:
(i) mRNA comprising the nucleotide sequence as set forth by SEQ ID NO: 11, wherein some uridines are replaced by N1-methylpseudouridine, and the mRNA has Cap-1 as a 5' cap structure,
(ii) mRNA comprising the nucleotide sequence as set forth by SEQ ID NO: 11, wherein all uridines are replaced by N1-methylpseudouridine, and the mRNA has Cap-1 as a 5' cap structure (mRNA comprising the nucleotide sequence as set forth by SEQ ID NO: 13, the mRNA having Cap-1 as a 5' cap structure),
(iii) mRNA consisting of the nucleotide sequence as set forth by SEQ ID NO: 11, wherein some uridines are replaced by N1-methylpseudouridine, and
(iv) mRNA consisting of the nucleotide sequence as set forth by SEQ ID NO: 11, wherein all uridines are replaced by N1-methylpseudouridine (mRNA consisting of the nucleotide sequence as set forth by SEQ ID NO: 13).

In one embodiment, the pharmaceutical composition of the present invention comprising the mRNA encoding the NeuroD1 protein is a pharmaceutical composition comprising two or more mRNAs encoding the NeuroD1 protein among the following (i) to (iv):
(i) mRNA comprising a polynucleotide consisting of a nucleotide sequence of positions 1 to 1231 of SEQ ID NO: 11, and a polyA sequence, wherein some uridines are replaced by N1-methylpseudouridine, and the mRNA has Cap-1 as a 5' cap structure,
(ii) mRNA comprising a polynucleotide consisting of a nucleotide sequence of positions 1 to 1231 of SEQ ID NO: 11, and a polyA sequence, wherein all uridines are replaced by N1-methylpseudouridine, and the mRNA has Cap-1 as a 5' cap structure,
(iii) mRNA consisting of a polynucleotide consisting of a nucleotide sequence of positions 1 to 1231 of SEQ ID NO: 11, and a polyA sequence, wherein some uridines are replaced by N1-methylpseudouridine, and
(iv) mRNA consisting of a polynucleotide consisting of a nucleotide sequence of positions 1 to 1231 of SEQ ID NO: 11, and a polyA sequence, wherein all uridines are replaced by N1-methylpseudouridine.

In one embodiment, the pharmaceutical composition of the present invention comprising the mRNA encoding the NeuroD1 protein is a pharmaceutical composition comprising two or more mRNAs encoding the NeuroD1 protein among the following (i) to (iv):
(i) mRNA comprising a polynucleotide consisting of a nucleotide sequence of positions 1 to 1231 of SEQ ID NO: 11, and a polyA sequence, wherein some uridines are replaced by N1-methylpseudouridine, and the mRNA has Cap-1 as a 5' cap structure,
(ii) mRNA comprising a polynucleotide consisting of a nucleotide sequence of positions 1 to 1231 of SEQ ID NO: 11, and a polyA sequence, wherein all uridines are replaced by N1-methylpseudouridine, and the mRNA has Cap-1 as a 5' cap structure,
(iii) mRNA consisting of a polynucleotide consisting of a nucleotide sequence of positions 1 to 1231 of SEQ ID NO: 11, and a polyA sequence, wherein some uridines are replaced by N1-methylpseudouridine, and
(iv) mRNA consisting of a polynucleotide consisting of a nucleotide sequence of positions 1 to 1231 of SEQ ID NO: 11, and a polyA sequence, wherein all uridines are replaced by N1-methyl pseudouridine,
and wherein the polyA sequence is 40 to 200 nucleotides in length (e.g., 50 to 200, 50 to 150, 50 to 100, 50 to 90, 60 to 150, 60 to 100, 60 to 90, 70 to 130, 70 to 120, 70 to 100, 70 to 90, 70 to 85, 70 to 80, 75 to 130, 75 to 120, 75 to 100, or 75 to 90; preferably, 74 to 84, 75 to 85, 75 to 83, or 76 to 82 nucleotides in length).

The pharmaceutical composition of the present invention may also include a pharmaceutical composition comprising a plurality of mRNAs encoding the NeuroD1 protein, the mRNAs differing in the length of a polyA sequence.

The pharmaceutical composition of the present invention may also include a pharmaceutical composition formulated without mRNA being incorporated in DDS (e.g., encapsulated into a drug delivery carrier).

The pharmaceutical composition of the present invention may contain a pharmaceutically acceptable additive, for example, an inert carrier (solid or liquid carrier), an excipient, a surfactant, a solubilizer, a suspending agent, a colorant, a corrigent, a preservative, a buffer, or a pH adjuster. The pharmaceutical composition of the present invention can be produced or formulated by a method usually used using a pharmaceutically acceptable additive, for example, a pharmaceutical excipient or a pharmaceutical carrier usually used. The "pharmaceutical composition" described in the present invention means a composition available as a medicament.

The pharmaceutical composition of the present invention can be used for the treatment of cerebral infarction. In one embodiment, the pharmaceutical composition of the present invention can be used for the treatment of penetrating branch infarction (e.g., lacunar infarction and/or BAD). In one embodiment, the pharmaceutical composition of the present invention can be used for the treatment of penetrating branch infarction i) having brain damage in the basal ganglia and thalamic region, ii) having brain damage in the basal ganglia and/or the thalamus, iii) having brain damage in the basal ganglia and thalamic region and the internal capsule, or iv) having brain damage in the basal ganglia, the thalamus, and the internal capsule. In one embodiment, the pharmaceutical composition of the present invention can be used for the treatment of cerebral infarction having brain damage in a penetrating branch territory. **In** one embodiment, the pharmaceutical composition of the present invention can be used for the treatment of cerebral infarction having brain damage in a penetrating branch territory, wherein the brain damage is brain damage including i) brain damage of the basal ganglia and thalamic region, ii) brain damage of the basal ganglia and/or the thalamus, iii) brain damage of the basal ganglia and thalamic region and the internal capsule, or iv) brain damage of the basal ganglia, the thalamus, and the internal capsule. In one embodiment, the pharmaceutical composition of the present invention can be used for the treatment of cerebral infarction having local brain damage.

In one embodiment, the pharmaceutical composition of the present invention can be used for the treatment of cerebral infarction at a subacute to chronic stage. In one embodiment, the pharmaceutical composition of the present invention can be used for the treatment of penetrating branch infarction at a subacute to chronic stage. In one embodiment, the pharmaceutical composition of the present invention can be used for the treatment of lacunar infarction and/or BAD at a subacute to chronic stage. In one embodiment, the pharmaceutical composition of the present invention can be used for the treatment of penetrating branch infarction at a subacute to chronic stage, the penetrating branch infarction i) having brain damage in the basal ganglia and thalamic region, ii) having brain damage in the basal ganglia and/or the thalamus, iii) having brain damage in the basal ganglia and thalamic region and the internal capsule, or iv) having brain damage in the basal ganglia, the thalamus, and the internal capsule. In one embodiment, the pharmaceutical composition of the present invention can be used for the treatment of cerebral infarction at a subacute to chronic stage, the cerebral infarction having brain damage in a penetrating branch territory. In one embodiment, the pharmaceutical composition of the present invention can be used for the treatment of cerebral infarction at a subacute to chronic stage, the cerebral infarction having brain damage in a penetrating branch territory, wherein the brain damage is brain damage including i) brain damage of the basal ganglia and thalamic region, ii) brain damage of the basal ganglia and/or the thalamus, iii) brain damage of the basal ganglia and thalamic region and the internal capsule, or iv) brain damage of the basal ganglia, the thalamus, and the internal capsule. In one embodiment, the pharmaceutical composition of the present invention can be used for the treatment of cerebral infarction at a subacute to chronic stage having local brain damage.

In one embodiment, the pharmaceutical composition of the present invention can be used for the treatment of cerebral infarction at a chronic stage. In one embodiment, the pharmaceutical composition of the present invention can be used for the treatment of penetrating branch infarction at a chronic stage. In one embodiment, the pharmaceutical composition of the present invention can be used for the treatment of lacunar infarction and/or BAD at a chronic stage. In one embodiment, the pharmaceutical composition of the present invention can be used for the treatment of penetrating branch infarction at a chronic stage, the penetrating branch infarction i) having brain damage in the basal ganglia and thalamic region, ii) having brain damage in the basal ganglia and/or the thalamus, iii) having brain damage in the basal ganglia and thalamic region and the internal capsule, or iv) having brain damage in the basal ganglia, the thalamus, and the internal capsule. In one embodiment, the pharmaceutical composition of the present invention can be used for the treatment of cerebral infarction at a chronic stage having brain damage in a penetrating branch territory. In one embodiment, the pharmaceutical composition of the present invention can be used for the treatment of cerebral infarction at a chronic stage, the cerebral infarction having brain damage in a penetrating branch territory, wherein the brain damage is brain damage including i) brain brain damage of the basal ganglia and thalamic region, ii) brain damage of the basal ganglia and/or the thalamus, iii) brain damage of the basal ganglia and thalamic region and the internal capsule, or iv) brain damage including brain damage of the basal ganglia, the thalamus, and the internal capsule. In one embodiment, the pharmaceutical composition of the present invention can be used for the treatment of cerebral infarction at a chronic stage having local brain damage.

In a preferred embodiment, the pharmaceutical composition of the present invention can markedly improve a motor function of a subject with cerebral infarction, for example, a subject with penetrating branch infarction (e.g., penetrating branch infarction at a subacute to chronic stage or penetrating branch infarction at a chronic stage) or a subject with cerebral infarction having brain damage in a penetrating branch territory (e.g., penetrating branch infarction at a subacute to chronic stage or penetrating branch infarction at a chronic stage) and/or cerebral infarction at a subacute to chronic stage (e.g., cerebral infarction at a chronic stage).

In one embodiment, the pharmaceutical composition of the present invention is a pharmaceutical composition for the treatment of cerebral infarction with mRS of 2 or more (e.g., 2 to 5, 3 to 5, or 3 or 4). In one embodiment, the pharmaceutical composition of the present invention is a pharmaceutical composition for the treatment of cerebral infarction at a subacute to chronic stage with mRS of 2 or more (e.g., 2 to 5, 3 to 5, or 3 or 4). In one embodiment, the pharmaceutical composition of the present invention is a pharmaceutical composition for the treatment of cerebral infarction at a chronic stage with mRS of 2 or more (e.g., 2 to 5, 3 to 5, or 3 or 4). In one embodiment, the pharmaceutical composition of the present invention is a pharmaceutical composition for the treatment of cerebral infarction at a chronic stage with mRS of 3 to 5. In one embodiment, the pharmaceutical composition of the present invention is a pharmaceutical composition for the treatment of cerebral infarction at a chronic stage with mRS of 3 or 4.

Scores of NIHSS which is a neurological severity evaluation scale of human stroke are 0 to 42, and a larger score represents being more severe. In a clinical trial on a therapeutic agent for cerebral infarction, NIHSS is also established as an index for recruiting patients or an index for evaluating the efficacy of drugs. In a clinical trial on a therapeutic agent for cerebral infarction at a chronic stage, for example, patients having an NIHSS score of 8 to 30 (https://clinicaltrials.gov/ct2/show/NCT04088149), 6 to 20 (https://www.clinicaltrials.gov/ct2/show/NCT04590118), or 5 to 14 (https://clinicaltrials.gov/ct2/show/NCT03202147) are subject to the clinical trial.

In one embodiment, the pharmaceutical composition of the present invention may be a pharmaceutical composition for the treatment of cerebral infarction (e.g., cerebral infarction at a subacute to chronic stage or cerebral infarction at a chronic stage) with NIHSS of 5 or more (e.g., 8 or more). In one embodiment, the pharmaceutical composition of the present invention may be a pharmaceutical composition for the treatment of cerebral infarction at a chronic stage with NIHSS of 8 or more. In one embodiment, the pharmaceutical composition of the present invention may be a pharmaceutical composition for the treatment of cerebral infarction (e.g., cerebral infarction at a subacute to chronic stage or cerebral infarction at a chronic stage) with mRS of 3 to 5 and NIHSS of 8 or more. In one embodiment, the pharmaceutical composition of the present invention may be a pharmaceutical composition for the treatment of cerebral infarction (e.g., cerebral infarction at a subacute to chronic stage or cerebral infarction at a chronic stage) with mRS of 3 or 4 and NIHSS of 8 or more.

In one embodiment, the pharmaceutical composition of the present invention is a pharmaceutical composition for the treatment of penetrating branch infarction (e.g., penetrating branch infarction i) having brain damage in the basal ganglia and thalamic region, ii) having brain damage in the basal ganglia and/or the thalamus, iii) having brain damage in the basal ganglia and thalamic region and the internal capsule, or iv) having brain damage in the basal ganglia, the thalamus, and the internal capsule, and/or lacunar infarction and/or BAD) at a chronic stage with mRS of 2 to 5 (e.g., 3 to 5 or 3 or 4). In one embodiment, the pharmaceutical composition of the present invention is a pharmaceutical composition for the treatment of penetrating branch infarction (e.g., penetrating branch infarction i) having brain damage in the basal ganglia and thalamic region, ii) having brain damage in the basal ganglia and/or the thalamus, iii) having brain damage in the basal ganglia and thalamic region and the internal capsule, or iv) having brain damage in the basal ganglia, the thalamus, and the internal capsule, and/or lacunar infarction and/or BAD) at a chronic stage with NIHSS of 5 or more (e.g., 8 or more).

In one embodiment, the pharmaceutical composition of the present invention may be a pharmaceutical composition for the treatment of cerebral infarction having brain damage in a penetrating branch territory (e.g., cerebral infarction having brain damage in a penetrating branch territory, wherein the brain damage is brain damage including i) brain damage of the basal ganglia and thalamic region, ii) brain damage of the basal ganglia and/or the thalamus, iii) brain damage of the basal ganglia and thalamic region and the internal capsule, or iv) brain damage of the basal ganglia, the thalamus, and the internal capsule) at a chronic stage with mRS of 2 to 5 (e.g., 3 to 5 or 3 or 4). In one embodiment, the pharmaceutical composition of the present invention may be a pharmaceutical composition for the treatment of cerebral infarction having brain damage in a penetrating branch territory (e.g., cerebral infarction having brain damage in a penetrating branch territory, wherein the brain damage is brain damage including i) brain damage of the basal ganglia and thalamic region, ii) brain damage of the basal ganglia and/or the thalamus, iii) brain damage of the basal ganglia and thalamic region and the internal capsule, or iv) brain damage of the basal ganglia, the thalamus, and the internal capsule) at a chronic stage with NIHSS of 5 or more (e.g., 8 or more).

In one embodiment, the pharmaceutical composition of the present invention is a pharmaceutical composition for the treatment of cerebral infarction at a chronic stage having local brain damage with mRS of 2 to 5 (e.g., 3 to 5 or 3 or 4).

In one embodiment, the pharmaceutical composition of the present invention may be a pharmaceutical composition for the treatment of brain damage in a penetrating branch territory associated with cerebral infarction (e.g., cerebral infarction at a subacute to chronic stage or cerebral infarction at a chronic stage). Examples of the brain damage in a penetrating branch territory include, but are not limited to, i) brain damage of the basal ganglia and thalamic region, ii) brain damage of the basal ganglia and/or the thalamus, iii) brain damage of the basal ganglia and thalamic region and the internal capsule, and iv) brain damage of the basal ganglia, the thalamus, and the internal capsule. In one embodiment, the pharmaceutical composition of the present invention may be a pharmaceutical composition for the treatment of i) brain damage of the basal ganglia and thalamic region, ii) brain damage of the basal ganglia and/or the thalamus, iii) brain damage of the basal ganglia and thalamic region and the internal capsule, or iv) brain damage of the basal ganglia, the thalamus, and the internal capsule, associated with cerebral infarction (e.g., cerebral infarction at a subacute to chronic stage or cerebral infarction at a chronic stage).

In one embodiment, the pharmaceutical composition of the present invention may be a pharmaceutical composition for the treatment of brain damage in a penetrating branch territory associated with cerebral infarction at a chronic stage with mRS of 2 to 5 (e.g., 3 to 5 or 3 or 4).

In one embodiment, the pharmaceutical composition of the present invention may be a pharmaceutical composition for the treatment of brain damage in a penetrating branch territory associated with cerebral infarction at a chronic stage with NIHSS of 5 or more (e.g., 8 or more).

The present invention also provides a method for treating cerebral infarction, comprising the step of administering the pharmaceutical composition of the present invention, a NeuroD1 protein, or a polynucleotide encoding the NeuroD1 protein to a subject (hereinafter, also referred to as the "treatment method of the present invention"). In one embodiment, the treatment method of the present invention may be a method for treating cerebral infarction, comprising the step of administering a therapeutically effective amount of the pharmaceutical composition of the present invention, a NeuroD1 protein, or a polynucleotide encoding the NeuroD1 protein to a subject. In one embodiment, the treatment method of the present invention may be a method for treating cerebral infarction, comprising the step of administering a therapeutically effective amount of a NeuroD1 protein or a polynucleotide encoding the NeuroD1 protein to a subject. In one embodiment, the treatment method of the present invention may be a method for treating cerebral infarction, comprising the step of administering a therapeutically effective amount of a polynucleotide encoding the NeuroD1 protein to a subject. In a typical embodiment, the polynucleotide encoding the NeuroD1 protein encodes the NeuroD1 protein in an expressible state. In one embodiment, the treatment method of the present invention comprises the step of administering DNA encoding the NeuroD1 protein to a subject. In one embodiment, the treatment method of the present invention comprises the step of administering a therapeutically effective amount of DNA encoding the NeuroD1 protein to a subject. In one embodiment, the treatment method of the present invention comprises the step of administering an expression vector comprising the polynucleotide encoding the NeuroD1 protein to a subject. In one embodiment, the treatment method of the present invention comprises the step of administering a therapeutically effective amount of an expression vector comprising the polynucleotide encoding the NeuroD1 protein to a subject. In one embodiment, the expression vector for use in the treatment method of the present invention is an AAV vector, a retrovirus vector, a Sendai virus vector, or a lentivirus vector. In one embodiment, the expression vector for use in the treatment method of the present invention is an AAV vector. In one embodiment, the treatment method of the present invention comprises the step of administering mRNA encoding the NeuroD1 protein to a subject. In one embodiment, the treatment method of the present invention comprises the step of administering a therapeutically effective amount of mRNA encoding the NeuroD1 protein to a subject. In one embodiment, the mRNA for use in the treatment method of the present invention is incorporated in DDS. In one embodiment, the mRNA for use in the treatment method of the present invention is encapsulated into a drug delivery carrier. In one embodiment, the mRNA for use in the treatment method of the present invention is encapsulated into nanoparticles. In one embodiment, the mRNA for use in the treatment method of the present invention is encapsulated into lipid nanoparticles.

In one embodiment, the treatment method of the present invention may be a method for treating penetrating branch infarction (e.g., penetrating branch infarction i) having brain damage in the basal ganglia and thalamic region, ii) having brain damage in the basal ganglia and/or the thalamus, iii) having brain damage in the basal ganglia and thalamic region and the internal capsule, or iv) having brain damage in the basal ganglia, the thalamus, and the internal capsule, and/or lacunar infarction and/or BAD). In one embodiment, the treatment method of the present invention may be a method for treating cerebral infarction at a subacute to chronic stage. In one embodiment, the treatment method of the present invention may be a method for treating cerebral infarction at a chronic stage. In one embodiment, the treatment method of the present invention may be a method for treating penetrating branch infarction (e.g., penetrating branch infarction i) having brain damage in the basal ganglia and thalamic region, ii) having brain damage in the basal ganglia and/or the thalamus, iii) having brain damage in the basal ganglia and thalamic region and the internal capsule, or iv) having brain damage in the basal ganglia, the thalamus, and the internal capsule, and/or lacunar infarction and/or BAD) at a subacute to chronic stage. In one embodiment, the treatment method of the present invention may be a method for treating lacunar infarction and/or BAD at a subacute to chronic stage. In one embodiment, the treatment method of the present invention may be a method for treating penetrating branch infarction (e.g., penetrating branch infarction i) having brain damage in the basal ganglia and thalamic region, ii) having brain damage in the basal ganglia and/or the thalamus, iii) having brain damage in the basal ganglia and thalamic region and the internal capsule, or iv) having brain damage in the basal ganglia, the thalamus, and the internal capsule, and/or lacunar infarction and/or BAD) at a chronic stage. In one embodiment, the treatment method of the present invention may be a method for treating, cerebral infarction having brain damage in a penetrating branch territory (e.g., cerebral infarction having brain damage in a penetrating branch territory, wherein the brain damage is brain damage including i) brain damage of the basal ganglia and thalamic region, ii) brain damage of the basal ganglia and/or the thalamus, iii) brain damage of the basal ganglia and thalamic region and the internal capsule, or iv) brain damage of the basal ganglia, the thalamus, and the internal capsule). In one embodiment, the treatment method of the present invention may be a method for treating cerebral infarction at a subacute to chronic stage, the cerebral infarction having brain damage in a penetrating branch territory (e.g., cerebral infarction having brain damage in a penetrating branch territory, wherein the brain damage is brain damage including i) brain damage of the basal ganglia and thalamic region, ii) brain damage of the basal ganglia and/or the thalamus, iii) brain damage of the basal ganglia and thalamic region and the internal capsule, or iv) brain damage of the basal ganglia, the thalamus, and the internal capsule). In one embodiment, the treatment method of the present invention may be a method for treating cerebral infarction at a chronic stage, the cerebral infarction having brain damage in a penetrating branch territory (e.g., cerebral infarction having brain damage in a penetrating branch territory, wherein the brain damage is brain damage including i) brain damage of the basal ganglia and thalamic region, ii) brain damage of the basal ganglia and/or the thalamus, iii) brain damage of the basal ganglia and thalamic region and the internal capsule, or iv) brain damage of the basal ganglia, the thalamus, and the internal capsule).

In one embodiment, the treatment method of the present invention may be a method for treating cerebral infarction having local brain damage. In one embodiment, the treatment method of the present invention may be a method for treating cerebral infarction at a subacute to chronic stage having local brain damage. In one embodiment, the treatment method of the present invention may be a method for treating cerebral infarction at a chronic stage having local brain damage.

In one embodiment, the treatment method of the present invention may be a method for treating cerebral infarction (e.g., cerebral infarction at a subacute to chronic stage or cerebral infarction at a chronic stage) with mRS of 2 or more. In one embodiment, the treatment method of the present invention may be a method for treating cerebral infarction (e.g., cerebral infarction at a subacute to chronic stage, or cerebral infarction at a chronic stage) with mRS of 3 to 5. In one embodiment, the treatment method of the present invention may be a method for treating cerebral infarction (e.g., cerebral infarction at a subacute to chronic stage or cerebral infarction at a chronic stage) with NIHSS of 5 or more. In one embodiment, the treatment method of the present invention may be a method for treating cerebral infarction (e.g., cerebral infarction at a subacute to chronic stage or cerebral infarction at a chronic stage) with NIHSS of 8 or more. In one embodiment, the treatment method of the present invention may be a method for treating cerebral infarction (e.g., cerebral infarction at a subacute to chronic stage or cerebral infarction at a chronic stage) with mRS of 3 to 5 and NIHSS of 8 or more.

The cerebral infarction with mRS of 2 or more to be treated using the pharmaceutical composition of the present invention or to be treated by the treatment of the present invention (hereinafter, also referred to as "to be treated according to the present invention") includes cerebral infarction evaluated as mRS of 2 or more for any period after the onset of cerebral infarction. The cerebral infarction with mRS of 3 to 5 to be treated according to the present invention includes cerebral infarction evaluated as mRS of 3 to 5 for any period after the onset of cerebral infarction. For example, cerebral infarction evaluated as mRS of 3 for a period from the onset of cerebral infarction to the obtainment of a stable score of mRS and then improved in mRS to 2 by symptomatic recovery is also included in the cerebral infarction with mRS of 3 to 5 to be treated according to the present invention. Likewise, the cerebral infarction with NIHSS of 5 or more to be treated according to the present invention includes cerebral infarction evaluated as NIHSS of 5 or more for any period after the onset of cerebral infarction. The cerebral infarction with NIHSS of 8 or more to be treated according to the present invention includes cerebral infarction evaluated as NIHSS of 8 or more for any period after the onset of cerebral infarction. For example, cerebral infarction evaluated as NIHSS of 8 or more for a period from the onset of cerebral infarction to the obtainment of a stable score of NIHSS and then improved in NIHSS by symptomatic recovery is also included in the cerebral infarction with NIHSS of 8 or more to be treated according to the present invention. The cerebral infarction to be treated according to the present invention may be penetrating branch infarction (e.g., lacunar infarction and/or BAD) and may be, for example, penetrating branch infarction at a subacute to chronic stage or at a chronic stage. Alternatively, the cerebral infarction to be treated according to the present invention may be cerebral infarction other than penetrating branch infarction, for example, cerebral infarction at a subacute to chronic stage other than penetrating branch infarction.

The subject to which the treatment method and the pharmaceutical composition, etc. of the present invention are applied may be an arbitrary mammal (test subject) including a primate (a human and a nonhuman primate), livestock (a horse, a bovine, sheep, a goat, a pig, etc.), a pet animal (a dog, a cat, a rabbit, etc.), a laboratory (test) animal (a mouse, a rat, etc.). The subject is preferably a primate, particularly preferably a human. The subject to which the treatment method and the pharmaceutical composition, etc. of the present invention are applied is preferably a subject in need of the application of the treatment method and the pharmaceutical composition, etc. and may be, for example, a subject having cerebral infarction, for example, penetrating branch infarction (e.g., penetrating branch infarction at a subacute to chronic stage or penetrating branch infarction at a chronic stage) or cerebral infarction at a subacute to chronic stage (e.g., cerebral infarction at a chronic stage), or a subject suspected of having cerebral infarction, for example, penetrating branch infarction (e.g., penetrating branch infarction at a subacute to chronic stage or penetrating branch infarction at a chronic stage) or cerebral infarction at a subacute to chronic stage (e.g., cerebral infarction at a chronic stage). Examples of the penetrating branch infarction include, but are not limited to, i) penetrating branch infarction having brain damage in the basal ganglia and thalamic region, ii) penetrating branch infarction having brain damage in the basal ganglia and/or the thalamus, iii) penetrating branch infarction having brain damage in the basal ganglia and thalamic region and the internal capsule, and iv) penetrating branch infarction having brain damage in the basal ganglia, the thalamus, and the internal capsule. The subject to which the treatment method and the pharmaceutical composition, etc. of the present invention are applied may also be a subject having cerebral infarction having brain damage in a penetrating branch territory (e.g., cerebral infarction at a subacute to chronic stage or cerebral infarction at a chronic stage), or a subject suspected of having it. Examples of the cerebral infarction having brain damage in a penetrating branch territory include, but are not limited to, cerebral infarction in which the brain damage is brain damage including i) brain damage of the basal ganglia and thalamic region, ii) brain damage of the basal ganglia and/or the thalamus, iii) brain damage of the basal ganglia and thalamic region and the internal capsule, or iv) brain damage of the basal ganglia, the thalamus, and the internal capsule.

For a human, a period having the possibility of worsening symptoms from immediately after the onset of cerebral infarction to less than 72 hours after the onset is called "acute stage". For a human, a period from 72 hours after the onset of cerebral infarction to less than 1 month after the onset is called "subacute stage" (also called convalescent stage). A period of 1 month or later after the onset is called "chronic stage". The subacute stage is a period in which a high degree of recovery from paralysis can be expected as compared with the chronic stage. For a human, the subacute to chronic stage of cerebral infarction refers to a period of 72 hours or later after the onset of cerebral infarction and may be, for example, a period of 1 month or later after the onset of cerebral infarction or may be a period of 3 months or later after the onset of cerebral infarction, for example, 6 months or later after the onset of cerebral infarction. The "acute stage", the "subacute stage", and the "chronic stage" for a nonhuman animal subject can be properly determined as periods corresponding to the "acute stage", the "subacute stage", and the "chronic stage", respectively, for a human by those skilled in the art.

The dose and the number of doses of the pharmaceutical composition of the present invention, the NeuroD1 protein, or the polynucleotide encoding the NeuroD1 protein to the subject can be appropriately adjusted according to the disease to be treated, severity, the age, body weight, and state of the subject, etc. The dose can be, for example, 0.0001 mg to 15 mg/kg body weight of the NeuroD1 protein per dose.

Examples of the method for administering the pharmaceutical composition of the present invention, the NeuroD1 protein, or the polynucleotide encoding the NeuroD1 protein to the subject include, but are not particularly limited to, local administration by a surgical approach, catheter administration to the heart, administration using a ultrasound contrast agent (microbubble), intravenous administration, administration by needling in a lower limb, local injection administration, subcutaneous administration, intradermal administration, and intramuscular administration. Local administration (e.g., intracranial administration, intraparenchymal administration, and intracerebroventricular administration) by a surgical approach to an infarct area or a damaged area in the subject is preferred.

In one embodiment, the pharmaceutical composition of the present invention, the NeuroD1 protein, or the polynucleotide encoding the NeuroD1 protein is administered to the subject by local administration by a surgical approach. In one embodiment, the pharmaceutical composition of the present invention, the NeuroD1 protein, or the polynucleotide encoding the NeuroD1 protein is administered to the subject by intracranial administration. In one embodiment, the pharmaceutical composition of the present invention, the NeuroD1 protein, or the polynucleotide encoding the NeuroD1 protein is administered to the subject by intraparenchymal administration. In one embodiment, the pharmaceutical composition of the present invention, the NeuroD1 protein, or the polynucleotide encoding the NeuroD1 protein is administered to the subject by intracerebroventricular administration.

The method for administering the pharmaceutical composition of the present invention, the NeuroD1 protein, or the polynucleotide encoding the NeuroD1 protein to the subject comprises administering the pharmaceutical composition of the present invention, the NeuroD1 protein, or the polynucleotide encoding the NeuroD1 protein to at least one administration site of an infarct area or a damaged area. When a plurality of infarct areas or damaged areas are present, the pharmaceutical composition of the present invention, the NeuroD1 protein, or the polynucleotide encoding the NeuroD1 protein may be administered to at least one administration site of each infarct area or damaged area. In one embodiment, in this method, the pharmaceutical composition of the present invention, the NeuroD1 protein, or the polynucleotide encoding the NeuroD1 protein may be administered to one administration site per infarct area or damaged area of one or a plurality of infarct areas or damaged areas in the subject.

The number of doses of the pharmaceutical composition of the present invention, the NeuroD1 protein, or the polynucleotide encoding the NeuroD1 protein to the subject is not particularly limited and may be any of single administration and multiple administration. The timing of single administration may be an arbitrary timing after the onset of cerebral infarction, for example, a subacute to chronic stage or a chronic stage and is not particularly limited. The timing may be, for example, 1 month, 3 months, 6 months, 1 year, 1.5 years, 2 years, 3 years, 5 years, 7 years, or 10 years after the onset. In the case of multiple administration, initial administration may be performed, for example, at an arbitrary timing after the onset of cerebral infarction, for example, at a subacute to chronic stage, or at a chronic stage. After the initial administration, administration may be performed at intervals of given time, for example, every six months.

A device for use in the administration of the pharmaceutical composition of the present invention, the NeuroD1 protein, or the polynucleotide encoding the NeuroD1 protein into the brain of the subject is not particularly limited as long as the device is capable of administration to the site of interest. For example, a biopsy/injection needle kit (MIZUHO Corp., MES-CG07-200-01) or a puncture needle (International Publication No. WO2020/241833) may be used.

The treatment method of the present invention can effectively treat cerebral infarction. In one embodiment, the treatment method of the present invention can effectively treat cerebral infarction at a subacute to chronic stage. In one embodiment, the treatment method of the present invention can effectively treat cerebral infarction at a chronic stage. In one embodiment, the treatment method of the present invention can effectively treat penetrating branch infarction (e.g., lacunar infarction and/or BAD). In one embodiment, the treatment method of the present invention can effectively treat penetrating branch infarction at a subacute to chronic stage (particularly, at a chronic stage). In one embodiment, the treatment method of the present invention can effectively treat lacunar infarction and/or BAD at a subacute to chronic stage (particularly, at a chronic stage). In one embodiment, the treatment method of the present invention can effectively treat cerebral infarction having brain damage in a penetrating branch territory. In one embodiment, the treatment method of the present invention can effectively treat cerebral infarction at a subacute to chronic stage (particularly, at a chronic stage) having brain damage in a penetrating branch territory. In a preferred embodiment, the treatment method of the present invention can markedly improve a motor function of a subject with penetrating branch infarction (e.g., penetrating branch infarction at a subacute to chronic stage, particularly, penetrating branch infarction at a chronic stage) or a subject with cerebral infarction having brain damage in a penetrating branch territory (e.g., cerebral infarction at a subacute to chronic stage, particularly, cerebral infarction at a chronic stage) and/or cerebral infarction at a subacute to chronic stage (e.g., cerebral infarction at a chronic stage). In the present invention, the "treatment" of cerebral infarction is not limited to the disappearance or alleviation of thrombus causative of cerebral infarction or the resulting interruption of brain blood flow itself and includes the disappearance, relief, improvement, or prevention or delay of progression of symptoms (e.g., motor dysfunction) of cerebral infarction. In one embodiment, the "treatment" of cerebral infarction according to the present invention may be the treatment of symptoms (e.g., motor dysfunction) of cerebral infarction.

The treatment method and the pharmaceutical composition of the present invention can preferably bring about recovery from both damage of the basal ganglia and thalamic region and damage of the internal capsule. The damage of the basal ganglia and thalamic region and the recovery therefrom can be indicated, for example, by using the expression level of dopamine transporter as an index, as shown in Examples mentioned later. The damage of the internal capsule and the recovery therefrom can be indicated, for example, by using an FA value which indicates corticospinal tract as an index by DTI analysis as shown in Examples mentioned later. The treatment method and the pharmaceutical composition of the present invention can also preferably bring about the suppression of increase in the volume of the lateral ventricle associated with brain damage. The suppression of the increase in the volume of the lateral ventricle refers to the suppression of parenchymal atrophy of the brain or recovery from injury of a parenchymal tissue of the brain. The volume of the lateral ventricle can be determined by arbitrary brain image analysis, for example, the analysis of T1-weighted images and/or T2-weighted images obtained by MRI photographing as shown in Examples mentioned later. The treatment method and the pharmaceutical composition of the present invention can also preferably promote recovery from motor dysfunction.

The present invention also provides the pharmaceutical composition of the present invention, a NeuroD1 protein, or a polynucleotide encoding the NeuroD1 protein for use in the treatment of cerebral infarction, preferably penetrating branch infarction (e.g., penetrating branch infarction at a subacute to chronic stage or penetrating branch infarction at a chronic stage), cerebral infarction having brain damage in a penetrating branch territory (e.g., cerebral infarction at a subacute to chronic stage or cerebral infarction at a chronic stage), cerebral infarction having local brain damage (e.g., cerebral infarction at a subacute to chronic stage or cerebral infarction at a chronic stage), or cerebral infarction at a subacute to chronic stage (e.g., cerebral infarction at a chronic stage). The present invention further provides the pharmaceutical composition of the present invention, a NeuroD1 protein, or a polynucleotide encoding the NeuroD1 protein for use in the treatment of cerebral infarction (e.g., cerebral infarction at a subacute to chronic stage or cerebral infarction at a chronic stage) with mRS of 2 or more (e.g., 3 to 5) and/or NIHSS of 5 or more (e.g., 8 or more).

The present invention also provides use of a NeuroD1 protein or a polynucleotide encoding the NeuroD1 protein in the manufacture of a pharmaceutical composition for the treatment of cerebral infarction, preferably penetrating branch infarction (e.g., penetrating branch infarction at a subacute to chronic stage or penetrating branch infarction at a chronic stage), cerebral infarction having brain damage in a penetrating branch territory (e.g., cerebral infarction at a subacute to chronic stage or cerebral infarction at a chronic stage), cerebral infarction having local brain damage (e.g., cerebral infarction at a subacute to chronic stage or cerebral infarction at a chronic stage), or cerebral infarction at a subacute to chronic stage (e.g., cerebral infarction at a chronic stage). The present invention further provides use of a NeuroD1 protein or a polynucleotide encoding the NeuroD1 protein in the manufacture of a pharmaceutical composition for the treatment of cerebral infarction (e.g., cerebral infarction at a subacute to chronic stage or cerebral infarction at a chronic stage) with mRS of 2 or more (e.g., 3 to 5) and/or NIHSS of 5 or more (e.g., 8 or more).

As mentioned above, the present invention also includes the treatment method of the present invention, a NeuroD1 protein or a polynucleotide encoding the NeuroD1 protein for use in the treatment of cerebral infarction, and use of a NeuroD1 protein or a polynucleotide encoding the NeuroD1 protein in the manufacture of a pharmaceutical composition for the treatment of cerebral infarction. Examples of the cerebral infarction to be treated by the pharmaceutical application of the NeuroD1 protein or the polynucleotide encoding the NeuroD1 protein include the cerebral infarction described in the "pharmaceutical composition of the present invention" mentioned above. The pharmaceutical application of the NeuroD1 protein or the polynucleotide encoding the NeuroD1 protein of the present invention can be readily carried out by those skilled in the art with reference to the description regarding the "pharmaceutical composition of the present invention".

### [Examples]

Hereinafter, the present invention will be described further specifically with reference to Examples. However, the technical scope of the present invention is not limited by these Examples.

The operations of each step described in Examples given below were able to be carried out by use of a known technique unless otherwise specified. As for a part using a commercially available kit or reagent, etc., an experiment was conducted in accordance with the attached protocol unless otherwise specified.

Some compounds described herein were named using naming software such as ACD/Name^{(R)} (Advanced Chemistry Development, Inc.).

For convenience, the concentration unit, mol/L, is represented by letter M. For example, "1 M aqueous sodium hydroxide solution" means 1 mol/L aqueous sodium hydroxide solution.

For the development of therapeutic agents for cerebral infarction, rodent cerebral infarction models have been used in many previous studies on therapeutic agents for cerebral infarction at an acute stage. Meanwhile, the guideline of the International Stroke Conference recommends making evaluation using nonhuman primates having more similar vascular vessels and tissue structures of the brain to the human ones for the purpose of reducing the discrepancy between nonclinical and clinical results. For example, a MCAO model or a model having internal capsule damage has been reported as a cerebral infarction model obtained using a nonhuman primate. However, in these models, motor dysfunction with high severity is not maintained.

The present inventors considered that rodent cerebral infarction models failed to allow gross motor skills to be evaluated by motor dysfunction scoring of NHPSS or the like and were thus difficult to use in research on therapeutic agents for cerebral infarction which brought about recovery from motor dysfunction. There is no report on a case where gross motor skills can be evaluated by NHPSS or the like up to a subacute to chronic stage with high reproducibility in previously reported cerebral infarction models obtained using rodents, nonhuman primates, or the like. Thus, the present inventors considered that the existing cerebral infarction models failed to allow therapeutic agents for cerebral infarction at a subacute to chronic stage to be sufficiently evaluated. Accordingly, a nonhuman primate was used by the present inventors to construct a cerebral infarction model that enabled evaluation by motor dysfunction scoring and was able to maintain long-term motor dysfunction. The present inventors hypothesized that: infarction in the basal ganglia would be important for motor dysfunction, from the findings that in cerebral cortex infarction, a decreased level of consciousness tends to appear as a symptom; and on the other hand, BAD, a form of penetrating branch infarction, easily causes infarction in the basal ganglia, and motor paralysis tends to persist after cerebral infarction. The present inventors further hypothesized, from their previous findings, that thalamus damage would be important for the long-term maintenance of motor dysfunction. Moreover, the present inventors considered that internal capsule damage would be also important for the cerebral infarction model of interest because the internal capsule is a cluster of nerve fibers. From these findings and considerations, the present inventors predicted that a model of cerebral infarction (particularly, a model of penetrating branch infarction or a model of cerebral infarction having brain damage in a penetrating branch territory) capable of maintaining long-term motor dysfunction could be constructed by damaging a territory supplied by the penetrating branch (the basal ganglia, the thalamus, and the internal capsule) in a nonhuman primate, and then carried out the following studies.

For the production of a nonhuman primate animal model of cerebral infarction, a model having brain damage induced on either the right side of the brain or the left side of the brain of a nonhuman primate was considered. In cerebral infarction patients, the infarction often occurs either on the right side of the brain or on the left side of the brain. Therefore, an animal model of cerebral infarction in which brain damage was also induced either on the right side of the brain or on the left side of the brain was considered as an animal model of cerebral infarction more similar to a clinical model. In the present Examples, endothelin was injected to the left side of the brain to induce brain damage on the left side of the brain, thereby inducing motor dysfunction on the right side of the body.

### (Example 1) Study for construction of cynomolgus macaque cerebral infarction model

A study was made on a method for producing a cerebral infarction model (a nonhuman primate animal model of cerebral infarction) having basal ganglia damage, thalamus damage, and internal capsule damage in which motor dysfunction would persist for 3 months or longer, using male cynomolgus macaques (3 years of age or older, SNBL). The present inventors considered that endothelin-1, which has a vasoconstrictive effect, could be injected to the basal ganglia region and the thalamus region to induce vasoconstriction at the injection site, resulting in focal cerebral ischemia to cause neuronal death. Accordingly, endothelin-1 (human-derived; Peptide Institute, Inc., product code: 4198-v, Cys-Ser-Cys-Ser-Ser-Leu-Met-Asp-Lys-Glu-Cys-Val-Tyr-Phe-Cys-His-Leu-Asp-Ile-Ile-Trp (having disulfide bonds between Cys¹ and Cys¹⁵ and between Cys³ and Cys¹¹; SEQ ID NO: 9)) was injected to the brain of the cynomolgus macaque at different injection sites, injection doses, and injection rates as given below, and examined for the degree to which symptoms of motor dysfunction were induced in association with neuronal death.

First, a systemic anesthetic ketamine hydrochloride (Ketaral for intramuscular injection, 500 mg; Daiichi Sankyo Propharma Co., Ltd., Approval No.: 21600AMZ00252; hereinafter, referred to as "ketamine hydrochloride") was introduced in an amount of approximately 10 mg/kg body weight to the male cynomolgus macaques by intramuscular injection (hereinafter, referred to as "i.m."). The cynomolgus macaque under anesthesia was subjected to intratracheal intubation using a tracheal tube (Covidien Japan Inc., 301-40G), and anesthesia was maintained by the inhalation of isoflurane (Isoflurane Inhalation Solution "VTRS"; Mylan N.V., Approval No: 22700AMX00134; hereinafter, referred to as "isoflurane") under artificial breathing or spontaneous breathing. Hair on the head of the cynomolgus macaque under anesthesia was shaved with an electric shaver. Then, the cynomolgus macaque was fixed in a stereotaxic instrument (Narishige Scientific Instrument Lab., SN-3N). Then, the head skin of the cynomolgus macaque was incised with attention given to bleeding. These steps were performed by the same procedures among all of the following study experiments.

Injection sites of endothelin-1 into the brain were studied with reference to a known cynomolgus macaque brain map (J. Szabo et al., J. Comp. Neurol., vol. 222 (2), pp. 265-300, 1984). Since the cynomolgus macaque brain map employs an anterior-posterior (AP) coordinate on the anteroposterior axis and a midline (ML) coordinate on the lateral axis, the AP coordinate on the anteroposterior axis and the ML coordinate on the lateral axis were used as coordinates of the injection site of endothelin-1 into the brain. At first, the intersection between the line connecting both ear canals (interaural line) and the cranial sagittal suture was used as a base point (AP coordinate: 0 mm, ML coordinate: 0 mm; median) for the injection site. The injection site is indicated by the following coordinates.
A: anterior distance from the base point of the AP coordinate
L: left distance from the base point of the ML coordinate
D: vertically downward depth in the brain from the pachymeninx

### (a) Study Experiment 1

An injection site and an injection dose of endothelin-1 into the brain were studied. Holes of approximately 1 mm in diameter were opened (a total of 3 or 6 holes) at positions of (i) A: 20.0 mm and L: 7.0 mm, (ii) A: 20.0 mm and L: 9.0 mm, and (iii) A: 20.0 mm and L: 11.0 mm in the skull of one out of two cynomolgus macaque individuals and at the positions of (i) to (iii) as well as positions of (iv) A: 15.0 mm and L: 7.0 mm, (v) A: 15.0 mm and L: 9.0 mm, and (vi) A: 15.0 mm and L: 11.0 mm in the skull of the other individual using a drill (Muromachi Kikai Co., Ltd., HSD-1000; hereinafter, this product is referred to as a "drill"). A cannula (Top Spinal Needle; TOP Corp., 02036 (25 G × 89 mm); hereinafter, this product is referred to as a "cannula") and a tube (Plastics One, C312VT; hereinafter, this product is referred to as a "tube") were inserted from each hole and indwelled such that the tip of the cannula was located at the depth (D) of 19.0 mm. Endothelin-1 diluted with 0.1% acetic acid (prepared by diluting acetic acid (Wako, 017-00256) with Milli-Q^{(R)} water) was injected to a total of 3 or 6 sites per individual, which correspond to the respective positions described above, using the indwelled cannula. Endothelin-1 was injected at a concentration of 2.0 µg/µL, 2 µL/site, and an injection rate of 1 µL/min. The injection to the three sites and the injection to the six sites were each carried out at N = 1.

In this Study Experiment 1, mild motor dysfunction was observed on the next day in the individual treated by the injection to three sites, of the two cynomolgus macaque individuals treated as described above, and however, the dysfunction disappeared 1 week after the treatment. Symptoms of motor dysfunction in the right forelimb and hindlimb were clearly observed up to 2 weeks after treatment in the individual treated by the injection to six sites, and however, the dysfunction reversed spontaneously 4 weeks later to the extent that mild paralysis was seen.

### (b) Study Experiment 2

The injection site of endothelin-1 and the concentration of endothelin-1 to be injected were changed from the conditions used in Study Experiment 1. Holes of approximately 1 mm in diameter were opened (a total of 6 holes) at positions of (i) A: 22.0 mm and L: 11.0 mm, (ii) A: 19.0 mm and L: 11.0 mm, (iii) A: 16.0 mm and L: 11.0 mm, (iv) A: 22.0 mm and L: 8.0 mm, (v) A: 19.0 mm and L: 8.0 mm, and (vi) A: 16.0 mm and L: 8.0 mm in the skull of a cynomolgus macaque using a drill. A microsyringe (HAMILTON, 1705RN; hereinafter, this product is referred to as a "microsyringe") connected to a microinjector (Narishige Scientific Instrument Lab., IMS-20; hereinafter, this product is referred to as a "microinjector") was inserted from each hole and indwelled such that the tip of its needle was located at the depth (D) of 19.0 mm. Endothelin-1 diluted with 0.1% acetic acid was injected to a total of 6 sites per individual, which correspond to the respective positions described above, using the microsyringe. Endothelin-1 was injected at a concentration of 4.0 µg/µL, 4 µL/site, and an injection rate of 1 µL/min. The injection was carried out at N = 2.

Symptoms of motor dysfunction manifested by the cynomolgus macaques treated as described above were mild paralysis in the right forelimb and hindlimb on the impaired side (dysfunction side) in one individual and no symptom in the other individual.

In Study Experiment 2, deposition was seen in a residual sample of the endothelin-1 solution. Therefore, in Study Experiment 3 or later, the concentration of endothelin-1 was decreased to 2.0 µg/µL so as not to deposit endothelin-1 in a solution.

### (c) Study Experiment 3

The results of Study Experiment 1 and Study Experiment 2 revealed that in the case of using an interaural line as a base point of the AP coordinate, endothelin-1 was not able to be always injected to the region of interest due to difference among individuals. Accordingly on the basis of the present inventors' findings obtained so far, the present inventors conceived the idea that the bregma (Bregma) which is the intersection of the sagittal and coronal sutures on the anterior surface of the skull was used as a base point. In Study Experiment 3 or later, Bregma was used as a base point (B: 0 mm, ML coordinate: 0 mm; median) for the injection site. The injection site is indicated by the following coordinates.
B: distance along the anteroposterior axis from Bregma as a base point (a positive numeric value represents an anterior distance)
L: left distance from the base point of the ML coordinate
D: vertically downward depth in the brain from the pachymeninx

There exists no cynomolgus macaque brain map with Bregma as a base point. Therefore, the injection sites were examined on the basis of the findings obtained so far by the present inventors.

In Study Experiment 3, the injection dose of endothelin-1 per injection site was increased compared to the conditions of Study Experiment 2. In Study Experiment 3, holes of approximately 1 mm in diameter were opened (a total of 3 holes) at positions of (i) B: 2.0 mm and L: 8.0 mm, (ii) B: 5.0 mm and L: 8.0 mm, and (iii) B: 9.0 mm and L: 8.0 mm in the skull of a cynomolgus macaque using a drill. A microsyringe connected to a microinjector was inserted from each hole and indwelled such that the tip of its needle was located at the depth (D) of 19.0 mm. Endothelin-1 diluted with 0.1% acetic acid was injected to a total of 3 sites per individual, which correspond to the respective positions described above, using the microsyringe. Endothelin-1 was injected at a concentration of 2.0 µg/µL, a dose of 10 µL/site, and an injection rate of 1 µL/min. The injection was carried out at N = 2.

One individual of the treated cynomolgus macaques had an evaluation scale mRS (Table 1) of 5 on the day following treatment and manifested a recumbent posture, a decreased level of consciousness, decline in locomotor activity, and complete paralysis of the right forelimb and hindlimb on the impaired side. This individual had mRS of 4 even 1 week after surgery. The other cynomolgus macaque individual treated in the same manner had mRS of 3 on the day following treatment and manifested decline in locomotor activity and complete paralysis of the right forelimb and hindlimb on the impaired side, though a decreased level of consciousness was absent. Furthermore, this latter individual also persistently manifested decline in locomotor activity and complete paralysis of the right forelimb and hindlimb on the impaired side even 1 week after treatment. These results indicated that: the injection of endothelin under the conditions of Study Experiment 3 can induce motor dysfunction in an individual and provide more stable persistence of the induced symptoms as compared with Study Experiments 1 and 2. In the two cynomolgus macaque individuals treated in Study Experiment 3, motor dysfunction was found to persist up to at least a subacute stage.

### (d) Study Experiment 4

The following study was conducted on the basis of the conditions of Study Experiment 3. In order to more reliably induce brain damage, the injection dose of endothelin-1 was increased to 30 µL/site at 2.0 µg/µL. Holes of approximately 1 mm in diameter were opened (a total of 3 holes; only the injection site of (iii) differed between individuals) at positions of (i) B: 2.0 mm and L: 8.0 mm, (ii) B: 5.0 mm and L: 8.0 mm, and (iii) B: 9.0 mm or 11.0 mm and L: 8.0 mm from Bregma as a base point in the skull of each of two cynomolgus macaque individuals using a drill. A microsyringe connected to a microinjector was inserted from each hole and indwelled such that the tip of its needle was located at the depth (D) of 19.0 mm (from the pachymeninx). Endothelin-1 was injected to a total of 3 sites per individual, which correspond to the respective positions described above, using the microsyringe. Endothelin-1 was injected at a concentration of 2.0 µg/µL, a dose of 30 µL/site, and an injection rate of 1 µL/min. The injection was carried out at N = 1 under conditions where only the injection site of (iii) differed from each other.

The two cynomolgus macaque individuals treated had mRS (Table 1) of 4 after approximately 1 month from the treatment and manifested severe impairment. These individuals manifested decline in locomotor activity and recumbent or seated posture (being unable to stand-up). The two individuals had an NHPSS motor system score (Table 2) of 19 (impaired side/unimpaired side = 14/5) or 17 (impaired side/unimpaired side = 14/3). The score of 16 is the maximum score for the forelimb and hindlimb on one side. The two individuals then mildly recovered spontaneously and were however one individual (even on day 91 after the treatment) and the other individual (even on day 92 after the treatment) had mRS of 3 or 4 and an NHPSS motor system score (Table 2) of 12 (impaired side/unimpaired side = 12/0) or 14 (impaired side/unimpaired side = 14/0).

From the results of Study Experiment 4, it was shown that in the case of using a cynomolgus macaque as a nonhuman primate model of cerebral infarction, conditions involving injecting endothelin-1 at a concentration of 2.0 µg/µL and 30 µL/site (60 µg per injection site) per injection site are sufficient for maintaining the impairment for a long period. The two cynomolgus macaque individuals treated in Study Experiment 4 were considered as models enabling evaluation of human cerebral infarction at a subacute to chronic stage.

### (e) Study Experiment 5

In Study Experiment 5, a damaged area (infarct area) generated by the injection of endothelin was identified. Holes of approximately 1 mm in diameter were opened (a total of 3 holes) at positions of (i) B: 2.0 mm and L: 8.0 mm, (ii) B: 9.0 mm and L: 5.0 mm, and (iii) B: 9.0 mm and L: 13.0 mm from Bregma as a base point in the skull of a cynomolgus macaque using a drill. A microsyringe connected to a microinjector was inserted from each hole and indwelled such that the tip of its needle was located at the depth (D) of 19.0 mm (for (i) and (iii) described above) or the depth (D) of 13.0 mm (for (ii) described above) from the pachymeninx. Endothelin-1 was injected to a total of 3 sites per individual, which correspond to the respective positions described above, using the microsyringe. On the next day, holes of approximately 1 mm in diameter were further opened at positions of (iv) B: 5.0 mm and L: 8.0 mm and (v) B: 9.0 mm and L: 8.0 mm using a drill, and a microsyringe connected to a microinjector was inserted from the holes of (iv) and (v) and indwelled such that the tip of its needle was located at the depth (D) of 19.0 mm from the pachymeninx. Endothelin-1 was injected to a total of 2 sites per individual, which correspond to the respective positions ((iv) and (v)) described above, using the microsyringe. In this experiment, endothelin-1 was injected at a concentration of 2.0 µg/µL, a dose of 30 µL/site, and an injection rate of 1 µL/min. The injection was carried out at N = 1.

A damaged area on 3 days after the initial injection treatment (2 days after the additional injection treatment) was identified in the brains of the treated cynomolgus macaque individuals by a staining method (TTC staining) using a 2% solution (prepared by dissolution in physiological saline) of 2,3,5-triphenyl tetrazolium chloride (Nacalai Tesque, Inc., 35317-32). As a result, damaged areas were observed in the caudate nucleus, the putamen, the globus pallidus, the ventral lateral nucleus of the thalamus, and the ventral posterior nucleus of the thalamus, and in addition, a damaged area also extended over the internal capsule (Figure 1). It was also considered that: injection at the site of (i) injected endothelin to the ventral lateral nucleus of the thalamus and the ventral posterior nucleus of the thalamus; injection at the site of (ii) injected endothelin to the caudate nucleus; injection at the site of (iii) injected endothelin to the putamen; injection at the site of (iv) injected endothelin to the globus pallidus; and injection at the site of (v) injected endothelin to a deep part of the putamen.

The injection sites were the same between (i) of Study Experiment 4 and (i) of Study Experiment 5, between (ii) of Study Experiment 4 and (iv) of Study Experiment 5, and between (iii) B: 9.0 mm and L: 8.0 mm of Study Experiment 4 and (v) of Study Experiment 5. Accordingly, the results obtained here indicated that endothelin was injected to a deep part of the putamen; the globus pallidus, the ventral lateral nucleus of the thalamus, and the ventral posterior nucleus of the thalamus in at least one individual of Study Experiment 5.

### (f) Study Experiment 6

In view of the results of Study Experiments 4 and 5, a study was made on injection sites more suitable for administration to the caudate nucleus, the putamen, the globus pallidus, the ventral lateral nucleus of the thalamus, and the ventral posterior nucleus of the thalamus.

Holes of approximately 1 mm in diameter were opened (a total of 4 holes) at positions of (i) B: 2.0 mm and L: 8.0 mm, (ii) B: 5.0 mm and L: 8.0 mm, (iii) B: 9.0 mm and L: 5.0 mm, and (iv) B: 9.0 mm and L: 12.0 mm from Bregma as a base point in the skull of a cynomolgus macaque using a drill. A microsyringe connected to a microinjector was inserted from each hole and indwelled such that the tip of its needle was located at the depth (D) of 19.0 mm (for (i) and (ii) described above), the depth (D) of 13.0 mm (for (iii) described above), or the depth (D) of 15.0 mm (for (iv) described above) from the pachymeninx. Endothelin-1 was injected to a total of 4 sites per individual, which correspond to the respective positions described above, using the microsyringe. Endothelin-1 was injected at a concentration of 2.0 µg/µL, a dose of 30 µL/site, and an injection rate of 1.5 µL/min. The injection was carried out at N = 2. Each injection was performed targeting the following nerve nuclei: (i) the ventral lateral nucleus of the thalamus and the ventral posterior nucleus of the thalamus, (ii) the globus pallidus, (iii) the caudate nucleus, or (iv) the putamen.

One individual of the treated cynomolgus macaques had mRS (Table 1) of 5 on the day following the treatment and manifested serious impairment. Then, the condition worsened, and the individual died of the cessation of respiration. The brain was immediately harvested from the individual. Brain section samples were prepared, and a damaged area was observed by the same TTC staining method as in Study Experiment 5. As a result, it was confirmed that: damaged areas occurred in the target nerve nuclei; and a damaged area also occurred in the internal capsule. Figure 2 shows exemplary stain images of the obtained brain sections. The stain images show the damaged areas in the targeted caudate nucleus, putamen, ventral lateral nucleus of the thalamus, and ventral posterior nucleus of the thalamus and further the damaged area in the internal capsule. This revealed that: use of Bregma as a base point enables an endothelin solution to be successfully injected to a target site; and such injection enables an effect of endothelin to also extend over the internal capsule.

The other treated individual which survived had mRS (Table 1) of 5 on the day following the treatment, manifested serious impairment, and also had an NHPSS (Table 2) score of 49 (composed of motor system: 23 (impaired side/unimpaired side = 16/7), consciousness: 10, and skeletal muscle coordination: 16), and complete paralysis of the forelimb and hindlimb on the impaired side and slight disturbance of consciousness were observed. Then, in the treated individual, the condition having mRS of 3 or more and a NHPSS motor system score of 16 which indicates complete paralysis of the forelimb and hindlimb on the impaired side ("complete paralysis of the forelimb and hindlimb on the impaired side") persisted for almost 3 months after the treatment (Figures 3 and 4). This indicated that long-term motor dysfunction which lasts for at least 3 months can also be induced under the conditions of Study Experiment 6. Although NHPSS is used as a severity evaluation scale of cerebral infarction at an acute stage, the results of the present study experiments demonstrated that persistence of motor dysfunction in this model can be evaluated by using an NHPSS motor system score as an index. Also in the case of using mRS as an index, persistence of motor dysfunction was also able to be evaluated, as with NHPSS, indicating that mRS can be also used in the evaluation of persistence of motor dysfunction in this model.

Damaged areas were found in the nerve nuclei as a territory supplied by the penetrating branch and in the internal capsule, and motor dysfunction was confirmed to persist for 4 weeks or longer, suggesting that the model produced under the conditions of Study Experiment 6 is a model that enables evaluation of cerebral infarction at a subacute to chronic stage (particularly, penetrating branch infarction, or cerebral infarction having brain damage in a penetrating branch territory).

The results of Study Experiments 1 to 6 revealed that: the administration (injection) of endothelin to the basal ganglia and thalamic region of a nonhuman primate generates damaged area in the basal ganglia, the thalamus, and the internal capsule, thereby inducing basal ganglia damage, thalamus damage, and internal capsule damage; and such a nonhuman primate animal model having basal ganglia damage, thalamus damage, and internal capsule damage enables evaluation of cerebral infarction at a subacute to chronic stage (particularly, penetrating branch infarction, or cerebral infarction having brain damage in a penetrating branch territory).

### (Example 2) Production of cerebral infarction model having endothelin-1-induced basal ganglia damage, thalamus damage, and internal capsule damage to be used in AAV administration test into brain

Six individuals of male cynomolgus macaques (3 years of age or older, SNBL) were subjected to anesthesia induction using i.m. of ketamine hydrochloride in an amount of approximately 10 mg/kg body weight. Each cynomolgus macaque under anesthesia was subjected to intratracheal intubation, and anesthesia was maintained by the inhalation of isoflurane from the trachea under artificial breathing or spontaneous breathing. Hair on the head of the cynomolgus macaque under anesthesia was shaved with an electric shaver. Then, the cynomolgus macaque was fixed in a stereotaxic instrument. Then, the head skin of the cynomolgus macaque was incised with attention given to bleeding to identify the bregma.

An endothelin-1 solution was injected to the six individuals of male cynomolgus macaques in accordance with the method described in Study Experiment 6 of Example 1. Specifically, holes of approximately 1 mm in diameter were opened (a total of 4 holes) at positions of (i) B: 2.0 mm and L: 8.0 mm, (ii) B: 5.0 mm and L: 8.0 mm, (iii) B: 9.0 mm and L: 5.0 mm, and (iv) B: 9.0 mm and L: 12.0 mm from Bregma as a base point for injection sites in the skull of each cynomolgus macaque using a drill. A microsyringe connected to a microinjector was inserted from each hole and indwelled such that the tip of its needle was located at the depth (D) of 19.0 mm (for (i) and (ii) described above), the depth (D) of 13.0 mm (for (iii) described above), or the depth (D) of 15.0 mm (for (iv) described above) from the pachymeninx. Endothelin-1 was injected to a total of 4 sites per individual, which correspond to the respective positions described above, using the microsyringe. Endothelin-1 was injected at a concentration of 2.0 µg/µL, a dose of 30 µL/site, and an injection rate of 1.5 µL/min. After the completion of injection, the syringe was left standing for approximately 20 minutes in order to prevent liquid leakage. In this way, cerebral infarction models having basal ganglia damage, thalamus damage, and internal capsule damage were produced.

### (Example 3) Production of expression vector to be used in AAV administration test

An expression vector for the coexpression of mouse NeuroD1 and GFP (AAV-GFAP-Neurod1-IRES-GFP, SEQ ID NO: 1; hereinafter, also referred to as a "NeuroD1 expression vector") and, as a control, an expression vector for the expression of GFP (green fluorescent protein) alone (AAV-GFAP-GFP, SEQ ID NO: 2; hereinafter, also referred to as a "control vector") were produced (commissioned to Takara Bio Inc.) using an AAV9 vector. In the nucleotide sequence of the expression vector AAV-GFAP-Neurod1-IRES-GFP shown in SEQ ID NO: 1, a nucleotide sequence of positions 171 to 1842 corresponds to the nucleotide sequence (SEQ ID NO: 14) of human GFAP promoter; a nucleotide sequence of positions 2355 to 3428 (SEQ ID NO: 5) corresponds to the nucleotide sequence (CDS) of mouse NeuroD1 gene and encodes mouse NeuroD1 protein (SEQ ID NO: 6); and a nucleotide sequence of positions 4033 to 4752 (SEQ ID NO: 3) corresponds to the nucleotide sequence (CDS) of GFP gene and encodes GFP (SEQ ID NO: 4). GFAP in the expression vector was human GFAP promoter, and the mouse NeuroD1 gene (SEQ ID NO: 5) was placed under the control of the human GFAP promoter. Use of the GFAP promoter enables expression of a gene positioned downstream of the promoter in a manner specific for astrocytes.

The NeuroD1 expression vector and the control vector were specifically produced by the following method: the nucleotide sequence of the human GFAP promoter was inserted to a region of CMV promoter of a pAAV-CMV vector (Takara Bio Inc., 6230), and the nucleotide sequence of the mouse NeuroD1 gene, and the nucleotide sequences of IRES and the GFP gene were inserted into a multiple cloning site to produce a pAAV plasmid (pAAV-hGFAP-mNeuroD1-IRES-GFP). Also, the nucleotide sequence of the human GFAP promoter was inserted to a region of CMV promoter of a pAAV-CMV vector (Takara Bio Inc., 6230), and the nucleotide sequence of the GFP gene was inserted into a multiple cloning site to produce a pAAV plasmid (pAAV-hGFAP-GFP). The two types of pAAV plasmids produced, pRC_AAV9 (SEQ ID NO: 12), and pHelper (Takara Bio Inc., 6230) were each prepared in a large amount. Subsequently, 293T cells (ATCC, CRL-3216) were seeded into HYPERFlask (Corning Inc., 10034) and cultured until semiconfluent. The resulting 293T cells were cotransfected with pRC_AAV9, pHelper and the pAAV plasmid (pAAV-hGFAP-mNeuroD1-IRES-GFP or pAAV-hGFAP-GFP) using a transfection reagent PEIpro (PolyPlus-transfection, 115) and further cultured for approximately 72 hours. Triton X-100 (Merck KGaA, 1.08643.1000) was added to the AAV9 vector-producing cell culture, which was then treated at 37°C for 1 hour and centrifuged, followed by the recovery of a supernatant. The recovered supernatant was subjected to affinity purification using POROS CaptureSelect AAV9 Affinity Resin (Thermo Fisher Scientific Inc., A27353) to adsorb AAV9, which was then eluted with 50 mM glycine-HCl (pH 2.7) to recover multiple fractions. The recovered solutions were neutralized using 2 M Tris-HCl (pH 8.0). Subsequently, the fractions were purified by cesium chloride density gradient centrifugation as follows: cesium chloride (Nacalai Tesque, Inc., 07807-11) was added to the recovered AAV9 vector solutions, and the refractive index of the solutions was adjusted to 1.371, followed by ultracentrifugation at 148,500 × g at 21°C for 42 hours. After centrifugation, solutions were sequentially separated from the upper part of the centrifuge tube. The recovered solutions were filtered through a 0.22 µm filter to obtain a NeuroD1 expression vector or a control vector.

### (Example 4) Administration of NeuroD1 expression vector into brain

Six individuals of the cerebral infarction models produced in Example 2 were divided to two groups (N = 3 each) on day 21 after endothelin-1 injection so as not to differ in mRS (see Table 1) and NHPSS (see Table 2) between the groups. The respective groups were an AAV-NeuroD1 group which was to be given the NeuroD1 expression vector and a control group which was to be given the control vector.

The cerebral infarction model individuals in each group were subjected to anesthesia induction using i.m. of ketamine hydrochloride at 15 to 50 mg/0.3 to 1.0 mL/head and xylazine (Selactal 2% Injection Solution; Bayer Yakuhin, Ltd., Approval Protocol No: 27 Douyaku 2679) at 1 to 4 mg/0.05 to 0.2 mL/head, and anesthesia was maintained by repetitive administration of the same anesthetics as above. Hair on the head under continuous anesthesia was shaved with an electric shaver. Then, each model individual was fixed in a stereotaxic instrument. Next, the operative field was disinfected, and the head skin was incised with attention given to bleeding to expose the bregma.

In the cerebral infarction model individuals in each group, a microsyringe was indwelled into each of a total of four holes to which endothelin-1 was injected at the time of cerebral infarction model production in Example 2 such that the tip of its needle was located at the same depth (D) as in Example 2. The AAV vector produced in Example 3 was administered thereto. The NeuroD1 expression vector was administered to three individuals, and the control vector was administered to three individuals. The AAV vector was prepared at 1.05 × 10¹² vg (vector genome)/mL with phosphate-buffered saline (PBS), and the AAV vector solution was administered at 30 µL/site and an administration rate of 1.5 µL/min. After the completion of administration, the syringe was left standing for approximately 20 minutes.

### (Example 5) Observation of motor function

Observation based on mRS (Table 1) and NHPSS motor system scoring (Table 2) was carried out as to each individual in the AAV-NeuroD1 group and the control group as treated with the AAV vector in Example 4, before endothelin-1 injection treatment (day 0) and on day 1, 7, 14, 21, 28, 42, 56, 70, and 84 after endothelin-1 injection.

As a result, there is no difference between the AAV-NeuroD1 group and the control group in motor function on day 7 after administration of AAV vector and both of the groups manifested similar motor dysfunction to that before AAV vector administration. However, the motor function was not yet improved in the control group at day 84 after endothelin-1 injection (day 63 after AAV vector administration), whereas the motor function was restored in the AAV-NeuroD1 group from day 56 after endothelin-1 injection (from day 35 after AAV vector administration) and found to be significantly restored at day 84 after endothelin-1 injection (day 63 after AAV vector administration) (Figures 5 and 6).

On the other hand, there is no difference between the control group and the AAV-NeuroD1 group in body weight and feed intake status (Figures 7 and 8). The feed intake status was evaluated by feed intake scoring on a 1-week basis in accordance with the following criteria.
0: no feed intake
1: intake of less than half the feed dosage
2: intake of more than half the feed dosage and less than the entire dosage
3: intake of the entire feed dosage

### (Example 6) Imaging analysis of cynomolgus macaque model of cerebral infarction

The brain of one individual of the cerebral infarction model produced by the method described in Example 2 was analyzed by the following method. In this Example, the individual was treated by i.m. administration of atropine sulfate hydrate (Mitsubishi Tanabe Pharma Corporation, ATROPINE SULFATE Injection 0.5 mg "TANABE") at 0.01 mg/ kg body weight, and then subjected to anesthesia induction using i.m. of ketamine hydrochloride at 10 mg/kg body weight. Then, anesthesia was maintained by the inhalation of isoflurane.

### (a) Tissue image evaluation of endothelin-1 injection region

The brain of the cerebral infarction model was noninvasively imaged on a daily basis using an MRI apparatus (MAGNETOM Trio, 3T, Siemens AG). Imaging was performed by the T2-weighted imaging method and the fluid attenuated inversion recovery (FLAIR) method as widely used in clinical practice. In T2-weighted images, it is known that water is visualized white with a high signal intensity, and inflammation or damaged areas are also visualized with a high signal intensity. Images obtained by the FLAIR method are T2-weighted images in which a signal of water including cerebrospinal fluid is reduced, and thus can visualize damaged areas more clearly than the usual T2-weighted images do.

Daily T2-weighted image of the brain are shown in Figure 9A, and daily FLAIR images of the brain are shown in Figure 9B. Both the T2-weighted image and the FLAIR image visualized endothelin-1 injection regions with a high signal intensity in the cerebral infarction model on day 1 after the induction of brain damage (day 1 after endothelin-1 injection, i.e., the day following endothelin-1 injection), which indicated that damage occurred in the injection regions. In imaging also perfomed on day 7, 21, 41, and 69 after the induction of brain damage, high-signal-intensity regions were detected in both the T2-weighted images and the FLAIR images, but the high-signal-intensity regions were found to be reduced in size on a daily basis (see, L in Figures 9A and 9B). On the other hand, in the non-damaged side, no change was observed in both the T2-weighted images and the FLAIR images (see, R in Figures 9A and 9B). Further, daily change was observed focusing on the lateral ventricles in the T2-weighted images, and the lateral ventricle on the damaged side was found to be reduced in size as compared with that on the non-damaged side on day 1 after endothelin-1 injection (Figure 9A(a)). This was presumably due to change resulting from the compression of the lateral ventricle on the damaged side by angioedema associated with brain damage, as also observed in clinical cerebral infarction. The reduction in the size of the lateral ventricle was mitigated on day 7 or later after endothelin-1 injection, which indicates that edema at an acute stage associated with brain damage was alleviated. On the other hand, it was found in the T2-weighted images on day 21, 41, and 69 after endothelin-1 injection that the lateral ventricle on the damaged side enlarged compared with that on the non-damaged side. This was presumably change that reflected the brain parenchymal atrophy on the damaged side associated with neuronal loss at a damaged area, and was also similar to change in clinical cerebral infarction. These results of imaging analysis indicated that this model is a cerebral infarction model that involves edema at an acute stage after induction of brain damage and further shows daily reduction in the size of the edema region at a cerebral infarction site and brain parenchymal atrophy associated with neuronal loss at a damaged area, as with clinical cerebral infarction.

### (b) Perfusion-weighted imaging evaluation of endothelin-1 injection region

A gadolinium contrast agent (Bracco-Eisai Co., Ltd., ProHance Intravenous Injection 10 mL) was intravenously injected to the cerebral infarction model, and blood flow was imaged on a daily basis by the MRI perfusion-weighted imaging. Daily perfusion-weighted images of the brain are shown in Figure 9C. Endothelin-1 injection regions had a low signal intensity in the cerebral infarction model on day 1 after induction of brain damage, indicating a decreased blood flow. In imaging also performed on day 7, 21, 41, and 69 after induction of brain damage, the endothelin-1 injection regions having the low signal intensity was reduced in size on a daily basis, indicating recovery from the decreased blood flow. On the other hand, no change in blood flow was observed on the non-damaged side. The results of imaging analysis at an acute stage and on a daily basis at the damaged areas indicated that this model manifests ischemia at an acute stage after induction of brain damage and further shows recovery at a subacute stage from decreased blood flow at a cerebral ischemia site by angiogenesis or the like, as with a clinical report.

### (c) Imaging evaluation of white matter nerve fiber

Diffusion tensor imaging (DTI) of the cerebral infarction model were taken, and tensor analysis was conducted. On day 1 after induction of brain damage, a region of interest (ROI) was established with the cerebral crus as a start point and the precentral gyrus and the postcentral gyrus as end points so as to include the internal capsule near the endothelin-1 injection region. Fractional anisotropy (FA) was measured. An FA value of DTI analysis in the ROI is considered to mainly reflect a corticospinal tract that passes through white matter.

Daily DTI of the brain are shown in Figure 9D. As a result of measurement, an FA value on the damaged side was low (Figure 9D, arrow), indicating that nerve fibers of the corticospinal tract on the damaged side were ruptured and injured. Decrease in FA value was observed in all the images taken on day 1, 7, 21, 41, and 69 after induction of brain damage, indicating that the rupture of nerve fibers was maintained from an acute stage up to a chronic stage. On the other hand, the entire corticospinal tract was visualized on the non-damaged side, and no change was observed. This presumably shows rupture of a corticospinal tract in a brain damage region, as also observed in clinical cerebral infarction. This indicated that this model is also useful as a model of cerebral infarction with corticospinal tract injury.

### (Example 7) Astrocyte accumulation and neuronal loss at and around damaged area in brain of cynomolgus macaque model of cerebral infarction

One individual of the cerebral infarction model produced by the method described in Example 2 was euthanized on day 21 after endothelin-1 injection, and the brain was harvested and fixed using 4% paraformaldehyde (FUJIFILM Wako Pure Chemical Corporation, 163-20145). Then, water in the fixed brain tissue was replaced with a sucrose solution (10, 20, and 30%) of sucrose (Nacalai Tesque, Inc., 30404-45) dissolved in PBS (FUJIFILM Wako Pure Chemical Corporation, 045-29795). Then, frozen brain sections (10 µm thick) were prepared. The brain sections were immunohistochemically stained using an anti-GFAP antibody (Abcam plc., ab53554) against the astrocyte marker GFAP and an anti-NeuN antibody (Sigma-Aldrich Co., LLC, MAB377) against the neuron marker NeuN.

As a result, it was found that: GFAP-positive cells accumulated at and around a damaged area; and the number of NeuN-positive cells was decreased in the damaged area as compared with the counterpart on the non-damaged side. This result indicates that at and around a damaged area, astrocytes accumulate, while the number of neurons is decreased.

### (Example 8) Astrocyte accumulation and neuronal loss at and around cerebral infarction site of human cerebral infarction patient

A formalin-fixed human brain sample (Life Net Health) derived from a basal ganglia infarction patient 10 months after the onset of cerebral infarction was treated with a solution of sucrose dissolved in PBS in the same manner as in Example 7 to replace water in the tissue therewith. Then, frozen brain sections (10 µm thick) were prepared. The brain sections were immunohistochemically stained using an anti-GFAP antibody (Cell Signaling Technology, Inc., 12389S) and an anti-NeuN antibody (Sigma-Aldrich Co., LLC, MAB377).

As a result, it was found that: GFAP-positive cells accumulated at and around a cerebral infarction site; and the number of NeuN-positive cells was decreased in the infarction site as compared with the counterpart on the non-infarction side. This result also indicates that at and around a damaged area, astrocytes accumulate, while the number of neurons is decreased.

The results of Example 7 and Example 8 indicated that the cerebral infarction model produced by the method described in Example 2 is a cerebral infarction model that shows neuronal loss and astrocyte accumulation, as in human cerebral infarction at a chronic stage.

### (Example 9) Preparation of mRNA-LNP

As two types of NeuroD1 mRNAs encoding human NeuroD1 protein, (i) mRNA that consisted of a nucleotide sequence comprising 5' UTR and 3' UTR provided by TriLink BioTechnologies, Inc., CDS (SEQ ID NO: 7) of human NeuroD1 gene, and a polyA sequence of 120 nucleotides, and had a 5' cap structure being Cap-1 (hereinafter, referred to as "ND1-1"), and (ii) mRNA that consisted of a nucleotide sequence (SEQ ID NO: 13) in which all uridines are replaced by N1-methylpseudouridine in a nucleotide sequence (SEQ ID NO: 11) comprising 5' UTR derived from human α globin gene, 3' UTR derived from human α globin gene, CDS (SEQ ID NO: 10) of human NeuroD1 gene and a polyA sequence of 79 nucleotides, and had a 5' cap structure being Cap-1 (hereinafter, referred to as "ND1-2") were produced (commissioned to TriLink BioTechnologies, Inc.). In the nucleotide sequences as set forth by SEQ ID NOs: 11 and 13, nucleotide positions 1 to 3 correspond to a 5'-terminal sequence suitable for a capping method using CleanCap^{(R)} Reagent AG (TriLink BioTechnologies, Inc.); positions 4 to 43 correspond to 5' UTR; positions 44 to 1114 correspond to CDS (SEQ ID NO: 10) of human NeuroD1 gene; positions 1115 to 1120 correspond to consecutive two stop codons; positions 1121 to 1231 correspond to 3' UTR; and positions 1232 to 1310 correspond to a polyA sequence. As a control, eGFP mRNA (TriLink BioTechnologies, Inc., L-7601) was used.

Each of the mRNAs of (i) and (ii) was mixed with a lipid nanoparticle (L1 or L6) having the composition indicated in Table 3 to prepare mRNA encapsulated into lipid nanoparticles (hereinafter, referred to as "mRNA-LNP").

**Table 3**

| | | L1 | L6 |
|---|---|---|---|
| Lipid composition (mol %) | Cationic lipid (Compound 1 (Ex1)) | 50 | 40 |
| | DSPC | 10 | 12 |
| | Cholesterol | 38.5 | 46.5 |
| | DMG-PEG2000 | 1.5 | 1.5 |
| Buffer | | 10 mM citrate buffer (pH 4) | 10 mM citrate buffer (pH 6) |

| | | | |
|---|---|---|---|
| DSPC: distearoylphosphatidylcholine DMG: dimyristoyl glycerol PEG2000: polyethylene glycol 2000 | | | |

The preparation of mRNA-LNP was specifically carried out by the following method. A cationic lipid Compound 1 (Ex1; its production method is shown in Example 15), DSPC (NOF Corporation, COATSOME^{(R)} MC-8080), cholesterol (Nippon Fine Chemical Co., Ltd., Cholesterol HP), DMG-PEG2000 (NOF Corporation, SUNBRIGHT^{(R)} GM-020) were dissolved at N/P ratio of 6 in ethanol to obtain an oil phase. A 10 mM citrate buffer (pH 4 or 6) containing each mRNA described above was used as an aqueous phase, and the oil phase was added thereto such that the volume ratio between the aqueous phase and the oil phase was aqueous phase:oil phase = 3:1. These phases were mixed using a microfluidic device (NanoAssemblr^{(R)}, Precision NanoSystems Inc.), and the mixture was diluted with PBS to obtain a mRNA-LNP dispersion. Ethanol was removed by the dialysis or ultrafiltration of this dispersion. Subsequently, the dispersion was concentrated by ultrafiltration and then mRNA-LNP adjusted to an arbitrary concentration was obtained. The "N/P ratio" is a value obtained by dividing the number of moles of an amino group (N) in the cationic lipid by the number of moles of phosphoric acid (P) in mRNA in mRNA-LNP.

ND1-1 encapsulated into the lipid nanoparticle L1, prepared as mRNA-LNP was designated as ND1-1-L1, and ND1-2 encapsulated into the lipid nanoparticle L6 was designated as ND1-2-L6.

Likewise, eGFP mRNA encapsulated into the lipid nanoparticle L1 was prepared as mRNA-LNP. This was designated as eGFP mRNA-L1.

ND1-2 or eGFP mRNA was each mixed with a lipid nanoparticle (L7) having the composition indicated in Table 4 to prepare mRNA-LNP in a similar way to that described above. The cationic lipid contained in L7 is Compound 2 (Ex2; its production method is shown in Example 16). ND1-2 or eGFP mRNA encapsulated into the lipid nanoparticle L7, prepared as mRNA-LNP was designated as ND1-2-L7 or eGFP mRNA-L7, respectively.

**Table 4**

| | | L7 |
|---|---|---|
| Lipid composition (mol %) | Cationic lipid (Compound 2 (Ex2)) | 50 |
| | DSPC | 10 |
| | Cholesterol | 38.5 |
| | DMG-PEG2000 | 1.5 |
| Buffer | | 10 mM citrate buffer (pH 4) |

### (Example 10) In vitro conversion of rat primary astrocyte into neuron through NeuroD1 mRNA 1. Addition of mRNA-LNP to rat primary astrocyte

Rat primary astrocytes were obtained in accordance with the literature of Lynette C. Foo. et al. (Purification of Rat and Mouse Astrocytes by Immunopanning: Cold Spring Harbor Protocols, vol. 5, pp. 421-432, 2013) and used. The recovery of the cells was performed under 5% CO₂. The rat used was a newborn Wister rat (CLEA Japan, Inc., P1-P10). The obtained rat primary astrocytes were seeded at 10,000 to 40,000 cells/well to a 96-well plate (Corning Inc., 356640) coated with poly-D-lysine, and cultured overnight at 37°C under 5% CO₂. For the culture, DMEM/F-12 medium (Thermo Fisher Scientific Inc., 11320-033) containing 2% B-27^{(R)} supplement (Thermo Fisher Scientific Inc., A1895601), 10% fetal bovine serum (FBS) (Cytiva, SH30084.03), and 1% penicillin-streptomycin (PS) (Thermo Fisher Scientific Inc., 15070063) was used at 200 µL/well. In Example 10, this DMEM/F12 medium containing B-27^{(R)} supplement, FBS, and PS was also used as a culture medium in the description below. On the next day, mRNA-LNP (ND1-1-L1 or ND1-2-L6) diluted with PBS and a culture medium was added at a final concentration of 0.5 µg/mL to a well, and the cells were cultured for 24 hours. PBS and a culture medium were added to control wells.

### 2. Examination of NeuroD1 protein expression

24 hours after mRNA-LNP addition described above, the culture medium was removed, and the cells were fixed with 4% paraformaldehyde. Then, the cells were washed with PBS and immunocytochemically stained using an anti-NeuroD1 antibody (Santa Cruz Biotechnology, Inc., sc46684) to detect the expression of the NeuroD1 protein. As a result, it was found that the number of NeuroD1 protein-positive cells was markedly increased in the wells supplemented with ND1-1-L1 or ND1-2-L6, as compared with the wells supplemented with PBS (control). This result indicates that mRNA-LNP was taken up into the astrocytes and translated into the NeuroD1 protein in the astrocytes.

### 3. Examination of neuron marker expression

24 hours after mRNA-LNP addition described above, the culture medium was removed and changed to a transdifferentiation medium, and the medium was replaced with a fresh one every 2 to 3 days. The transdifferentiation medium was DMEM/F 12 medium containing 2% B-27^{(R)} supplement, 1% GlutaMAX^{(™)} supplement (Thermo Fisher Scientific Inc., 35050-061), 1% PS, and a 0.02% BDNF (brain derived neurotrophic factor) solution, and this medium was added at 200 µL/well. The BDNF solution was prepared by dissolving a BDNF powder (PeproTech, Inc., 450-02) at 100 µg/mL in pure water. On day 6 to 7 after mRNA-LNP addition, the transdifferentiation medium was removed, and the cells were fixed with 4% paraformaldehyde. Then, the cells were washed with PBS and immunocytochemically stained using an anti-DCX antibody (Cell Signaling Technology, Inc., 4604S) in order to examine the expression of an immature neuron marker doublecortin (DCX).

It was found that the number of DCX-positive cells was markedly increased in the wells supplemented with ND1-1-L1 or ND1-2-L6, as compared with the wells supplemented with PBS (control). This result indicates that NeuroD1 mRNA converted rat primary astrocytes into neurons.

### (Example 11) Administration of NeuroD1 mRNA-LNP into brain of cynomolgus macaque model of cerebral infarction

Four individuals of the cerebral infarction models produced by the method described in Example 2 were divided to two groups (control group: N = 1, drug administration group: N = 3) on day 21 after endothelin-1 injection (at a chronic stage) so as not to differ in mRS (see Table 1) between the groups.

The cerebral infarction model individuals in each group were subjected to anesthesia induction using i.m. of ketamine hydrochloride in an amount of approximately 10 mg/kg body weight, and anesthesia was maintained by the inhalation of isoflurane. Hair on the head under continuous anesthesia was shaved with an electric shaver. Then, each model individual was fixed in a stereotaxic instrument. Next, the operative field was disinfected, and the head skin was incised with attention given to bleeding to expose the bregma.

In the cerebral infarction model individuals in each group, a microsyringe was indwelled into each of a total of four holes to which endothelin-1 was injected at the time of cerebral infarction model production by the method described in Example 2 such that the tip of its needle was located at the same depth (D) as in Example 2. The microsyringe was used for the administration of mRNA-LNP. ND1-1-L1 was administered as mRNA-LNP to the three individuals of the drug administration group (NeuroD1 mRNA-LNP group), and eGFP mRNA-L1 was administered to the one individual of the control group on day 21 after endothelin-1 injection (at a chronic stage). The mRNA-LNP was prepared at 0.3 mg/mL with PBS and administered at 30 µL/site and an administration rate of 1.5 µL/min. After the completion of administration, the syringe was left standing for approximately 20 minutes.

### (Example 12) Effect of NeuroD1 mRNA

### (a) Observation of motor function

Observation based on mRS (Table 1) was carried out as to each individual in the NeuroD1 mRNA-LNP group and the control group as treated with the mRNA-LNP in Example 11, before endothelin-1 injection treatment (day 0) and on day 1, 7, 14, 21, 22, 28, 42, 56, 70, and 84 after endothelin-1 injection. Behavioral evaluation was carried out every 2 weeks on day 28 or later after endothelin-1 injection as mentioned above. Nevertheless, as the behavioral evaluation of the NeuroD1 mRNA-LNP group on day 56 after endothelin-1 injection (day 35 after mRNA-LNP administration), each individual was evaluated on day 57, 58, or 59 after endothelin-1 injection (day 36, 37, or 38 after mRNA-LNP administration). As the behavioral evaluation of the control group on day 56 after endothelin-1 injection (day 35 after mRNA-LNP administration), the one individual of the control group was evaluated on day 58 after endothelin-1 injection (day 37 after mRNA-LNP administration). These results were used as the results on day 56 after endothelin-1 injection (day 35 after mRNA-LNP administration) in this Example.

As a result, there is no difference between the NeuroD1 mRNA-LNP group and the control group in motor function on day 1 after mRNA-LNP administration and both of the groups manifested similar motor dysfunction to that before mRNA-LNP administration. However, the motor function was not yet improved in the control group at day 84 after endothelin-1 injection (day 63 after mRNA-LNP administration), whereas the motor function was restored in the NeuroD1 mRNA-LNP group from day 56 after endothelin-1 injection (from day 35 after mRNA-LNP administration) and found to be significantly restored at day 84 after endothelin-1 injection (day 63 after mRNA-LNP administration) (Figure 10). This demonstrated that not only the administration of NeuroD1 using the AAV vector known to cause sustained protein expression (Example 5) but the administration of NeuroD1 using mRNA known to cause transient protein expression provides recovery from motor dysfunction associated with brain damage.

### (b) DTI analysis of FA value

For each individual of the NeuroD1 mRNA-LNP group and the control group treated with mRNA-LNP in Example 11, imaging of the brain was performed by MRI on day 114 to 115 after endothelin-1 injection. ROI was established in the same manner as in Example 6(c), and DTI analysis was conducted. DTT in which the continuous running of nerve fibers was three-dimensionally imaged was obtained on the basis of the DTI analysis.

In the control group, an FA value on the damaged side (the left side of the brain) was lower than that on the non-dagamed side (the right side of the brain) in DTI analysis, thereby indicating rupture of nerve fibers in DTT (Figure 11A). On the other hand, in the NeuroD1 mRNA-LNP group, the FA value on the damaged side was restored, and the nerve fibers were restored in DTT (Figure 11B, arrow). Figure 11C shows results of quantifying FA values by DTI analysis in the control group and the NeuroD1 mRNA-LNP group, as the ratio (relative value) of the FA value on the damaged side to that on the non-damaged side. This result indicates that an injured corticospinal tract was restored by the administration of NeuroD1.

### (c) Analysis of volume of lateral ventricle

For each individual of the NeuroD1 mRNA-LNP group and the control group treated with mRNA-LNP in Example 11, imaging of the brain was performed by MRI on day 114 to 115 after endothelin-1 injection.

By imaging evaluation analysis using T2-weighted images, it was found that in the control group, a volume of the lateral ventricle on the damaged side (the left side of the brain) was increased as compared with that on the non-damaged side (the right side of the brain) one (Figure 12A). By contrast, it was found that in the NeuroD1 mRNA-LNP group, the increase in the volume of the lateral ventricle on the damaged side was suppressed (Figure 12B, arrow). Figure 12C shows results of quantifying a volume of the lateral ventricle in the control group and the NeuroD1 mRNA-LNP group, as the ratio (relative value) of the volume of the lateral ventricle on the damaged side to that on the non-damaged side based on the T1-weighted images. In the T1-weighted images, cerebrospinal fluid contained in the lateral ventricle is detected with a low signal intensity, and thus the analysis of T1-weighted images enables the volume of the lateral ventricle to be calculated. The increase in the volume of the lateral ventricle is considered to reflect parenchymal atrophy of the brain. Therefore, the suppressed increase in the volume of the lateral ventricle in the NeuroD1 mRNA-LNP group indicated the suppression of parenchymal atrophy of the brain or recovery from injury in a parenchymal tissue of the brain by the administration of NeuroD1.

### (d) Expression analysis of dopamine transporter

The present cerebral infarction model has basal ganglia damage, and thus it is considered that the expression of dopamine transporter is decreased in the corpus striatum of the model. Thus, the following experiments were performed.

The individuals in the NeuroD1 mRNA-LNP group and the control group as treated with the mRNA-LNP in Example 11 were euthanized on day 118 to 122 after endothelin-1 injection, and the brains were harvested and fixed using 4% paraformaldehyde. Then, water in the fixed brain tissue was replaced with a sucrose solution (10, 20, and 30%) of sucrose dissolved in PBS, and frozen brain sections (10 µm thick) were prepared. The brain sections were subsequently immunohistochemically stained for the dopamine transporter. In the immunohistochemical staining, the brain section was first immersed in 10 mM citrate buffer and then heated at 98°C for 5 minites, for antigen activation. The citrate buffer was prepared by dissolving citric acid (Nacalai Tesque, Inc., 09108-95) in pure water, and adjusted to pH 6.2 with aqueous sodium hydroxide solution (FUJIFILM Wako Pure Chemical Corporation, 192-02175). After heating, the immunohistochemical staining with an anti-dopamine transporter (DAT) antibody (Abcam plc., ab5990) was carried out.

As a result, in the control group, the staining intensity of the dopamine transporter was distinctly decreased in the corpus striatum on the damaged side (on the left side of the brain) as compared with that on the non-damaged side (on the right side of the brain) (Figure 13A). By contrast, in the NeuroD1 mRNA-LNP group, the corpus striatum on the damaged side did not show a distinct decrease of the staining intensity of the dopamine transporter, which was seen in the control group (Figure 13B). This result indicates that the decreased expression of dopamine transporter caused by the damage was reversed by the administration of NeuroD1. Further, the recovery of the staining intensity of dopamine transporter is considered to reflect the recovery of dopamine nerve tract that is projected to the corpus striatum. Figure 13C shows the expression intensity of dopamine transporter in the control group and the NeuroD1 mRNA-LNP group, as the ratio (relative value) of the dopamine transporter staining intensity on the damaged side to that on the non-damaged side. From the results of the expression analysis of dopamine transporter, it was revealed that the present model is useful as a cerebral infarction model associated with a decreased expression of dopamine transporter.

From Examples 6 and 7 and this Example, it was revealed that: an animal model of cerebral infarction having basal ganglia damage, thalamus damage, and internal capsule damage which is produced by the method of the present invention can be an animal model of cerebral infarction that (1) manifests edema and ischemia at an acute stage, (2) exhibits reduction in the size of the edema region, enlargement of the lateral ventricle, and restoration of blood flow on a daily basis, (3) manifests astrocyte accumulation and neuronal loss at and around a damaged area at a chronic stage, and (4) has injury in a corticospinal tract at an acute to chronic stage. These pathological conditions were similar to those of cerebral infarction clinically reported. Thus, this model was found to be useful as a model of cerebral infarction (at an acute stage or at a subacute to chronic stage, particularly, at a chronic stage).

In the above-mentioned Examples, the administration of AAV-NeuroD1 or NeuroD1 mRNA to a damaged area was shown to provide the suppression of or recovery from parenchymal atrophy of the brain and the recovery from injury in a corticospinal tract in the internal capsule, and the recovery of dopamine nerve tract in the corpus striatum, which indicates that cells, such as astrocytes, at or around the damaged area were converted to neurons in a NeuroD1 protein-dependent manner, thereby restoring a brain function and resulting in recovery from motor dysfunction associated with brain damage. In Example 10, ND1-2-L6 was also shown to convert astrocytes into neurons, as in ND1-1-L1. It is therefore expected that the administration of ND1-2-L6 exhibits similar effects of NeuroD1, as in ND1-1-L1.

The results of Examples 1 to 12 described above indicated that a test substance can be evaluated or screened for therapeutic effect on cerebral infarction, particularly, penetrating branch infarction, cerebral infarction having brain damage in a penetrating branch territory, and cerebral infarction at a subacute to chronic stage, by using an animal model of cerebral infarction having basal ganglia damage, thalamus damage, and internal capsule damage which is produced by the method of the present invention. These results further demonstrated that the administration of NeuroD1 (1) exhibits a therapeutic effect on cerebral infarction (penetrating branch infarction, cerebral infarction having brain damage in a penetrating branch territory, and cerebral infarction at a subacute to chronic stage), and (2) also exhibits a therapeutic effect on cerebral infarction involving motor dysfunction with high severity.

### (Example 13) In vitro conversion of rat primary astrocyte into neuron with NeuroD1 mRNA-LNP (ND1-2-L7)

It was examined whether rat primary astrocytes are converted into neuron by ND1-2-L7, as with ND1-1-L1 and ND1-2-L6 in Example 10.

### 1. Addition of mRNA-LNP (ND1-2-L7) to rat primary astrocyte and differentiation induction

According to the method described in Section "1. Addition of mRNA-LNP to rat primary astrocyte" of Example 10, rat primary astrocytes were obtained and cultured overnight. On the next day, mRNA-LNP (ND1-2-L7) diluted with a culture medium was added at a final concentration of 0.5 µg/mL to the cultured rat primary astrocytes in a well, and the cells were cultured for 24 hours. A culture medium was added to a well of the control. DMEM/F12 medium containing B-27^{(R)} supplement, FBS (Cytiva, SH30070.03), and PS as described in "1. Addition of mRNA-LNP to rat primary astrocyte" of Example 10 was used as the culture medium.

24 hours after the mRNA-LNP addition, the culture medium was removed and changed to a transdifferentiation medium, and the medium was replaced with a fresh one every 2 to 3 days. The transdifferentiation medium used is the transdifferentiation medium as described in Section "3. Examination of neuron marker expression" of Example 10, and this medium was added at 200 µL/well.

### 2. Examination of expressions of NeuroD1 protein and DCX protein with immunoblotting

Before mRNA-LNP addition described above (day 0) and at 8 hours, day 1, day 2, day 4 and day 6 after the mRNA-LNP addition, the culture medium was removed, and the cells were washed with PBS and lysed with RIPA Buffer (Sigma-Aldrich Co., LLC, R0278-50ML). The cell lysis at day 1 after the mRNA-LNP addition was performed prior to the replacement of culture medium at 24 hours after the mRNA-LNP addition. After protein quantification with the Lowry method, 5 µg of each protein was electrophoresed, and the expression of NeuroD1 protein was detected through immunoblotting with an anti-NeuroD1 antibody (Santa Cruz Biotechnology, Inc., sc46684). Further, the expression of DCX protein was detected through immunoblotting with an anti-DCX antibody (Cell Signaling Technology, Inc., 4604S).

As a result, the maximum expression level of the NeuroD1 protein was shown at 8 hours after the mRNA-LNP addition, and the expression was found to decrease at day 1 or later after the mRNA-LNP addition. This result indicates that mRNA-LNP was taken up into the rat primary astrocytes and the NeuroD1 protein was transiently expressed in the rat primary astrocytes. The expression of the DCX protein was found to increase from day 1 after the mRNA-LNP addition and to still increase even at day 6 after the mRNA-LNP addition. These results indicate that not only a LNP containing Compound 1 (Ex 1) as a cationic lipid (Example 10) but also a LNP containing Compound 2 (Ex 2) as a cationic lipid can be used to deliver NeuroD1 mRNA to rat primary astrocytes; a NeuroD1 protein was expressed from the NeuroD1 mRNA in the rat primary astrocytes to realize the function of the protein, thereby converting the rat primary astrocytes into neurons with the NeuroD1 mRNA.

### (Example 14) Administration of NeuroD1 mRNA-LNP (ND1-2-L7) into brain of cynomolgus macaque model of cerebral infarction and observation of motor function

ND1-2-L7 was evaluated in the cynomolgus macaque model of cerebral infarction, as described in Examples 11 and 12. In this Example, the control group of N = 2 and the drug administration group of N = 2 were used; and as mRNA-LNP, ND1-2-L7 was administered to two individuals of the drug administration group ("NeuroD1 mRNA-LNP (ND1-2-L7) group") and eGFP mRNA-L7 was administered to two individuals of the control group, on day 21 after endothelin-1 injection (at a chronic stage).

Observation based on mRS (Table 1) was carried out as to each individual in the NeuroD1 mRNA-LNP (ND1-2-L7) group and the control group as treated with the mRNA-LNP, before endothelin-1 injection treatment (day 0) and on day 1, 7, 14, 21, 22, 28, 42, 56, 70, and 84 after endothelin-1 injection. Behavioral evaluation was carried out every 2 weeks on day 28 or later after endothelin-1 injection as in Example 12. Nevertheless, as the behavioral evaluation of the control group on day 70 after endothelin-1 injection (day 49 after mRNA-LNP administration), each individual of the control group was evaluated on day 69 or 70 after endothelin-1 injection (day 48 or 49 after mRNA-LNP administration). This result was used as the results on day 70 after endothelin-1 injection (day 49 after mRNA-LNP administration) in this Example.

As a result, there is no difference between the NeuroD1 mRNA-LNP (ND1-2-L7) group and the control group in motor function on day 1 after mRNA-LNP administration and both of the groups manifested similar motor dysfunction to that before mRNA-LNP administration. However, the motor function was not yet improved in the control group at day 84 after endothelin-1 injection (day 63 after mRNA-LNP administration), whereas the motor function was restored in the NeuroD1 mRNA-LNP (ND1-2-L7) group from day 56 after endothelin-1 injection (from day 35 after mRNA-LNP administration) and found to be significantly restored at day 84 after endothelin-1 injection (day 63 after mRNA-LNP administration) (Figure 14). This demonstrated that not only ND1-1-L1 evaluated in Examples 11 and 12 but ND1-2-L7 provides recovery from motor dysfunction associated with brain damage, by NeuroD1 mRNA-LNP administration.

mRNA contained in ND1-1-L1 and mRNA contained in ND1-2-L7 differ from each other in e.g., the sequences of UTRs, the length of polyA sequence, and the presence or absence of modified nucleosides. Further, ND1-1-L1 and ND1-2-L7 each comprise mRNA encapsulated into lipid nanoparticles containing a different cationic lipid. The results of Examples 11 and 12 and this Example indicated that even if a different sequence or the like of mRNA and different components of lipid nanoparticle are used, the administration of NeuroD1 mRNA-LNP provides recovery from motor dysfunction associated with brain damage, as long as the NeuroD1 mRNA-LNP allows human NeuroD1protein to be expressed in cells of interest.

### (Example 15) Production of cationic lipid Compound 1 (Ex1)

Under nitrogen atmosphere, N,N-(dimethylamino)pyridine (61.9 g) and 1-(3-dimethylaminopropyl)-3-ethylcarbodiimide hydrochloride (97.2 g) were added to a mixture of 8-bromooctanoic acid (75.5 g) and 9-heptadecanol (86.8 g) in N,N-dimethylformamide (755 mL) under ice cooling. Then, the mixture was stirred at room temperature for 28 hours. The reaction mixture was added to ice water, and subjected to extraction with toluene. The aqueous layer was subjected to extraction with toluene, and the combined organic layer was washed with aqueous saturated sodium chloride solution. The washed solution was subjected to extraction with toluene and ethyl acetate, and the combined organic layer was dried over anhydrous magnesium sulfate. The organic layer was concentrated under reduced pressure. Then, hexane was added, and the mixture was concentrated again under reduced pressure. The obtained residue was purified by silica gel column chromatography (hexane/ethyl acetate) to obtain heptadecan-9-yl 8-chlorooctanoate (80.3 g; hereinafter, also referred to as "Intermediate 1" (or "PEx1")) as an oily product. Table 5 shows the chemical structural formula of Intermediate 1 (PEx1).

Under nitrogen atmosphere, potassium carbonate (1.7 g) and potassium iodide (51 mg) were added to a mixture of 2-(4-amino-1-piperidinyl)ethanol dihydrochloride (672 mg), heptadecan-9-yl 8-chlorooctanoate (3.0 g), and N,N-dimethylacetamide (6.7 mL). After stirring at an internal temperature of 100°C for 24 hours, the reaction mixture was allowed to cool at room temperature, and the insoluble matter was removed by filtration and washed with toluene. Water was added to the filtrate, and the mixture was subjected to extraction of organic layer. The aqueous layer was subjected to extraction with toluene and ethyl acetate, and the combined organic layer was washed with aqueous saturated sodium chloride solution. The aqueous layer was subjected to extraction with ethyl acetate, and the combined organic layer was dried over anhydrous sodium sulfate. The organic layer was concentrated under reduced pressure, and the obtained residue was purified by silica gel column chromatography (hexane/ethyl acetate) and silica gel column chromatography (chloroform/methanol). The obtained crude purified product was purified by silica gel column chromatography (basic silica gel, hexane/ethyl acetate) to obtain di(heptadecan-9-yl) 8,8'-{[1-(2-hydroxyethyl)piperidin-4-yl]azanediyl}di(octanoate) (540 mg; hereinafter, also referred to as "Compound 1" (or "Ex1")) as an oily product. Table 5 shows the chemical structural formula of Compound 1 (Ex1).

**Table 5**

| Compound name | Chemical structural formula |
|---|---|
| Intermediate 1 (PEx1) | 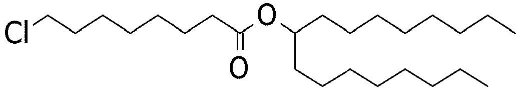 |
| Compound 1 (Ex1) | 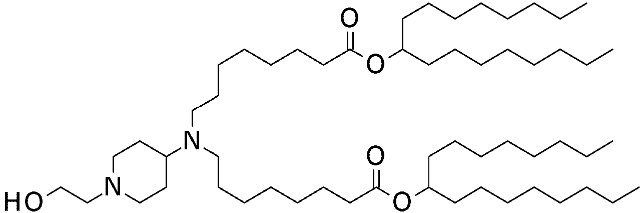 |

Intermediate 1 (PEx1) and Compound 1 (Ex1) were subjected to electrospray ionization mass spectrometry (ESI-MS) using a positive high voltage for ionization. The obtained mass-to-charge ratios (m/z) were as follows.
Intermediate 1 (PEx1) ESI+ m/z: 440
Compound 1 (Ex1) ESI+ m/z: 906

The NMR spectroscopic analysis of Compound 1 (Ex1) was conducted. The result is shown below (representative signal values of ¹H-NMR spectra are shown; t: triplet, quin: quintet, td: triple doublet, m: multiplet, brd: broad doublet).
NMR (500 MHz, CDCl₃): δ:0.88 (t, J = 7.00Hz, 12H), 1.23-1.42 (m, 66H), 1.45-1.74 (m, 14H), 2.04 (td, J = 11.80, 1.98Hz, 2H), 2.27 (t, J = 7.57Hz, 4H), 2.36-2.52 (m, 7H), 2.94 (brd, J = 11.47Hz, 2H), 3.58 (t, J = 5.43Hz, 2H), 4.86 (quin, J = 6.23Hz, 2H)

### (Example 16) Production of cationic lipid Compound 2 (Ex2)

To a mixture of diethyl 7,7'-azanediyldi(heptanoate) (10.7 g) and dichloromethane (107 mL), 1,1'-carbonyldiimidazole (7.35 g) was added at room temperature, and the mixture was and stirred at room temperature for 16 hours. The reaction mixture was concentrated under reduced pressure, and the obtained residue was purified by silica gel column chromatography (hexane/ethyl acetate) to obtain diethyl 7,7'-[(1H-imidazole-1-carbonyl)azanediyl]di(heptanoate) (13.5 g; hereinafter, also referred to as "Intermediate 2a" (or "PEx2a")) as an oily product. Table 6 shows the chemical structural formula of Intermediate 2a (PEx2a).

To a mixture of diethyl 7,7'-[(1H-imidazole-1-carbonyl)azanediyl]di(heptanoate) (3 g) and acetonitrile (30 mL), methyl iodide (2.2 mL) was added at room temperature, and the mixture was stirred at room temperature for two hours. Methyl iodide (2.2 mL) was added to the reaction mixture at room temperature, and the mixture was stirred at room temperature for 16 hours. The reaction mixture was concentrated under reduced pressure, and thus 1-[bis(7-ethoxy-7-oxoheptyl)carbamoyl]-3-methyl-1H-imidazol-3-ium iodide (4.2 g; hereinafter, also referred to as "Intermediate 2b/I" (or "PEx2b/I")) as an oily product. Table 6 shows the chemical structural formula of Intermediate 2b/I (PEx2b/I).

To a mixture of 1-[bis(7-ethoxy-7-oxoheptyl)carbamoyl]-3-methyl-1H-imidazol-3-ium iodide (4.2 g) and N,N-dimethylformamide (10 mL), a mixture of 2-(dimethylamino)-1-(4-hydroxypiperidin-1-yl)ethan-1-one (1.45 g) and tetrahydrofuran (10 mL) was added at room temperature, and next sodium hydride (60% in oil, 310 mg) was added at room temperature. The mixture was stirred at room temperature for three hours. To the reaction mixture, 1M hydrochloric acid was added to adjust the pH to around 7. After diluting with ethyl acetate, water was added, and the organic layer was separated. The aqueous layer was subjected to extraction with ethyl acetate. The combined organic layer was washed with saturated brine/water (1/1), dried over anhydrous sodium sulfate, and then concentrated under reduced pressure. The obtained residue was purified by amino silica gel column chromatography (hexane/ethyl acetate) to obtain diethyl 7,7'-[({[1-(N,N-dimethylglycyl)piperidin-4-yl]oxy}carbonyl)azanediyl]di(heptanoate) (1.93 g; hereinafter, also referred to as "Intermediate 2c" (or "PEx2c")) as an oily substance. Table 6 shows the chemical structural formula of Intermediate 2c (PEx2c).

To a mixture of diethyl 7,7'-[({[1-(N,N-dimethylglycyl)piperidin-4-yl]oxy}carbonyl)azanediyl]di(heptanoate) (1.13 g), tetrahydrofuran (17 mL) , and ethanol (17 mL), 1 M aqueous sodium hydroxide solution (4.17 mL) was added at room temperature, and the mixture was stirred at room temperature for 72 hours. The reaction mixture was concentrated under reduced pressure, and concentrated after adding ethanol (100 mL), and thus sodium 7,7'-[({[1-(N,N-dimethylglycyl)piperidin-4-yl]oxy}carbonyl)azanediyl]di(heptanoate) (1.21 g; hereinafter, also referred to as "Intermediate 2d/Na" (or "PEx2d/Na")) was obtained as a solid. Table 6 shows the chemical structural formula of Intermediate 2d/Na (PEx2d/Na).

To a mixture of sodium 7,7'-[({[1-(N,N-dimethylglycyl)piperidin-4-yl]oxy}carbonyl)azanediyl]di(heptanoate) (200 mg) and dichloromethane (3 mL), 2-nonylundecan-1-ol (270 mg), 1-[bis(dimethylamino)methylene]-1H-1,2,3-triazolo[4,5-b]pyridinium 3-oxidehexafluorophosphate (430 mg), N,N-diisopropylethylamine (0.4 mL) and N,N-dimethylaminopyridine (10 mg) were added at room temperature, and the mixture was stirred at room temperature for 16 hours. Saturated sodium bicarbonate aqueous solution was added to the reaction mixture, and the mixture was stirred at room temperature for 10 minutes. After diluting with dichloromethane, water was added, and extraction operation was conducted using phase separator. The organic layer was dried over anhydrous sodium sulfate, and then concentrated under reduced pressure. The obtained residue was purified by silica gel column chromatography (chloroform/methanol), and then the crude purified product was purified by amino silica gel column chromatography (hexane/ethyl acetate) to obtain bis(2-nonylundecyl) 7,7'-[({[1-(N,N-dimethylglycyl)piperidin-4-yl]oxy}carbonyl)azanediyl]di(heptanoate) (197 mg; hereinafter, referred to as "Compound 2" (or "Ex2")) as an oily substance. Table 6 shows the chemical structural formula of Compound 2 (Ex2).

**Table 6**

| Compound name | Chemical structural formula |
|---|---|
| Intermediate 2a (PEx2a) | 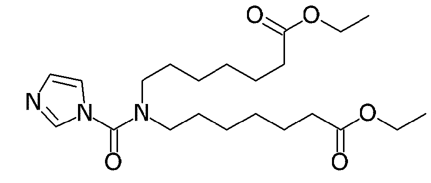 |
| Intermediate 2b/I (PEx2b/I) | 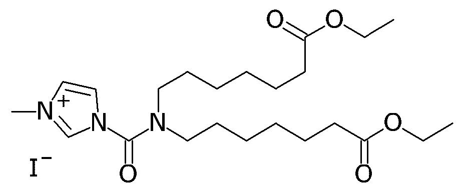 |
| Intermediate 2c (PEx2c) | 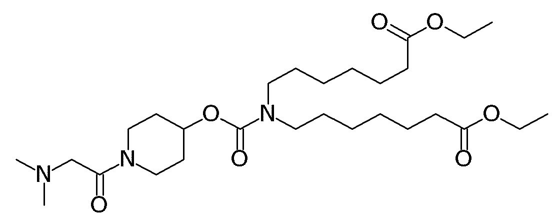 |
| Intermediate 2d/Na (PEx2d/Na) | 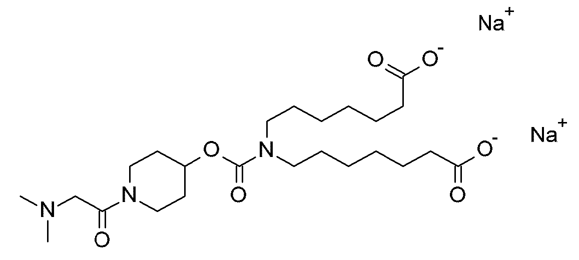 |
| Compound 2 (Ex2) | 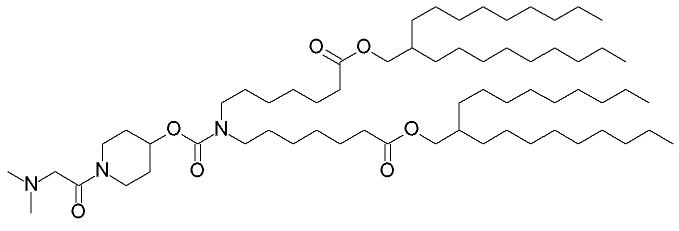 |

Intermediate 2a (PEx2a) Intermediate 2b/I (PEx2b/I), Intermediate 2c (PEx2c), Intermediate 2d/Na (PEx2d/Na) and Compound 2 (Ex2) were subjected to electrospray ionization mass spectrometry (ESI-MS) using a positive high voltage for ionization. The obtained mass-to-charge ratios (m/z) were as follows.
Intermediate 2a (PEx2a) ESI+ m/z: 424.5
Intermediate 2b/I (PEx2b/I) ESI+ m/z: 438.5
Intermediate 2c (PEx2c) ESI+ m/z: 542.7
Intermediate 2d/Na (PEx2d/Na) ESI+ m/z: 486.5
Compound 2 (Ex2) ESI+ m/z: 1047.3

The NMR spectroscopic analysis of Compound 2 (Ex2) was conducted. The result is shown below (representative signal values of ¹H-NMR spectra are shown; m: multiplet, d: doublet).
NMR (500 MHz, CD₃OD): δ:0.86-0.93 (m, 12H), 1.23-1.41 (m, 72H), 1.52-1.73 (m, 12H), 1.85-1.99 (m, 2H), 2.28-2.35 (m, 10H), 3.21-3.27 (m, 6H), 3.47-3.54 (m, 2H), 3.69-3.80 (m, 2H), 3.99 (d, J = 5.50Hz, 4H), 4.85-4.91 (m, 1H)

### [Industrial Applicability]

The method of the present invention can produce a nonhuman primate animal model of cerebral infarction such as penetrating branch infarction, cerebral infarction having brain damage in a penetrating branch territory, and cerebral infarction at a subacute to chronic stage (particularly, penetrating branch infarction at a subacute to chronic stage and cerebral infarction at a subacute to chronic stage having brain damage in a penetrating branch territory). The nonhuman primate animal model of the present invention is expected to be used as an effective animal model for the search and development of a therapeutic agent for cerebral infarction, particularly, a therapeutic agent for penetrating branch infarction, cerebral infarction having brain damage in a penetrating branch territory, and cerebral infarction at a subacute to chronic stage. The pharmaceutical composition or the treatment method of the present invention is expected to be useful for the treatment of cerebral infarction such as penetrating branch infarction, cerebral infarction having brain damage in a penetrating branch territory, and cerebral infarction at a subacute to chronic stage (particularly, penetrating branch infarction at a subacute to chronic stage and cerebral infarction at a subacute to chronic stage having brain damage in a penetrating branch territory).

SEQ ID NO: 1: NeuroD1 expression vector AAV-GFAP-Neurod1-IRES-GFP
SEQ ID NO: 2: Control vector AAV-GFAP-GFP
SEQ ID NO: 3: GFP gene (CDS)
SEQ ID NO: 4: GFP
SEQ ID NO: 5: Mouse NeuroD1 gene (CDS)
SEQ ID NO: 6: Mouse NeuroD1 protein
SEQ ID NO: 7: Human NeuroD1 gene (CDS)
SEQ ID NO: 8: Human NeuroD1 protein
SEQ ID NO: 9: Endothelin-1 (human-derived)
SEQ ID NO: 10: Human NeuroD1 gene (CDS)
SEQ ID NO: 11: Human NeuroD1 mRNA sequence (ND1-2)
SEQ ID NO: 12: pRC_AAV9
SEQ ID NO: 13: Human NeuroD1 mRNA sequence containing modified nucleosides (ND1-2)
SEQ ID NO: 14: Human GFAP promoter

All the publications, patents, and patent applications cited herein are incorporated herein by reference in their entirety.

## Claims

1. A method for producing a nonhuman primate animal model of cerebral infarction, comprising administering endothelin to basal ganglia and thalamic region of a nonhuman primate, and thereby inducing basal ganglia damage, thalamus damage, and internal capsule damage.

2. The method according to claim 1, wherein endothelin is administered to one or two or more nerve nuclei selected from the group consisting of putamen, caudate nucleus, globus pallidus, ventral lateral nucleus of thalamus, and ventral posterior nucleus of thalamus in the basal ganglia and thalamic region.

3. The method according to claim 1 or 2, wherein endothelin is administered to at least three nerve nuclei selected from the group consisting of putamen, caudate nucleus, globus pallidus, ventral lateral nucleus of thalamus, and ventral posterior nucleus of thalamus in the basal ganglia and thalamic region.

4. The method according to any one of claims 1 to 3, wherein endothelin is administered to at least four nerve nuclei selected from the group consisting of putamen, caudate nucleus, globus pallidus, ventral lateral nucleus of thalamus, and ventral posterior nucleus of thalamus in the basal ganglia and thalamic region.

5. The method according to any one of claims 1 to 4, wherein endothelin is administered to all of five nerve nuclei of the group consisting of putamen, caudate nucleus, globus pallidus, ventral lateral nucleus of thalamus, and ventral posterior nucleus of thalamus in the basal ganglia and thalamic region.

6. The method according to any one of claims 1 to 5, wherein the cerebral infarction is cerebral infarction at a subacute to chronic stage.

7. The method according to any one of claims 1 to 6, wherein the cerebral infarction is cerebral infarction at a chronic stage.

8. The method according to any one of claims 1 to 7, wherein the cerebral infarction is penetrating branch infarction.

9. The method according to any one of claims 1 to 7, wherein the cerebral infarction is cerebral infarction having brain damage in a penetrating branch territory.

10. The method according to claim 8 or 9, wherein the penetrating branch infarction, or the cerebral infarction having brain damage in a penetrating branch territory is cerebral infarction at a subacute to chronic stage.

11. The method according to claim 8 or 9, wherein the penetrating branch infarction, or the cerebral infarction having brain damage in a penetrating branch territory is cerebral infarction at a chronic stage.

12. The method according to any one of claims 2 to 11, comprising determining an administration site so as to target said nerve nuclei, and administering endothelin to the administration site.

13. A method for producing a nonhuman primate animal model of cerebral infarction, comprising administering endothelin to basal ganglia and thalamic region of a nonhuman primate, and thereby inducing basal ganglia damage, thalamus damage, and internal capsule damage, wherein the method comprises the steps of:
(a) determining administration sites so as to target at least four nerve nuclei selected from the group consisting of putamen, caudate nucleus, globus pallidus, ventral lateral nucleus of thalamus, and ventral posterior nucleus of thalamus in the basal ganglia and thalamic region; and
(b) administering endothelin to the administration sites.

14. A method for producing a nonhuman primate animal model of cerebral infarction, comprising administering endothelin to basal ganglia and thalamic region of a nonhuman primate, and thereby inducing basal ganglia damage, thalamus damage, and internal capsule damage, wherein the method comprises the steps of:
(a) determining administration sites so as to target all of five nerve nuclei of the group consisting of putamen, caudate nucleus, globus pallidus, ventral lateral nucleus of thalamus, and ventral posterior nucleus of thalamus in the basal ganglia and thalamic region; and
(b) administering endothelin to the administration sites.

15. The method according to claim 13 or 14, wherein the administration sites are at least three sites.

16. The method according to any one of claims 1 to 15, wherein the nonhuman primate is a cynomolgus macaque.

17. The method according to any one of claims 1 to 16, wherein a dose of endothelin per administration site is at least 10 µg.

18. The method according to any one of claims 1 to 17, wherein the endothelin is endothelin-1.

19. A nonhuman primate animal model of cerebral infarction, which has basal ganglia damage, thalamus damage, and internal capsule damage, wherein the nonhuman primate animal model is produced by a method according to any one of claims 1 to 18.

20. A method for producing a nonhuman primate animal model of cerebral infarction at a subacute to chronic stage, comprising administering endothelin to a cynomolgus macaque, and thereby inducing basal ganglia damage, thalamus damage, and internal capsule damage, wherein endothelin administration sites include the following:
administration sites respectively positioned at (i) B: 1 to 3 mm, L: 7 to 9 mm, and D: 18 to 20 mm, (ii) B: 4 to 6 mm, L: 7 to 9 mm, and D: 18 to 20 mm, and (iii) B: 8 to 12 mm, L: 4 to 13 mm, and D: 12 to 20 mm from Bregma as a base point.

21. The method according to claim 20, wherein the endothelin administration sites include any one of the following (1) to (3):
(1) administration sites respectively positioned at (i) B: 1 to 3 mm, L: 7 to 9 mm, and D: 18 to 20 mm, (ii) B: 4 to 6 mm, L: 7 to 9 mm, and D: 18 to 20 mm, (iii) B: 8 to 10 mm, L: 4 to 6 mm, and D: 12 to 14 mm, and (iv) B: 8 to 10 mm, L: 11 to 13 mm, and D: 14 to 16 mm from Bregma as a base point;
(2) administration sites respectively positioned at (i) B: 1 to 3 mm, L: 7 to 9 mm, and D: 18 to 20 mm, (ii) B: 4 to 6 mm, L: 7 to 9 mm, and D: 18 to 20 mm, and (iii) B: 8 to 10 mm, L: 7 to 9 mm, and D: 18 to 20 mm from Bregma as a base point; and
(3) administration sites respectively positioned at (i) B: 1 to 3 mm, L: 7 to 9 mm, and D: 18 to 20 mm, (ii) B: 4 to 6 mm, L: 7 to 9 mm, and D: 18 to 20 mm, and (iii) B: 10 to 12 mm, L: 7 to 9 mm, and D: 18 to 20 mm from Bregma as a base point.

22. The method according to claim 20 or 21, wherein the endothelin administration sites include any one of the following (1) to (3):
(1) administration sites respectively positioned at (i) B: 2 mm, L: 8 mm, and D: 19 mm, (ii) B: 5 mm, L: 8 mm, and D: 19 mm, (iii) B: 9 mm, L: 5 mm, and D: 13 mm, and (iv) B: 9 mm, L: 12 mm, and D: 15 mm from Bregma as a base point;
(2) administration sites respectively positioned at (i) B: 2 mm, L: 8 mm, and D: 19 mm, (ii) B: 5 mm, L: 8 mm, and D: 19 mm, and (iii) B: 9 mm, L: 8 mm, and D: 19 mm from Bregma as a base point; and
(3) administration sites respectively positioned at (i) B: 2 mm, L: 8 mm, and D: 19 mm, (ii) B: 5 mm, L: 8 mm, and D: 19 mm, and (iii) B: 11 mm, L: 8 mm, and D: 19 mm from Bregma as a base point.

23. The method according to any one of claims 20 to 22, wherein the endothelin administration sites include the following administration sites:
administration sites respectively positioned at (i) B: 2 mm, L: 8 mm, and D: 19 mm, (ii) B: 5 mm, L: 8 mm, and D: 19 mm, (iii) B: 9 mm, L: 5 mm, and D: 13 mm, and (iv) B: 9 mm, L: 12 mm, and D: 15 mm from Bregma as a base point.

24. The method according to any one of claims 20 to 23, wherein the endothelin administration sites are the following administration sites:
administration sites respectively positioned at (i) B: 2 mm, L: 8 mm, and D: 19 mm, (ii) B: 5 mm, L: 8 mm, and D: 19 mm, (iii) B: 9 mm, L: 5 mm, and D: 13 mm, and (iv) B: 9 mm, L: 12 mm, and D: 15 mm from Bregma as a base point.

25. The method according to any one of claims 20 to 24, wherein the cerebral infarction is cerebral infarction at a chronic stage.

26. The method according to any one of claims 20 to 25, wherein the cerebral infarction is penetrating branch infarction, or cerebral infarction having brain damage in a penetrating branch territory.

27. The method according to any one of claims 20 to 26, wherein a dose of endothelin per administration site is at least 10 µg.

28. The method according to any one of claims 20 to 27, wherein the endothelin is endothelin-1.

29. A cynomolgus macaque model of cerebral infarction at a subacute to chronic stage, produced by a method according to any one of claims 20 to 28.

30. A method for screening for a therapeutic agent for cerebral infarction, comprising the steps of: administering a test substance to a nonhuman primate animal model according to claim 19 or 29; and determining an effect of the test substance on cerebral infarction in the nonhuman primate animal model.

31. A pharmaceutical composition for the treatment of penetrating branch infarction, or cerebral infarction at a subacute to chronic stage, comprising a NeuroD1 protein or a polynucleotide encoding the NeuroD1 protein.

32. A pharmaceutical composition for the treatment of cerebral infarction having brain damage in a penetrating branch territory, comprising a NeuroD1 protein or a polynucleotide encoding the NeuroD1 protein.

33. The pharmaceutical composition according to claim 31 or 32, wherein the penetrating branch infarction, or the cerebral infarction having brain damage in a penetrating branch territory is cerebral infarction at a subacute to chronic stage.

34. The pharmaceutical composition according to any one of claims 31 to 33, wherein the penetrating branch infarction, or the cerebral infarction having brain damage in a penetrating branch territory is cerebral infarction at a chronic stage.

35. The pharmaceutical composition according to claim 31, wherein the cerebral infarction at a subacute to chronic stage is cerebral infarction at a chronic stage.

36. The pharmaceutical composition according to any one of claims 31 to 35, wherein the pharmaceutical composition comprises the polynucleotide encoding the NeuroD1 protein, and said polynucleotide is a polynucleotide comprising a nucleotide sequence encoding a protein consisting of the amino acid sequence as set forth by SEQ ID NO: 8.

37. The pharmaceutical composition according to any one of claims 31 to 36, wherein the polynucleotide encoding the NeuroD1 protein comprises a polynucleotide having 70% or higher sequence identity to the nucleotide sequence as set forth by SEQ ID NO: 7 or 10.

38. The pharmaceutical composition according to any one of claims 31 to 37, wherein the polynucleotide encoding the NeuroD1 protein is a polynucleotide comprising the nucleotide sequence as set forth by SEQ ID NO: 7 or 10.

39. The pharmaceutical composition according to any one of claims 31 to 38, wherein the polynucleotide encoding the NeuroD1 protein is mRNA encoding the NeuroD1 protein.

40. The pharmaceutical composition according to claim 39, wherein the mRNA encoding the NeuroD1 protein comprises a nucleotide sequence encoding the NeuroD1 protein of positions 44 to 1114 or positions 44 to 1120 of SEQ ID NO: 11 or 13.

41. The pharmaceutical composition according to claim 39 or 40, wherein the mRNA encoding the NeuroD1 protein comprises 5' UTR and/or 3' UTR.

42. The pharmaceutical composition according to any one of claims 39 to 41, wherein the mRNA encoding the NeuroD1 protein is mRNA comprising a polynucleotide consisting of the nucleotide sequence of positions 1 to 1231 of SEQ ID NO: 11 or 13.

43. The pharmaceutical composition according to any one of claims 39 to 42, wherein the mRNA encoding the NeuroD1 protein comprises a polyA sequence.

44. The pharmaceutical composition according to claim 43, wherein the polyA sequence is 50 to 200 nucleotides in length.

45. The pharmaceutical composition according to any one of claims 39 to 44, wherein the mRNA encoding the NeuroD1 protein has a 5' cap structure.

46. The pharmaceutical composition according to any one of claims 39 to 45, wherein the mRNA encoding the NeuroD1 protein is mRNA containing a modified nucleoside.

47. The pharmaceutical composition according to claim 46, wherein the modified nucleoside is N1-methylpseudouridine.

48. The pharmaceutical composition according to claim 47, wherein the mRNA encoding the NeuroD1 protein is a polynucleotide in which some or all uridines are replaced by N1-methylpseudouridine.

49. The pharmaceutical composition according to any one of claims 39 to 48, wherein the mRNA encoding the NeuroD1 protein is incorporated in a drug delivery system (DDS).

50. The pharmaceutical composition according to any one of claims 39 to 49, wherein the mRNA encoding the NeuroD1 protein is encapsulated into lipid nanoparticles.

51. The pharmaceutical composition according to any one of claims 31 to 38, wherein the pharmaceutical composition comprises the polynucleotide encoding the NeuroD1 protein, and said polynucleotide is DNA.

52. The pharmaceutical composition according to any one of claims 31 to 38 and 51, wherein the pharmaceutical composition comprises an expression vector comprising the polynucleotide encoding the NeuroD1 protein.

53. The pharmaceutical composition according to claim 52, wherein the expression vector is an adeno-associated virus vector, a retrovirus vector, a Sendai virus vector, or a lentivirus vector.

54. The pharmaceutical composition according to claim 53, wherein the expression vector is an adeno-associated virus vector.

55. A method for treating penetrating branch infarction, or cerebral infarction at a subacute to chronic stage, comprising the step of administering a pharmaceutical composition according to any one of claims 31 to 54, a NeuroD1 protein, or a polynucleotide encoding the NeuroD1 protein to a subject.

56. A method for treating cerebral infarction having brain damage in a penetrating branch territory, comprising the step of administering a pharmaceutical composition according to any one of claims 31 to 54, a NeuroD1 protein, or a polynucleotide encoding the NeuroD1 protein to a subject.

57. The method according to claim 55 or 56, wherein the penetrating branch infarction, or the cerebral infarction having brain damage in a penetrating branch territory is cerebral infarction at a subacute to chronic stage.

58. The method according to any one of claim 55 to 57, wherein the penetrating branch infarction, or the cerebral infarction having brain damage in a penetrating branch territory is cerebral infarction at a chronic stage.

59. The method according to claim 55, wherein the cerebral infarction at a subacute to chronic stage is cerebral infarction at a chronic stage.

60. The method according to any one of claims 55 to 59, wherein the method comprises the step of administering the polynucleotide encoding the NeuroD1 protein to the subject, and wherein said polynucleotide is a polynucleotide comprising a nucleotide sequence encoding a protein consisting of the amino acid sequence as set forth by SEQ ID NO: 8.

61. The method according to any one of claims 55 to 60, wherein the polynucleotide encoding the NeuroD1 protein comprises a polynucleotide having 70% or higher sequence identity to the nucleotide sequence as set forth by SEQ ID NO: 7 or 10.

62. The method according to any one of claims 55 to 61, wherein the polynucleotide encoding the NeuroD1 protein is a polynucleotide comprising the nucleotide sequence as set forth by SEQ ID NO: 7 or 10.

63. The method according to any one of claims 55 to 62, wherein the method comprises the step of administering the polynucleotide encoding the NeuroD1 protein to the subject, and wherein said polynucleotide is mRNA encoding the NeuroD1 protein.

64. The method according to claim 63, wherein the mRNA encoding the NeuroD1 protein comprises a nucleotide sequence encoding the NeuroD1 protein of positions 44 to 1114 or positions 44 to 1120 of SEQ ID NO: 11 or 13.

65. The method according to claim 63 or 64, wherein the mRNA encoding the NeuroD1 protein comprises 5' UTR and/or 3' UTR.

66. The method according to any one of claims 63 to 65, wherein the mRNA encoding the NeuroD1 protein is mRNA comprising a polynucleotide consisting of the nucleotide sequence of positions 1 to 1231 of SEQ ID NO: 11 or 13.

67. The method according to any one of claims 63 to 66, wherein the mRNA encoding the NeuroD1 protein comprises a polyA sequence.

68. The method according to claim 67, wherein the polyA sequence is 50 to 200 nucleotides in length.

69. The method according to any one of claims 63 to 68, wherein the mRNA encoding the NeuroD1 protein has a 5' cap structure.

70. The method according to any one of claims 63 to 69, wherein the mRNA encoding the NeuroD1 protein is mRNA containing a modified nucleoside.

71. The method according to claim 70, wherein the modified nucleoside is N1-methylpseudouridine.

72. The method according to claim 71, wherein the mRNA encoding the NeuroD1 protein is a polynucleotide in which some or all uridines are replaced by N1-methylpseudouridine.

73. The method according to any one of claims 63 to 72, wherein the mRNA encoding the NeuroD1 protein is incorporated in a drug delivery system (DDS).

74. The method according to any one of claims 63 to 73, wherein the mRNA encoding the NeuroD1 protein is encapsulated into lipid nanoparticles.

75. The method according to any one of claims 55 to 62, wherein the method comprises the step of administering the polynucleotide encoding the NeuroD1 protein to the subject, and wherein said polynucleotide is DNA.

76. The method according to any one of claims 55 to 62 and 75, wherein the method comprises the step of administering an expression vector comprising the polynucleotide encoding the NeuroD1 protein to the subject.

77. The method according to claim 76, wherein the expression vector is an adeno-associated virus vector, a retrovirus vector, a Sendai virus vector, or a lentivirus vector.

78. The method according to claim 77, wherein the expression vector is an adeno-associated virus vector.

79. A NeuroD1 protein or a polynucleotide encoding the NeuroD1 protein, for use in the treatment of penetrating branch infarction, or cerebral infarction at a subacute to chronic stage.

80. A NeuroD1 protein or a polynucleotide encoding the NeuroD1 protein, for use in the treatment of cerebral infarction having brain damage in a penetrating branch territory.

81. The protein or the polynucleotide for use according to claim 79 or 80, wherein the penetrating branch infarction, or the cerebral infarction having brain damage in a penetrating branch territory is cerebral infarction at a subacute to chronic stage.

82. The protein or the polynucleotide for use according to any one of claim 79 to 81, wherein the penetrating branch infarction, or the cerebral infarction having brain damage in a penetrating branch territory is cerebral infarction at a chronic stage.

83. The protein or the polynucleotide for use according to claim 79, wherein the cerebral infarction at a subacute to chronic stage is cerebral infarction at a chronic stage.

84. The polynucleotide for use according to any one of claim 79 to 83, wherein the polynucleotide encoding the NeuroD1 protein is a polynucleotide comprising a nucleotide sequence encoding a protein consisting of the amino acid sequence as set forth by SEQ ID NO: 8.

85. The polynucleotide for use according to any one of claims 79 to 84, wherein the polynucleotide encoding the NeuroD1 protein comprises a polynucleotide having 70% or higher sequence identity to the nucleotide sequence as set forth by SEQ ID NO: 7 or 10.

86. The polynucleotide for use according to any one of claims 79 to 85, wherein the polynucleotide encoding the NeuroD1 protein is a polynucleotide comprising the nucleotide sequence as set forth by SEQ ID NO: 7 or 10.

87. The polynucleotide for use according to any one of claims 79 to 86, wherein said polynucleotide is mRNA encoding the NeuroD1 protein.

88. The polynucleotide for use according to claim 87, wherein the mRNA encoding the NeuroD1 protein comprises a nucleotide sequence encoding the NeuroD1 protein of positions 44 to 1114 or positions 44 to 1120 of SEQ ID NO: 11 or 13.

89. The polynucleotide for use according to claim 87 or 88, wherein the mRNA encoding the NeuroD1 protein comprises 5' UTR and/or 3' UTR.

90. The polynucleotide for use according to any one of claims 87 to 89, wherein the mRNA encoding the NeuroD1 protein is mRNA comprising a polynucleotide consisting of the nucleotide sequence of positions 1 to 1231 of SEQ ID NO: 11 or 13.

91. The polynucleotide for use according to any one of claims 87 to 90, wherein the mRNA encoding the NeuroD1 protein comprises a polyA sequence.

92. The polynucleotide for use according to claim 91, wherein the polyA sequence is 50 to 200 nucleotides in length.

93. The polynucleotide for use according to any one of claims 87 to 92, wherein the mRNA encoding the NeuroD1 protein has a 5' cap structure.

94. The polynucleotide for use according to any one of claims 87 to 93, wherein the mRNA encoding the NeuroD1 protein is a polynucleotide containing a modified nucleoside.

95. The polynucleotide for use according to claim 94, wherein the modified nucleoside is N1-methylpseudouridine.

96. The polynucleotide for use according to claim 95, wherein the mRNA encoding the NeuroD1 protein is a polynucleotide in which some or all uridines are replaced by N1-methylpseudouridine.

97. The polynucleotide for use according to any one of claims 87 to 96, wherein the mRNA encoding the NeuroD1 protein is incorporated in a drug delivery system (DDS).

98. The polynucleotide for use according to any one of claims 87 to 97, wherein the mRNA encoding the NeuroD1 protein is encapsulated into lipid nanoparticles.

99. The polynucleotide for use according to any one of claims 79 to 86, wherein said polynucleotide is DNA.

100. The polynucleotide for use according to any one of claims 79 to 86 and 99, wherein said polynucleotide is an expression vector comprising the polynucleotide encoding the NeuroD1 protein.

101. The polynucleotide for use according to claim 100, wherein the expression vector is an adeno-associated virus vector, a retrovirus vector, a Sendai virus vector, or a lentivirus vector.

102. The polynucleotide for use according to claim 101, wherein the expression vector is an adeno-associated virus vector.

103. Use of a NeuroD1 protein or a polynucleotide encoding the NeuroD1 protein in the manufacture of a pharmaceutical composition for the treatment of penetrating branch infarction, or cerebral infarction at a subacute to chronic stage.

104. Use of a NeuroD1 protein or a polynucleotide encoding the NeuroD1 protein in the manufacture of a pharmaceutical composition for the treatment of cerebral infarction having brain damage in a penetrating branch territory.

105. Use according to claim 103 or 104, wherein the penetrating branch infarction, or the cerebral infarction having brain damage in a penetrating branch territory is cerebral infarction at a subacute to chronic stage.

106. Use according to any one of claims 103 to 105, wherein the penetrating branch infarction, or the cerebral infarction having brain damage in a penetrating branch territory is cerebral infarction at a chronic stage.

107. Use according to claim 103, wherein the cerebral infarction at a subacute to chronic stage is cerebral infarction at a chronic stage.

108. Use according to any one of claims 103 to 107, wherein the polynucleotide encoding the NeuroD1 protein is a polynucleotide comprising a nucleotide sequence encoding a protein consisting of the amino acid sequence as set forth by SEQ ID NO: 8.

109. Use according to any one of claims 103 to 108, wherein the polynucleotide encoding the NeuroD1 protein comprises a polynucleotide having 70% or higher sequence identity to the nucleotide sequence as set forth by SEQ ID NO: 7 or 10.

110. Use according to any one of claims 103 to 109, wherein the polynucleotide encoding the NeuroD1 protein is a polynucleotide comprising the nucleotide sequence as set forth by SEQ ID NO: 7 or 10.

111. Use according to any one of claims 103 to 110, wherein said polynucleotide is mRNA encoding the NeuroD1 protein.

112. Use according to claim 111, wherein the mRNA encoding the NeuroD1 protein comprises a nucleotide sequence encoding the NeuroD1 protein of positions 44 to 1114 or positions 44 to 1120 of SEQ ID NO: 11 or 13.

113. Use according to claim 111 or 112, wherein the mRNA encoding the NeuroD1 protein comprises 5' UTR and/or 3' UTR.

114. Use according to any one of claims 111 to 113, wherein the mRNA encoding the NeuroD1 protein is mRNA comprising a polynucleotide consisting of the nucleotide sequence of positions 1 to 1231 of SEQ ID NO: 11 or 13.

115. Use according to any one of claims 111 to 114, wherein the mRNA encoding the NeuroD1 protein comprises a polyA sequence.

116. Use according to claim 115, wherein the polyA sequence is 50 to 200 nucleotides in length.

117. Use according to any one of claims 111 to 116, wherein the mRNA encoding the NeuroD1 protein has a 5' cap structure.

118. Use according to any one of claims 111 to 117, wherein the mRNA encoding the NeuroD1 protein is mRNA containing a modified nucleoside.

119. Use according to claim 118, wherein the modified nucleoside is N1-methylpseudouridine.

120. Use according to claim 119, wherein the mRNA encoding the NeuroD1 protein is a polynucleotide in which some or all uridines are replaced by N1-methylpseudouridine.

121. Use according to any one of claims 111 to 120, wherein the mRNA encoding the NeuroD1 protein is incorporated in a drug delivery system (DDS).

122. Use according to any one of claims 111 to 121, wherein the mRNA encoding the NeuroD1 protein is encapsulated into lipid nanoparticles.

123. Use according to any one of claims 103 to 110, wherein said polynucleotide is DNA.

124. Use according to any one of claims 103 to 110 and 123, wherein said polynucleotide is an expression vector comprising the polynucleotide encoding the NeuroD1 protein.

125. Use according to claim 124, wherein the expression vector is an adeno-associated virus vector, a retrovirus vector, a Sendai virus vector, or a lentivirus vector.

126. Use according to claim 125, wherein the expression vector is an adeno-associated virus vector.
